# EUROPEAN PATENT APPLICATION

(11) **EP 4 592 290 A1**
(43) Date of publication of application: **30.07.2025**
(21) Application number: 23867500.3
(22) Date of filing: 19.09.2023
(51) Int. Cl.: C07D 487/04, C07D 473/00, C07D 417/14, C07D 413/14, A61K 31/519, A61P 35/00

(54) **CARBONYL FUSED HETEROCYCLIC DERIVATIVE USED AS UBIQUITIN-SPECIFIC PROTEASE INHIBITOR**

(30) Priority: 20.09.2022 CN 202211144812; 09.06.2023 CN 202310690391; 08.09.2023 CN 202311161323
(71) Applicant: Chia Tai Tianqing Pharmaceutical Group Co., Ltd., Lianyungang, Jiangsu 222062 (CN)
(72) Inventor: LIU, Baomin, Lianyungang, Jiangsu 222062 (CN); DAI, Zonghao, Lianyungang, Jiangsu 222062 (CN); HU, Jinfa, Lianyungang, Jiangsu 222062 (CN); HUANG, Yu, Lianyungang, Jiangsu 222062 (CN)
(74) Representative: Grünecker Patent- und Rechtsanwälte PartG mbB
(86) International application number: PCT/CN2023/119722
(87) International publication number: WO 2024/061213

(57) **Abstract**

The present invention provides a carbonyl fused heterocyclic derivative used as a ubiquitin-specific protease inhibitor, and specifically relates to a compound represented by formula (1), an isomer thereof, or a pharmaceutically acceptable salt thereof.

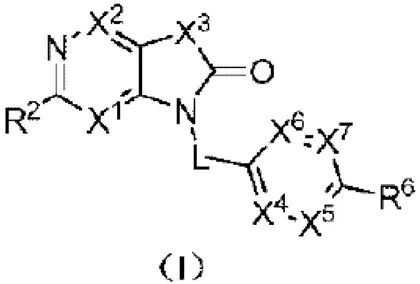

## Description

### CROSS-REFERENCE TO RELATED APPLICATIONS

The present application claims the priority and benefit to the Chinese Patent Application No. 202211144812.4 filed with the China National Intellectual Property Administration on September 20, 2022, the Chinese Patent Application No. 202310690391.3 filed with China National Intellectual Property Administration on June 09, 2023, and the Chinese Patent Application No. 202311161323.4 filed with China National Intellectual Property Administration on September 08, 2023, the contents of which are incorporated herein by reference in their entireties.

### TECHNICAL FIELD

The present disclosure relates to the field of pharmaceutical chemistry, and relates to a carbonyl fused heterocyclic derivative used as a ubiquitin specific protease inhibitor, in particular to a compound of formula (I), an isomer thereof, or a pharmaceutically acceptable salt thereof.

### BACKGROUND

USP1, known as ubiquitin specific peptidase 1, is a member of the deubiquitinating enzyme family. USP1, together with its cofactor UAF1 (USP1-associated factor 1), forms a USP1-UAF1 complex, which deubiquitinates Ub-FANCD2 and Ub-PCNA, two critical proteins involved in the FA and TLS pathways, thereby activating DNA cross-linking damage repair and promoting the survival of cancer cells. USP1 is highly expressed in various human tumors, is closely related to the development and progression of tumors, and has received extensive attention as a tumor therapeutic target.

Genome-wide target knockout studies based on the CRISPR technology have revealed that USP1 exhibits a synthetic lethal effect with BRCA1/2 mutations or other homologous recombination deficiencies (HRDs). From the mechanism of action, USP1 and PARP are key regulatory proteins in DNA cross-linking damage and single-strand damage repair pathways, which complement each other to promote DNA damage repair and prevent the formation of DNA double-strand breaks with lethal cytotoxicity. Therefore, USP1 is an important target with great clinical value in the field of DNA damage repair.

### SUMMARY

The present disclosure provides a compound of formula (I), an isomer thereof, or a pharmaceutically acceptable salt thereof, 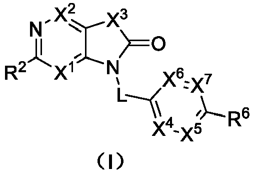 wherein,
X¹ and X² are each independently selected from the group consisting of C(R¹) and N;
X³ is selected from the group consisting of C(R¹)₂, C(=Z), N(R⁷), S, and O;
X⁴, X⁵, X⁶, and X⁷ are each independently selected from the group consisting of C(R⁵) and N;
each R¹ and R⁵ is independently selected from the group consisting of hydrogen, deuterium, halogen, -CN, -OH, - SH, -COOH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, - OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl, wherein the -OH, -SH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more substituents;
Z is selected from the group consisting of O, S, and N(R⁸);
or X² and X³ are linked to each other to form a 5- to 7-membered ring optionally substituted with one or more substituents;
or R⁵ groups on two adjacent carbon atoms, together with the carbon atoms to which they are linked, form C₅₋₇ cycloalkenyl, 5- to 7-membered heterocycloalkenyl, phenyl, or 5- to 6-membered heteroaryl optionally substituted with one or more substituents;
L is selected from the group consisting of -(C(R³R⁴))ₘ-, -O-, -S-, -N(R⁹)-, -S(O)-, and -S(O)₂-;
R² is selected from the group consisting of C₃₋₁₂ cycloalkyl, C₆₋₁₀ aryl, 5- to 10-membered heteroaryl, 3- to 12-membered heterocyclyl, and C₅₋₇ cycloalkenyl, wherein the C₃₋₁₂ cycloalkyl, C₆₋₁₀ aryl, 5- to 10-membered heteroaryl, 3- to 12-membered heterocyclyl, and C₅₋₇ cycloalkenyl are optionally independently substituted with one or more R^{2a};
each R³ and R⁴ is independently selected from the group consisting of hydrogen, deuterium, halogen, -CN, -OH, - SH, -COOH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C_{I-12} alkyl, -C(O)OC₁₋₁₂ alkyl, - OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl, wherein the -OH, -SH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more substituents;
or R³ and R⁴, together with the carbon atom to which they are linked, form C₃₋₆ cycloalkyl or 3- to 6-membered heterocycloalkyl optionally substituted with one or more substituents;
R⁶ is selected from the group consisting of hydrogen, halogen, -CN, -OH, -SH, -COOH, -NH₂, -NHC₁₋₁₂ alkyl, - N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, - NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, - CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl, wherein the -OH, - SH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more R^{6a};
each R⁷, R⁸, or R⁹ is independently selected from the group consisting of hydrogen, -NH₂, C₁₋₁₂ alkyl, C₁₋₁₂ deuterated alkyl, C₁₋₁₂ alkoxy, C₁₋₁₂ deuterated alkoxy, C₃₋₁₂ cycloalkyl, and 3- to 12-membered heterocyclyl, wherein the -NH₂, C₁₋₁₂ alkyl, C₁₋₁₂ deuterated alkyl, C₁₋₁₂ alkoxy, C₁₋₁₂ deuterated alkoxy, C₃₋₁₂ cycloalkyl, or 3- to 12-membered heterocyclyl is optionally substituted with one or more substituents;
each R^{2a} and R^{6a} is independently selected from the group consisting of hydrogen, deuterium, halogen, -CN, -OH, -SH, -COOH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, - OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl, wherein the -OH, -SH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more substituents;
m is selected from the group consisting of 1, 2, and 3;
provided that when X¹ is N, X³ is not NH.

In some embodiments of the present disclosure, at least one of X¹ and X² is selected from C(R¹).

In some embodiments of the present disclosure, X³ is selected from the group consisting of C(=O), N(R⁷), S, and O.

In some embodiments of the present disclosure, X² is selected from C(R¹), and X¹ is selected from N; X³ is selected from the group consisting of C(=O), N(R⁷), S, and O.

In some embodiments of the present disclosure, X² is selected from C(R¹), and X¹ is selected from N; X³ is selected from N(R⁷).

In some embodiments of the present disclosure, X² is selected from C(R¹), and X¹ is selected from N; X³ is selected from S.

In some embodiments of the present disclosure, X² is selected from C(R¹), and X¹ is selected from N; X³ is selected from O.

In some embodiments of the present disclosure, X² is selected from C(R¹), and X¹ is selected from N; X³ is selected from C(R¹)₂.

In some embodiments of the present disclosure, X² is selected from N, and X¹ is selected from C(R¹); X³ is selected from the group consisting of C(=O), N(R⁷), S, and O.

In some embodiments of the present disclosure, X² is selected from N, and X¹ is selected from C(R¹); X³ is selected from N(R⁷).

In some embodiments of the present disclosure, X² is selected from N, and X¹ is selected from C(R¹); X³ is selected from S.

In some embodiments of the present disclosure, X² is selected from N, and X¹ is selected from C(R¹); X³ is selected from O.

In some embodiments of the present disclosure, X² is selected from N, and X¹ is selected from C(R¹); X³ is selected from C(R¹)₂.

In some embodiments of the present disclosure, X¹ is selected from C(R¹), and X² is selected from C(R¹); X³ is selected from the group consisting of C(=O), N(R⁷), S, and O.

In some embodiments of the present disclosure, X¹ is selected from C(R¹), and X² is selected from C(R¹); X³ is selected from C(R¹)₂.

In some embodiments of the present disclosure, X¹ is selected from C(R¹), and X² is selected from C(R¹); X³ is selected from N(R⁷).

In some embodiments of the present disclosure, X¹ is selected from C(R¹), and X² is selected from C(R¹); X³ is selected from S.

In some embodiments of the present disclosure, X¹ is selected from C(R¹), and X² is selected from C(R¹); X³ is selected from O.

In some other embodiments of the present disclosure, the "one or more" described herein is selected from the group consisting of 1, 2, 3, 4, 5, and 6.

In some other embodiments of the present disclosure, the "one or more" described herein is selected from the group consisting of 1, 2, and 3.

In some embodiments of the present disclosure, each R¹ and R⁵ is independently selected from the group consisting of hydrogen, halogen, -CN, -OH, -SH, -COOH, -NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl, wherein the -OH, -SH, -NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, - NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, - CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more substituents.

In some embodiments of the present disclosure, each R¹ and R⁵ is independently selected from the group consisting of hydrogen, halogen, -CN, -OH, -SH, -COOH, -NH₂, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ alkylthio, -COC₁₋₆ alkyl, -OC(O)C₁₋₆ alkyl, -C(O)OC₁₋₆ alkyl, -OC(O)OC₁₋₆ alkyl, -SO₂C₁₋₆ alkyl, -NHSO₂C₁₋₆ alkyl, -N(C₁₋₆ alkyl)SO₂C₁₋₆ alkyl, -SO₂NH₂, -SO₂NHC₁₋₆ alkyl, -SO₂N(C₁₋₆ alkyl)₂, -CONH₂, -CONHC₁₋₆ alkyl, -CON(C₁₋₆ alkyl)₂, -NHCOC₁₋₆ alkyl, -N(C₁₋₆ alkyl)COC₁₋₆ alkyl, C₃₋₈ cycloalkyl, C₃₋₈ cycloalkenyl, phenyl, 5- to 10-membered heteroaryl, and 3- to 8-membered heterocyclyl, wherein the -OH, -SH, - NH₂, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ alkylthio, -COC₁₋₆ alkyl, -OC(O)C₁₋₆ alkyl, -C(O)OC₁₋₆ alkyl, -OC(O)OC₁₋₆ alkyl, -SO₂C₁₋₆ alkyl, -NHSO₂C₁₋₆ alkyl, -N(C₁₋₆ alkyl)SO₂C₁₋₆ alkyl, -SO₂NH₂, -SO₂NHC₁₋₆ alkyl, -SO₂N(C₁₋₆ alkyl)₂, -CONH₂, -CONHC₁₋₆ alkyl, -CON(C₁₋₆ alkyl)₂, -NHCOC₁₋₆ alkyl, -N(C₁₋₆ alkyl)COC₁₋₆ alkyl, C₃₋₈ cycloalkyl, C₃₋₈ cycloalkenyl, phenyl, 5- to 10-membered heteroaryl, and 3- to 8-membered heterocyclyl are optionally independently substituted with one or more substituents.

In some embodiments of the present disclosure, each R¹ and R⁵ is independently selected from the group consisting of hydrogen, halogen, -CN, -OH, -SH, -COOH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -COC₁₋₃ alkyl, -OC(O)C₁₋₃ alkyl, -C(O)OC₁₋₃ alkyl, -OC(O)OC₁₋₃ alkyl, -SO₂C₁₋₃ alkyl, -NHSO₂C₁₋₃ alkyl, -N(C₁₋₃ alkyl)SO₂C₁₋₃ alkyl, -SO₂NH₂, -SO₂NHC₁₋₃ alkyl, -SO₂N(C₁₋₃ alkyl)₂, -CONH₂, -CONHC₁₋₃ alkyl, -CON(C₁₋₃ alkyl)₂, -NHCOC₁₋₃ alkyl, -N(C₁₋₃ alkyl)COC₁₋₃ alkyl, C₃₋₆ cycloalkyl, C₃₋₆ cycloalkenyl, phenyl, 5- to 6-membered heteroaryl, and 3- to 6-membered heterocyclyl, wherein the -OH, -SH, - NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -COC₁₋₃ alkyl, -OC(O)C₁₋₃ alkyl, -C(O)OC₁₋₃ alkyl, -OC(O)OC₁₋₃ alkyl, -SO₂C₁₋₃ alkyl, -NHSO₂C₁₋₃ alkyl, -N(C₁₋₃ alkyl)SO₂C₁₋₃ alkyl, -SO₂NH₂, -SO₂NHC₁₋₃ alkyl, -SO₂N(C₁₋₃ alkyl)₂, -CONH₂, -CONHC₁₋₃ alkyl, -CON(C₁₋₃ alkyl)₂, -NHCOC₁₋₃ alkyl, -N(C₁₋₃ alkyl)COC₁₋₃ alkyl, C₃₋₆ cycloalkyl, C₃₋₆ cycloalkenyl, phenyl, 5- to 6-membered heteroaryl, and 3-to 6-membered heterocyclyl are optionally independently substituted with one or more substituents.

In some embodiments of the present disclosure, each R¹ and R⁵ is independently selected from the group consisting of hydrogen, halogen, -CN, -OH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl, wherein the -OH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl are optionally independently substituted with one or more substituents.

In some other embodiments of the present disclosure, each R¹ and R⁵ is independently selected from the group consisting of hydrogen, deuterium, halogen, -CN, -OH, -NH₂, -NHC₁₋₃ alkyl, -N(C₁₋₃ alkyl)₂, C₁₋₃ alkyl, C₁₋₃ deuterated alkyl, C₂₋₃ alkenyl, C₂₋₃ alkynyl, C₁₋₃ alkoxy, C₁₋₃ deuterated alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl, wherein the -OH, -NH₂, -NHC₁₋₃ alkyl, -N(C₁₋₃ alkyl)₂, C₁₋₃ alkyl, C₁₋₃ deuterated alkyl, C₂₋₃ alkenyl, C₂₋₃ alkynyl, C₁₋₃ alkoxy, C₁₋₃ deuterated alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl are optionally independently substituted with one or more substituents.

In some other embodiments of the present disclosure, each R¹ and R⁵ is independently selected from the group consisting of hydrogen, deuterium, halogen, -CN, -OH, -NH₂, -NHC₁₋₃ alkyl, -N(C₁₋₃ alkyl)₂, C₁₋₃ alkyl, C₁₋₃ deuterated alkyl, C₁₋₃ alkoxy, and C₁₋₃ deuterated alkoxy, wherein the C₁₋₃ alkoxy, C₁₋₃ deuterated alkoxy, C₁₋₃ alkoxy, or C₁₋₃ deuterated alkoxy is optionally independently substituted with one or more substituents.

In some other embodiments of the present disclosure, each R¹ and R⁵ is independently selected from the group consisting of hydrogen, deuterium, halogen, C₁₋₃ alkyl, and C₁₋₃ deuterated alkyl, wherein the C₁₋₃ alkyl or C₁₋₃ deuterated alkyl is optionally independently substituted with one or more substituents.

In some embodiments of the present disclosure, each R¹ and R⁵ is independently selected from the group consisting of hydrogen, halogen, -CN, -OH, -NH₂, C₁₋₃ alkyl, C₁₋₃ alkoxy, C₃₋₆ cycloalkyl, and 3- to 6-membered heterocyclyl, wherein the C₁₋₃ alkyl, C₁₋₃ alkoxy, C₃₋₆ cycloalkyl, and 3- to 6-membered heterocyclyl are optionally independently substituted with one or more substituents.

In some embodiments of the present disclosure, each R¹ and R³ is independently selected from the group consisting of hydrogen, halogen, -CN, -OH, -NH₂, methyl, ethyl, isopropyl, methoxy, ethoxy, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, and isoxazolidinyl, wherein the methyl, ethyl, isopropyl, methoxy, ethoxy, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, or isoxazolidinyl is optionally independently substituted with one or more substituents.

In some other embodiments of the present disclosure, each R¹ and R⁵ is optionally independently substituted with one or more groups selected from the group consisting of halogen, deuterium, OH, CN, and NH₂.

In some embodiments of the present disclosure, each R¹ and R⁵ is optionally independently substituted with one or more groups selected from the group consisting of halogen, OH, CN, and NH₂.

In some embodiments of the present disclosure, each R¹ and R⁵ is optionally independently substituted with 1, 2, or 3 groups selected from the group consisting of halogen, OH, CN, and NH₂.

In some embodiments of the present disclosure, each R¹ and R⁵ is independently selected from the group consisting of hydrogen, halogen, -CN, -OH, -NH₂, methyl, ethyl, isopropyl, methoxy, ethoxy, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, and isoxazolidinyl, wherein the methyl, ethyl, isopropyl, methoxy, ethoxy, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, or isoxazolidinyl is optionally independently substituted with 1, 2, or 3 groups selected from the group consisting of halogen, OH, CN, and NH₂. In some embodiments of the present disclosure, each R¹ and R⁵ is independently selected from the group consisting of hydrogen, -F, -Cl, -Br, -I, -CN, methyl, ethyl, isopropyl, methoxy, ethoxy, isopropoxy, cyclopropyl, cyclobutyl, oxetanyl, thietanyl, and azetidinyl, wherein the methyl, ethyl, isopropyl, methoxy, ethoxy, isopropoxy, cyclopropyl, cyclobutyl, oxetanyl, thietanyl, or azetidinyl is optionally independently substituted with 1, 2, or 3 groups selected from the group consisting of halogen, OH, CN, and NH₂.

In some embodiments of the present disclosure, each R¹ is independently selected from the group consisting of hydrogen, -F, -Cl, -Br, -I, -CN, methyl, ethyl, methoxy, monofluoromethyl, difluoromethyl, trifluoromethyl, monofluoromethoxy, difluoromethoxy, and trifluoromethoxy.

In some embodiments of the present disclosure, each R¹ is independently selected from the group consisting of hydrogen, -F, methyl, ethyl, and CN.

In some other embodiments of the present disclosure, each R¹ is independently selected from the group consisting of hydrogen, -F, methyl, -CD₃, ethyl, -CD₂CD₃, and CN.

In some embodiments of the present disclosure, each R¹ is independently selected from the group consisting of hydrogen and -F.

In some embodiments of the present disclosure, each R¹ is independently selected from -F.

In some embodiments of the present disclosure, Z is selected from the group consisting of O and S.

In some embodiments of the present disclosure, Z is selected from O.

In some embodiments of the present disclosure, X² and X³ are linked to each other to form a 5- to 7-membered ring optionally substituted with one or more substituents, wherein the 5- to 7-membered ring is selected from the group consisting of 5- to 7-membered heterocycloalkenyl, C₅₋₇ cycloalkenyl, phenyl, and 5- to 6-membered heteroaryl. In some embodiments of the present disclosure, X² and X³ are linked to each other to form a 5- to 7-membered ring optionally substituted with one or more substituents, wherein the ring atom at the position indicated by X³ is a N atom.

In some embodiments of the present disclosure, X² and X³ are linked to each other to form a 5- to 7-membered ring optionally substituted with one or more substituents, wherein the ring atom at the position indicated by X² is a C atom.

In some embodiments of the present disclosure, X² and X³ are linked to each other to form a 5- to 7-membered ring optionally substituted with one or more substituents, wherein the ring atom at the position indicated by X² is a C atom, and the ring atom at the position indicated by X³ is a N atom.

In some embodiments of the present disclosure, X² and X³ are linked to each other to form a 5- to 7-membered ring optionally substituted with one or more substituents, wherein the 5- to 7-membered ring is selected from the group consisting of: cyclopentenyl, cyclohexenyl, 2,5-dihydro-pyrrolyl, 2,3-dihydro-pyrrolyl, 2,5-dihydro-furanyl, 2,3-dihydro-furanyl, 2,5-dihydro-thienyl, 2,3-dihydro-thienyl, 1,2,3,4-tetrahydropyridinyl, 1,2,3,6-tetrahydropyridinyl, 3,4-dihydro-2*H-*pyranyl, 3,6-dihydro-2*H*-pyranyl, 3,4-dihydro-2*H*-thiopyranyl, 3,6-dihydro-2*H*-thiopyranyl, phenyl, pyrrolyl, pyrazolyl, imidazolyl, oxazolyl, isoxazolyl, thiazolyl, isothiazolyl, triazolyl, furanyl, thienyl, pyridinyl, pyrazinyl, pyrimidinyl, and pyridazinyl.

In some embodiments of the present disclosure, X² and X³ are linked to each other to form a 5- to 7-membered ring optionally substituted with one or more substituents, wherein the 5- to 7-membered ring is selected from the group consisting of: 1,2,3,4-tetrahydro-pyridinyl, cyclopentenyl, cyclohexenyl, 2,5-dihydro-pyrrolyl, 1,2,3,6-tetrahydropyridinyl, phenyl, pyrrolyl, pyrazolyl, imidazolyl, pyridinyl, and pyrimidinyl.

In some embodiments of the present disclosure, X² and X³ are linked to each other to form a 5- to 7-membered ring optionally substituted with one or more substituents selected from the group consisting of: halogen, -CN, -OH, -SH, -COOH, NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, - N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, - CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl.

In some embodiments of the present disclosure, X² and X³ are linked to each other to form a 5- to 7-membered ring optionally substituted with 1, 2, or 3 substituents selected from the group consisting of: halogen, OH, CN, and NH₂. In some embodiments of the present disclosure, X² is selected from C(R¹), X³ is selected from N(R⁷), and R¹ on X² and R⁷ on X³, together with the carbon atom and the nitrogen atom to which they are linked, form 5- to 7-membered heterocycloalkenyl or 5-membered heteroaryl optionally substituted with one or more substituents.

In some embodiments of the present disclosure, X² is selected from C(R¹), X³ is selected from C(R¹)₂, and R¹ on X² and one R¹ on X³, together with the carbon atoms to which they are linked, form C₅₋₇ cycloalkenyl or 5- to 7-membered heterocycloalkenyl optionally substituted with one or more substituents.

In some embodiments of the present disclosure, X² is selected from C(R¹), X³ is selected from C(R¹)₂, and R¹ on X² and two R¹ on X³, together with the carbon atoms to which they are linked, form phenyl or 5- to 6-membered heteroaryl optionally substituted with one or more substituents.

In some embodiments of the present disclosure, X² is selected from C(R¹), X³ is selected from N(R⁷), and R¹ on X² and R⁷ on X³, together with the carbon atom and the nitrogen atom to which they are linked, form 5- to 6-membered heterocycloalkenyl or 5-membered heteroaryl optionally substituted with one or more substituents.

In some embodiments of the present disclosure, X² is selected from C(R¹), X³ is selected from N(R⁷), and the substituent formed by R¹ on X² and R⁷ on X³ together with the carbon atom and the nitrogen atom to which they are linked is optionally substituted with one or more of the following substituents: halogen, -CN, -OH, -SH, -COOH, NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, or 3- to 12-membered heterocyclyl.

In some embodiments of the present disclosure, X² is selected from C(R¹), X³ is selected from N(R⁷), and R¹ on X² and R⁷ on X³, together with the carbon atom and the nitrogen atom to which they are linked, form 5- to 6-membered heterocycloalkenyl or 5-membered heteroaryl optionally substituted with 1, 2, or 3 groups selected from the group consisting of halogen, OH, CN, and NH₂.

In some embodiments of the present disclosure, X² is selected from C(R¹), X³ is selected from N(R⁷), and R¹ on X² and R⁷ on X³, together with the carbon atom and the nitrogen atom to which they are linked, form 2,3-dihydro-pyrrolyl, 1,2,3,4-tetrahydro-pyridinyl, 1,2-dihydro-pyridinyl, 1,4-dihydro-pyridinyl, pyrrolyl, pyrazolyl, or imidazolyl optionally substituted with 1, 2, or 3 groups selected from the group consisting of halogen, OH, CN, and NH₂.

In some embodiments of the present disclosure, X² is selected from C(R¹), X³ is selected from N(R⁷), and R¹ on X² and R⁷ on X³, together with the carbon atom and the nitrogen atom to which they are linked, form 1,2,3,4-tetrahydro-pyridinyl optionally substituted with 1, 2, or 3 groups selected from the group consisting of halogen, OH, CN, and NH₂.

In some embodiments of the present disclosure, X² is selected from C(R¹), X³ is selected from C(R¹)₂, and R¹ on X² and one R¹ on X³, together with the carbon atoms to which they are linked, form C₅₋₆ cycloalkenyl or 5- to 6-membered heterocycloalkenyl optionally substituted with one or more substituents.

In some embodiments of the present disclosure, X² is selected from C(R¹), X³ is selected from C(R¹)₂, and the substituent formed by R¹ on X² and one R¹ on X³ together with the carbon atoms to which they are linked is optionally substituted with one or more of the following substituents: halogen, -CN, -OH, -SH, -COOH, NH₂, - NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, or 3- to 12-membered heterocyclyl.

In some embodiments of the present disclosure, X² is selected from C(R¹), X³ is selected from C(R¹)₂, and R¹ on X² and one R¹ on X³, together with the carbon atoms to which they are linked, form C₅₋₆ cycloalkenyl or 5- to 6-membered heterocycloalkenyl optionally substituted with 1, 2, or 3 groups selected from the group consisting of halogen, OH, CN, and NH₂.

In some embodiments of the present disclosure, X² is selected from C(R¹), X³ is selected from C(R¹)₂, and R¹ on X² and one R¹ on X³, together with the carbon atoms to which they are linked, form cyclopentenyl, cyclohexenyl, 2,5-dihydro-pyrrolyl, 2,3-dihydro-pyrrolyl, 2,5-dihydro-furanyl, 2,3-dihydro-furanyl, 2,5-dihydro-thienyl, 2,3-dihydro-thienyl, 1,2,3,4-tetrahydropyridinyl, 1,2,3,6-tetrahydropyridinyl, 3,4-dihydro-2*H*-pyranyl, 3,6-dihydro-2*H*-pyranyl, 3,4-dihydro-2*H*-thiopyranyl, or 3,6-dihydro-2*H*-thiopyranyl optionally substituted with 1, 2, or 3 groups selected from the group consisting of halogen, OH, CN, and NH₂.

In some embodiments of the present disclosure, X² is selected from C(R¹), X³ is selected from C(R¹)₂, and R¹ on X² and one R¹ on X³, together with the carbon atoms to which they are linked, form cyclopentenyl, cyclohexenyl, 2,5-dihydro-pyrrolyl, or 1,2,3,6-tetrahydropyridinyl optionally substituted with 1, 2, or 3 groups selected from the group consisting of halogen, OH, CN, and NH₂.

In some embodiments of the present disclosure, X² is selected from C(R¹), X³ is selected from C(R¹)₂, and R¹ on X² and two R¹ on X³, together with the carbon atoms to which they are linked, form phenyl or 5- to 6-membered heteroaryl optionally substituted with one or more substituents; the substituent is selected from the group consisting of the following substituents: halogen, -CN, -OH, -SH, -COOH, NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, - OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl.

In some embodiments of the present disclosure, X² is selected from C(R¹), X³ is selected from C(R¹)₂, and R¹ on X² and two R¹ on X³, together with the carbon atoms to which they are linked, form phenyl, pyrrolyl, pyrazolyl, imidazolyl, oxazolyl, isoxazolyl, thiazolyl, isothiazolyl, triazolyl, furanyl, thienyl, pyridinyl, pyrazinyl, pyrimidinyl, or pyridazinyl optionally substituted with 1, 2, or 3 groups selected from the group consisting of halogen, OH, CN, and NH₂.

In some embodiments of the present disclosure, X² is selected from C(R¹), X³ is selected from C(R¹)₂, and R¹ on X² and two R¹ on X³, together with the carbon atoms to which they are linked, form phenyl, pyrrolyl, pyrazolyl, imidazolyl, pyridinyl, or pyrimidinyl optionally substituted with 1, 2, or 3 groups selected from the group consisting of halogen, OH, CN, and NH₂.

In some embodiments of the present disclosure, X¹ is selected from C(F), X² is selected from CH, and X³ is selected from NH.

In some embodiments of the present disclosure, X¹ is selected from C(F), X² is selected from CH, and X³ is selected from N(NH₂).

In some embodiments of the present disclosure, X¹ is selected from C(F), X² is selected from CH, and X³ is selected from N(CH₃).

In some embodiments of the present disclosure, X¹ is selected from C(F), X² is selected from CH, and X³ is selected from N(CH₂CH₃).

In some embodiments of the present disclosure, X¹ is selected from C(F), X² is selected from CH, and X³ is selected from

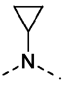

In some embodiments of the present disclosure, X¹ is selected from C(F), X² is selected from CH, and X³ is selected from N(CD₃).

In some embodiments of the present disclosure, X¹ is selected from C(F), X² is selected from CH, and X³ is selected from N(CD₂CD₃).

In some embodiments of the present disclosure, X¹ is selected from N, X² is selected from CH, and X³ is selected from S.

In some embodiments of the present disclosure, X¹ is selected from C(F), X² is selected from CH, and X³ is selected from C(=O).

In some embodiments of the present disclosure, X¹ is selected from CH, X² is selected from CH, and X³ is selected from S.

In some embodiments of the present disclosure, X¹ is selected from C(F), X² is selected from CH, and X³ is selected from S.

In some embodiments of the present disclosure, X¹ is selected from CH, X² is selected from CH, and X³ is selected from O.

In some embodiments of the present disclosure, X¹ is selected from C(F), X² is selected from CH, and X³ is selected from O.

In some embodiments of the present disclosure, the structural unit 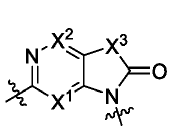 is

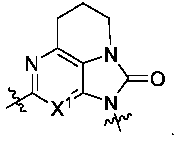

In some embodiments of the present disclosure, the structural unit 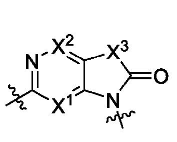 is

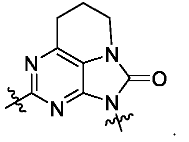

In some embodiments of the present disclosure, at least 2 of X⁴, X⁵, X⁶, and X⁷ are selected from C(R⁵).

In some embodiments of the present disclosure, X⁴ is selected from C(R⁵), and X⁵ is selected from C(R⁵).

In some embodiments of the present disclosure, X⁶ is selected from C(R⁵), and X⁷ is selected from C(R⁵).

In some embodiments of the present disclosure, X⁴ is selected from C(R⁵), X⁵ is selected from C(R⁵), X⁶ is selected from C(R⁵), and X⁷ is selected from C(R⁵).

In some embodiments of the present disclosure, X⁴ is selected from C(R⁵), X⁵ is selected from N, X⁶ is selected from C(R⁵), and X⁷ is selected from C(R⁵).

In some embodiments of the present disclosure, X⁴ is selected from N, X⁵ is selected from C(R⁵), X⁶ is selected from C(R⁵), and X⁷ is selected from C(R⁵).

In some embodiments of the present disclosure, X⁴ is selected from C(R⁵), X⁵ is selected from C(R⁵), X⁶ is selected from N, and X⁷ is selected from C(R⁵).

In some embodiments of the present disclosure, X⁴ is selected from C(R⁵), X⁵ is selected from C(R⁵), X⁶ is selected from C(R⁵), and X⁷ is selected from N.

In some embodiments of the present disclosure, X⁴ is selected from N, X⁵ is selected from C(R⁵), X⁶ is selected from N, and X⁷ is selected from C(R⁵).

In some embodiments of the present disclosure, X⁴ is selected from C(R⁵), X⁵ is selected from N, X⁶ is selected from C(R⁵), and X⁷ is selected from N.

In some other embodiments of the present disclosure, each R⁵ is independently selected from the group consisting of hydrogen, halogen, -CN, -OH, -NH₂, C₁₋₃ alkyl, C₁₋₃ deuterated alkyl, C₁₋₃ alkoxy, C₁₋₃ deuterated alkoxy, C₃₋₆ cycloalkyl, and 3- to 6-membered heterocyclyl, wherein the C₁₋₃ alkyl, C₁₋₃ deuterated alkyl, C₁₋₃ alkoxy, C₁₋₃ deuterated alkoxy, C₃₋₆ cycloalkyl, or 3- to 6-membered heterocyclyl is optionally independently substituted with one or more substituents.

In some embodiments of the present disclosure, each R⁵ is independently selected from the group consisting of hydrogen, -F, -Cl, -Br, -I, -CN, methyl, methoxy, monofluoromethyl, difluoromethyl, trifluoromethyl, monofluoromethoxy, difluoromethoxy, and trifluoromethoxy.

In some other embodiments of the present disclosure, each R⁵ is independently selected from the group consisting of hydrogen, -F, -Cl, -Br, -I, -CN, methyl, CD₃, methoxy, -OCD₃, monofluoromethyl, difluoromethyl, trifluoromethyl, monofluoromethoxy, difluoromethoxy, and trifluoromethoxy.

In some embodiments of the present disclosure, each R⁵ is independently selected from the group consisting of hydrogen and -F.

In some embodiments of the present disclosure, each R⁵ is independently selected from H.

In some embodiments of the present disclosure, X⁴ is selected from CH, and X⁵ is selected from CH.

In some embodiments of the present disclosure, X⁶ is selected from CH, and X⁷ is selected from CH.

In some embodiments of the present disclosure, X⁴ is selected from CH, X⁵ is selected from CH, X⁶ is selected from CH, and X⁷ is selected from CH.

In some embodiments of the present disclosure, X⁴ is selected from CH, X⁵ is selected from N, X⁶ is selected from CH, and X⁷ is selected from CH.

In some embodiments of the present disclosure, X⁴ is selected from CH, X⁵ is selected from CH, X⁶ is selected from CH, and X⁷ is selected from C(F).

In some embodiments of the present disclosure, X⁴ is selected from NH, X⁵ is selected from CH, X⁶ is selected from CH, and X⁷ is selected from CH.

In some embodiments of the present disclosure, X⁴ is selected from C(R⁵), X⁵ is selected from C(R⁵), and R⁵ groups on two carbon atoms of X⁴ and X⁵, together with the carbon atoms to which they are linked, form C₅₋₆ cycloalkenyl, 5- to 6-membered heterocycloalkenyl, phenyl, or 5- to 6-membered heteroaryl optionally substituted with one or more substituents.

In some embodiments of the present disclosure, X⁴ is selected from C(R⁵), X⁵ is selected from C(R⁵), and R⁵ groups on two carbon atoms of X⁴ and X⁵, together with the carbon atoms to which they are linked, form C₅₋₆ cycloalkenyl, 5- to 6-membered heterocycloalkenyl, phenyl, or 5- to 6-membered heteroaryl optionally substituted with 1, 2, or 3 groups selected from the group consisting of halogen, OH, CN, and NH₂.

In some embodiments of the present disclosure, X⁴ is selected from C(R⁵), X⁵ is selected from C(R⁵), and R⁵ groups on two carbon atoms of X⁴ and X⁵, together with the carbon atoms to which they are linked, form cyclopentenyl, cyclohexenyl, 2,5-dihydro-pyrrolyl, 2,3-dihydro-pyrrolyl, 2,5-dihydro-furanyl, 2,3-dihydro-furanyl, 2,5-dihydro-thienyl, 2,3-dihydro-thienyl, 1,2,3,4-tetrahydropyridinyl, 1,2,3,6-tetrahydropyridinyl, 3,4-dihydro-2*H*-pyranyl, 3,6-dihydro-2H-pyranyl, 3,4-dihydro-2*H*-thiopyranyl, 3,6-dihydro-2*H*-thiopyranyl, 3,4-dihydro-2*H*-1,4-oxazinyl, 3,4-dihydro-2H-1,4-thiazinyl, phenyl, pyrrolyl, pyrazolyl, imidazolyl, oxazolyl, isoxazolyl, thiazolyl, isothiazolyl, triazolyl, furanyl, thienyl, pyridinyl, pyrazinyl, pyrimidinyl, or pyridazinyl optionally substituted with 1, 2, or 3 groups selected from the group consisting of halogen, OH, CN, and NH₂.

In some embodiments of the present disclosure, X⁴ is selected from C(R⁵), X⁵ is selected from C(R⁵), and R⁵ groups on two carbon atoms of X⁴ and X³, together with the carbon atoms to which they are linked, form cyclopentenyl, cyclohexenyl, 2,5-dihydro-pyrrolyl, 2,3-dihydro-pyrrolyl, phenyl, pyrrolyl, pyrazolyl, pyridinyl, or pyrimidinyl optionally substituted with 1, 2, or 3 groups selected from the group consisting of halogen, OH, CN, and NH₂.

In some embodiments of the present disclosure, X⁴ is selected from C(R⁵), X⁵ is selected from C(R⁵), and R⁵ groups on two carbon atoms of X⁴ and X⁵, together with the carbon atoms to which they are linked, form C₅₋₆ cycloalkenyl, 5- to 6-membered heterocycloalkenyl, phenyl, or 5- to 6-membered heteroaryl optionally substituted with one or more substituents.

In some embodiments of the present disclosure, X⁴ is selected from C(R⁵), X⁵ is selected from C(R⁵), and R⁵ groups on two carbon atoms of X⁴ and X⁵, together with the carbon atoms to which they are linked, form C₅₋₆ cycloalkenyl, 5- to 6-membered heterocycloalkenyl, phenyl, or 5- to 6-membered heteroaryl optionally substituted with 1, 2, or 3 groups selected from the group consisting of halogen, OH, CN, and NH₂.

In some embodiments of the present disclosure, X⁶ is selected from C(R⁵), X⁷ is selected from C(R⁵), and R⁵ groups on two carbon atoms of X⁶ and X⁷, together with the carbon atoms to which they are linked, form cyclopentenyl, cyclohexenyl, 2,5-dihydro-pyrrolyl, 2,3-dihydro-pyrrolyl, 2,5-dihydro-furanyl, 2,3-dihydro-furanyl, 2,5-dihydro-thienyl, 2,3-dihydro-thienyl, 1,2,3,4-tetrahydropyridinyl, 1,2,3,6-tetrahydropyridinyl, 3,4-dihydro-2*H*-pyranyl, 3,6-dihydro-2*H-*pyranyl, 3,4-dihydro-2*H*-thiopyranyl, 3,6-dihydro-2*H*-thiopyranyl, 3,4-dihydro-2*H*-1,4-oxazinyl, 3,4-dihydro-2*H-*1,4-thiazinyl, phenyl, pyrrolyl, pyrazolyl, imidazolyl, oxazolyl, isoxazolyl, thiazolyl, isothiazolyl, triazolyl, furanyl, thienyl, pyridinyl, pyrazinyl, pyrimidinyl, or pyridazinyl optionally substituted with 1, 2, or 3 groups selected from the group consisting of halogen, OH, CN, and NH₂.

In some embodiments of the present disclosure, X⁶ is selected from C(R⁵), X⁷ is selected from C(R⁵), and R⁵ groups on two carbon atoms of X⁶ and X⁷, together with the carbon atoms to which they are linked, form cyclopentenyl, cyclohexenyl, 2,5-dihydro-pyrrolyl, 2,3-dihydro-pyrrolyl, phenyl, pyrrolyl, pyrazolyl, pyridinyl, or pyrimidinyl optionally substituted with 1, 2, or 3 groups selected from the group consisting of halogen, OH, CN, and NH₂.

In some embodiments of the present disclosure, L is selected from the group consisting of -(C(R³R⁴))ₘ-, -O-, -S-, and -N(R⁷)-.

In some embodiments of the present disclosure, each R⁷, R⁸, or R⁹ is independently selected from the group consisting of hydrogen, -NH₂, C₁₋₁₂ alkyl, C₁₋₁₂ alkoxy, C₃₋₁₂ cycloalkyl, and 3- to 12-membered heterocyclyl, wherein the -NH₂, C₁₋₁₂ alkyl, C₁₋₁₂ alkoxy, C₃₋₁₂ cycloalkyl, or 3- to 12-membered heterocyclyl is optionally substituted with one or more substituents.

In some embodiments of the present disclosure, each R⁷, R⁸, or R⁹ is independently selected from the group consisting of hydrogen, -NH₂, C₁₋₆ alkyl, C₁₋₆ alkoxy, C₃₋₆ cycloalkyl, and 3- to 6-membered heterocyclyl, wherein the -NH₂, C₁₋₆ alkyl, C₁₋₆ alkoxy, C₃₋₆ cycloalkyl, or 3- to 6-membered heterocyclyl is optionally substituted with one or more substituents.

In some other embodiments of the present disclosure, each R⁷, R⁸, or R⁹ is independently selected from the group consisting of hydrogen, -NH₂, C₁₋₆ deuterated alkyl, C₁₋₆ alkyl, C₁₋₆ alkoxy, C₁₋₆ deuterated alkoxy, C₃₋₆ cycloalkyl, and 3- to 6-membered heterocyclyl, wherein the -NH₂, C₁₋₆ deuterated alkyl, C₁₋₆ alkyl, C₁₋₆ alkoxy, C₁₋₆ deuterated alkoxy, C₃₋₆ cycloalkyl, or 3- to 6-membered heterocyclyl is optionally substituted with one or more substituents.

In some embodiments of the present disclosure, each R⁷, R⁸, or R⁹ is independently selected from the group consisting of hydrogen, -NH₂, C₁₋₃ alkyl, C₁₋₃ alkoxy, C₃₋₆ cycloalkyl, and 3- to 6-membered heterocyclyl, wherein the -NH₂, C₁₋₃ alkyl, C₁₋₃ alkoxy, C₃₋₆ cycloalkyl, or 3- to 6-membered heterocyclyl is optionally substituted with one or more substituents.

**In** some other embodiments of the present disclosure, each R⁷, R⁸, or R⁹ is independently selected from the group consisting of hydrogen, -NH₂, C₁₋₃ deuterated alkyl, C₁₋₃ alkyl, C₁₋₃ alkoxy, C₁₋₃ deuterated alkoxy, C₃₋₆ cycloalkyl, and 3- to 6-membered heterocyclyl, wherein the -NH₂, C₁₋₃ deuterated alkyl, C₁₋₃ alkyl, C₁₋₃ alkoxy, C₁₋₃ deuterated alkoxy, C₃₋₆ cycloalkyl, or 3- to 6-membered heterocyclyl is optionally substituted with one or more substituents.

**In** some embodiments of the present disclosure, each R⁷, R⁸, or R⁹ is independently selected from the group consisting of hydrogen, -NH₂, methyl, ethyl, isopropyl, methoxy, ethoxy, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, and isoxazolidinyl, wherein the - NH₂, methyl, ethyl, isopropyl, methoxy, ethoxy, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, or isoxazolidinyl is optionally independently substituted with one or more substituents.

**In** some other embodiments of the present disclosure, each R⁷, R⁸, or R⁹ is independently selected from the group consisting of hydrogen, -NH₂, methyl, -CD₃, ethyl, -CD₂CD₃, isopropyl, -CH(CD₃)₂, methoxy, -OCD₃, ethoxy, - OCD₂CD₃, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, and isoxazolidinyl, wherein the -NH₂, methyl, -CD₃, ethyl, -CD₂CD₃, isopropyl, - CH(CD₃)₂, methoxy, -OCD₃, ethoxy, -OCD₂CD₃, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, or isoxazolidinyl is optionally independently substituted with one or more substituents.

In some other embodiments of the present disclosure, each R⁷, R⁸, or R⁹ is optionally independently substituted with one or more groups selected from the group consisting of halogen, deuterium, OH, CN, and NH₂.

In some embodiments of the present disclosure, each R⁷, R⁸, or R⁹ is optionally independently substituted with one or more groups selected from the group consisting of halogen, OH, CN, and NH₂.

In some embodiments of the present disclosure, each R⁷, R⁸, or R⁹ is independently selected from the group consisting of hydrogen, -NH₂, methyl, ethyl, isopropyl, methoxy, ethoxy, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, and isoxazolidinyl, wherein the - NH₂, methyl, ethyl, isopropyl, methoxy, ethoxy, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, or isoxazolidinyl is optionally independently substituted with 1, 2, or 3 groups selected from the group consisting of halogen, OH, CN, and NH₂.

**In** some other embodiments of the present disclosure, each R⁷, R⁸, or R⁹ is independently selected from the group consisting of hydrogen, -NH₂, methyl, -CD₃, ethyl, -CD₂CD₃, isopropyl, -CH(CD₃)₂, methoxy, -OCD₃, ethoxy, - OCD₂CD₃, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, and isoxazolidinyl, wherein the -NH₂, methyl, -CD₃, ethyl, -CD₂CD₃, isopropyl, - CH(CD₃)₂, methoxy, -OCD₃, ethoxy, -OCD₂CD₃, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, or isoxazolidinyl is optionally independently substituted with 1, 2, or 3 groups selected from the group consisting of halogen, OH, CN, and NH₂.

In some embodiments of the present disclosure, each R⁷, R⁸, or R⁹ is independently selected from the group consisting of hydrogen, -NH₂, methyl, ethyl, isopropyl, methoxy, ethoxy, isopropoxy, cyclopropyl, cyclobutyl, oxetanyl, thietanyl, and azetidinyl, wherein the -NH₂, methyl, ethyl, isopropyl, methoxy, ethoxy, isopropoxy, cyclopropyl, cyclobutyl, oxetanyl, thietanyl, or azetidinyl is optionally independently substituted with 1, 2, or 3 groups selected from the group consisting of halogen, OH, CN, and NH₂.

In some other embodiments of the present disclosure, each R⁷, R⁸, or R⁹ is independently selected from the group consisting of hydrogen, -NH₂, methyl, -CD₃, ethyl, -CD₂CD₃, isopropyl, -CH(CD₃)₂, methoxy, -OCD₃, ethoxy, - OCD₂CD₃, isopropoxy, cyclopropyl, cyclobutyl, oxetanyl, thietanyl, and azetidinyl, wherein the -NH₂, methyl, - CD₃, ethyl, -CD₂CD₃, isopropyl, -CH(CD₃)₂, methoxy, -OCD₃, ethoxy, -OCD₂CD₃, isopropoxy, cyclopropyl, cyclobutyl, oxetanyl, thietanyl, or azetidinyl is optionally independently substituted with 1, 2, or 3 groups selected from the group consisting of halogen, OH, CN, and NH₂.

In some embodiments of the present disclosure, each R⁷, R⁸, or R⁹ is independently selected from the group consisting of hydrogen, -NH₂, methyl, ethyl, and cyclopropyl.

In some other embodiments of the present disclosure, each R⁷, R⁸, or R⁹ is independently selected from the group consisting of hydrogen, -NH₂, methyl, ethyl, -CD₂CD₃, cyclopropyl, and -CD₃.

In some other embodiments of the present disclosure, R⁷ is selected from the group consisting of -CD₃ and -CD₂CD₃. In some other embodiments of the present disclosure, R⁷ is selected from -CD₃.

In some other embodiments of the present disclosure, R⁷ is selected from the group consisting of hydrogen and methyl.

In some other embodiments of the present disclosure, R⁷ is selected from methyl.

In some embodiments of the present disclosure, each R⁷, R⁸, or R⁹ is selected from hydrogen.

In some embodiments of the present disclosure, L is selected from the group consisting of -(C(R³R⁴))ₘ-, -O-, -S-, and -NH-.

In some embodiments of the present disclosure, L is selected from the group consisting of -C(R³R⁴)- and -C(R³R⁴)-C(R³R⁴)-.

In some embodiments of the present disclosure, each R³ and R⁴ is independently selected from the group consisting of hydrogen, halogen, -CN, -OH, -SH, -COOH, -NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl, wherein the -OH, -SH, -NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, - NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, - CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more substituents.

In some embodiments of the present disclosure, each R³ and R⁴ is independently selected from the group consisting of hydrogen, halogen, -CN, -OH, -SH, -COOH, -NH₂, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ alkylthio, -COC₁₋₆ alkyl, -OC(O)C₁₋₆ alkyl, -C(O)OC₁₋₆ alkyl, -OC(O)OC₁₋₆ alkyl, -SO₂C₁₋₆ alkyl, -NHSO₂C₁₋₆ alkyl, -N(C₁₋₆ alkyl)SO₂C₁₋₆ alkyl, -SO₂NH₂, -SO₂NHC₁₋₆ alkyl, -SO₂N(C₁₋₆ alkyl)₂, -CONH₂, -CONHC₁₋₆ alkyl, -CON(C₁₋₆ alkyl)₂, -NHCOC₁₋₆ alkyl, -N(C₁₋₆ alkyl)COC₁₋₆ alkyl, C₃₋₈ cycloalkyl, C₃₋₈ cycloalkenyl, phenyl, 5- to 10-membered heteroaryl, and 3- to 8-membered heterocyclyl, wherein the -OH, -SH, - NH₂, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ alkylthio, -COC₁₋₆ alkyl, -OC(O)C₁₋₆ alkyl, -C(O)OC₁₋₆ alkyl, -OC(O)OC₁₋₆ alkyl, -SO₂C₁₋₆ alkyl, -NHSO₂C₁₋₆ alkyl, -N(C₁₋₆ alkyl)SO₂C₁₋₆ alkyl, -SO₂NH₂, -SO₂NHC₁₋₆ alkyl, -SO₂N(C₁₋₆ alkyl)₂, -CONH₂, -CONHC₁₋₆ alkyl, -CON(C₁₋₆ alkyl)₂, -NHCOC₁₋₆ alkyl, -N(C₁₋₆ alkyl)COC₁₋₆ alkyl, C₃₋₈ cycloalkyl, C₃₋₈ cycloalkenyl, phenyl, 5- to 10-membered heteroaryl, and 3- to 8-membered heterocyclyl are optionally independently substituted with one or more substituents.

In some embodiments of the present disclosure, each R³ and R⁴ is independently selected from the group consisting of hydrogen, halogen, -CN, -OH, -SH, -COOH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -COC₁₋₃ alkyl, -OC(O)C₁₋₃ alkyl, -C(O)OC₁₋₃ alkyl, -OC(O)OC₁₋₃ alkyl, -SO₂C₁₋₃ alkyl, -NHSO₂C₁₋₃ alkyl, -N(C₁₋₃ alkyl)SO₂C₁₋₃ alkyl, -SO₂NH₂, -SO₂NHC₁₋₃ alkyl, -SO₂N(C₁₋₃ alkyl)₂, -CONH₂, -CONHC₁₋₃ alkyl, -CON(C₁₋₃ alkyl)₂, -NHCOC₁₋₃ alkyl, -N(C₁₋₃ alkyl)COC₁₋₃ alkyl, C₃₋₆ cycloalkyl, C₃₋₆ cycloalkenyl, phenyl, 5- to 6-membered heteroaryl, and 3- to 6-membered heterocyclyl, wherein the -OH, -SH, - NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -COC₁₋₃ alkyl, -OC(O)C₁₋₃ alkyl, -C(O)OC₁₋₃ alkyl, -OC(O)OC₁₋₃ alkyl, -SO₂C₁₋₃ alkyl, -NHSO₂C₁₋₃ alkyl, -N(C₁₋₃ alkyl)SO₂C₁₋₃ alkyl, -SO₂NH₂, -SO₂NHC₁₋₃ alkyl, -SO₂N(C₁₋₃ alkyl)₂, -CONH₂, -CONHC₁₋₃ alkyl, -CON(C₁₋₃ alkyl)₂, -NHCOC₁₋₃ alkyl, -N(C₁₋₃ alkyl)COC₁₋₃ alkyl, C₃₋₆ cycloalkyl, C₃₋₆ cycloalkenyl, phenyl, 5- to 6-membered heteroaryl, and 3-to 6-membered heterocyclyl are optionally independently substituted with one or more substituents.

In some embodiments of the present disclosure, each R³ and R⁴ is independently selected from the group consisting of hydrogen, halogen, -CN, -OH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl, wherein the -OH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl are optionally independently substituted with one or more substituents.

In some other embodiments of the present disclosure, each R³ and R⁴ is independently selected from the group consisting of hydrogen, halogen, -CN, -OH, -NH₂, C₁₋₃ alkyl, C₁₋₃ deuterated alkyl, C₁₋₃ alkoxy, and C₁₋₃ deuterated alkoxy, wherein the C₁₋₃ alkyl, C₁₋₃ deuterated alkyl, C₁₋₃ alkoxy, or C₁₋₃ deuterated alkoxy is optionally independently substituted with one or more substituents.

In some embodiments of the present disclosure, each R³ and R⁴ is independently selected from the group consisting of hydrogen, halogen, -CN, -OH, -NH₂, C₁₋₃ alkyl, and C₁₋₃ alkoxy, wherein the C₁₋₃ alkyl and C₁₋₃ alkoxy are optionally independently substituted with one or more substituents.

In some embodiments of the present disclosure, each R³ and R⁴ is independently selected from the group consisting of hydrogen, halogen, -CN, -OH, -NH₂, methyl, ethyl, isopropyl, methoxy, ethoxy, and isopropoxy, wherein the methyl, ethyl, isopropyl, methoxy, ethoxy, and isopropoxy are optionally independently substituted with one or more substituents.

**In** some other embodiments of the present disclosure, each R³ and R⁴ is optionally independently substituted with one or more groups selected from the group consisting of halogen, deuterium, OH, CN, and NH₂.

**In** some embodiments of the present disclosure, each R³ and R⁴ is optionally independently substituted with one or more groups selected from the group consisting of halogen, OH, CN, and NH₂.

**In** some embodiments of the present disclosure, each R³ and R⁴ is independently selected from the group consisting of hydrogen, halogen, -CN, -OH, -NH₂, methyl, ethyl, isopropyl, methoxy, ethoxy, and isopropoxy, wherein the methyl, ethyl, isopropyl, methoxy, ethoxy, and isopropoxy are optionally independently substituted with 1, 2, or 3 groups selected from the group consisting of halogen, OH, CN, and NH₂.

**In** some embodiments of the present disclosure, each R³ and R⁴ is independently selected from the group consisting of hydrogen, -F, -Cl, -Br, -I, -CN, methyl, and ethyl.

In some embodiments of the present disclosure, each R³ is independently selected from hydrogen, and each R⁴ is independently selected from the group consisting of hydrogen, -F, -Cl, -Br, -I, -CN, methyl, and ethyl.

In some embodiments of the present disclosure, each R³ is independently selected from the group consisting of hydrogen, -F, -Cl, -Br, -I, -CN, methyl, and ethyl, and each R⁴ is independently selected from hydrogen.

In some embodiments of the present disclosure, each R³ is independently selected from hydrogen, and each R⁴ is independently selected from the group consisting of hydrogen, methyl, and ethyl.

In some embodiments of the present disclosure, each R³ is independently selected from the group consisting of hydrogen, methyl, and ethyl, and each R⁴ is independently selected from hydrogen.

In some embodiments of the present disclosure, each R³ is independently selected from methyl, and each R⁴ is independently selected from methyl; or each R³ is independently selected from hydrogen, and each R⁴ is independently selected from hydrogen; or each R³ is independently selected from hydrogen, and each R⁴ is independently selected from methyl; each R³ is independently selected from methyl, and each R⁴ is independently selected from hydrogen; or each R³ is independently selected from hydrogen, and each R⁴ is independently selected from ethyl; or each R³ is independently selected from ethyl, and each R⁴ is independently selected from hydrogen. In some embodiments of the present disclosure, R³ and R⁴, together with the carbon atom to which they are linked, form cyclopropyl, cyclobutyl, cyclopentyl, cyclohexyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, isoxazolidinyl, piperidinyl, piperazinyl, or morpholinyl optionally substituted with one or more substituents.

In some embodiments of the present disclosure, R³ and R⁴, together with the carbon atom to which they are linked, form cyclopropyl, cyclobutyl, oxetanyl, thietanyl, or azetidinyl optionally substituted with one or more substituents. In some embodiments of the present disclosure, R³ and R⁴, together with the carbon atom to which they are linked, form cyclopropyl, cyclobutyl, oxetanyl, thietanyl, or azetidinyl optionally substituted with 1, 2, or 3 groups selected from the group consisting of halogen, OH, CN, and NH₂.

In some embodiments of the present disclosure, the substituent formed by R³ and R⁴ together with the carbon atom to which they are linked is optionally substituted with 1, 2, or 3 groups selected from the group consisting of halogen, OH, CN, and NH₂.

In some embodiments of the present disclosure, R³ and R⁴, together with the carbon atom to which they are linked, form cyclopropyl, oxetanyl, or azetidinyl optionally substituted with 1, 2, or 3 groups selected from the group consisting of halogen, OH, CN, and NH₂.

In some embodiments of the present disclosure, L is selected from -C(R³R⁴)-C(R³R⁴)-; R³ and R³, R³ and R⁴, R⁴ and R³, as well as R⁴ and R⁴ on two adjacent carbon atoms, together with the carbon atoms to which they are linked, form cyclopropyl, cyclobutyl, cyclopentyl, cyclohexyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, or isoxazolidinyl optionally substituted with one or more substituents.

In some embodiments of the present disclosure, L is selected from -C(R³R⁴)-C(R³R⁴)-; R³ and R³, R³ and R⁴, R⁴ and R³, as well as R⁴ and R⁴ on two adjacent carbon atoms, together with the carbon atoms to which they are linked, form cyclopropyl, cyclobutyl, oxetanyl, thietanyl, or azetidinyl optionally substituted with one or more substituents. In some embodiments of the present disclosure, L is selected from -C(R³R⁴)-C(R³R⁴)-; the substituent formed by R³ and R³, R³ and R⁴, R⁴ and R³, as well as R⁴ and R⁴ on two adjacent carbon atoms together with the carbon atoms to which they are linked is optionally substituted with 1, 2, or 3 groups selected from the group consisting of halogen, OH, CN, and NH₂.

In some embodiments of the present disclosure, L is selected from -C(R³R⁴)-C(R³R⁴)-; R³ and R³, R³ and R⁴, R⁴ and R³, as well as R⁴ and R⁴ on two adjacent carbon atoms, together with the carbon atoms to which they are linked, form cyclopropyl, cyclobutyl, oxetanyl, thietanyl, or azetidinyl optionally substituted with 1, 2, or 3 groups selected from the group consisting of halogen, OH, CN, and NH₂.

In some embodiments of the present disclosure, L is selected from -C(R³R⁴)-C(R³R⁴)-; R³ and R³, R³ and R⁴, R⁴ and R³, as well as R⁴ and R⁴ on two adjacent carbon atoms, together with the carbon atoms to which they are linked, form cyclobutyl, oxetanyl, or azetidinyl optionally substituted with 1, 2, or 3 groups selected from the group consisting of halogen, OH, CN, and NH₂.

In some embodiments of the present disclosure, L is selected from the group consisting of -O-, -S-, -NH-, -CH₂-, - CH(CH₃)-, -CH₂CH₂-, -CH(F)-, -CH(Cl)-, -CH(Br)-, -CH(I)-, -CH(CN)-, -CH(CF₃)-, -CH(OH)-, -CH(NH₂)-, - C(O)-, 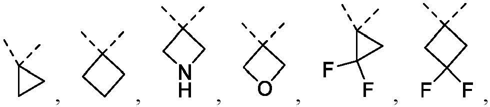 -CH(CH₃)CH₂-, -CH(F)CH₂-, -CH(Cl)CH₂-, - CH(Br)CH₂-, -CH(I)CH₂-, -CH(CN)CH₂-, -CH(CF₃)CH₂-, -CH(OH)CH₂-, -CH(NH₂)CH₂-,

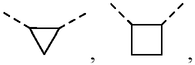

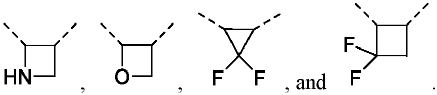

In some embodiments of the present disclosure, L is selected from the group consisting of -O-, -S-, -NH-, -CH₂-, - CH(CH₃)-, -CH₂CH₂-, -C(O)-,

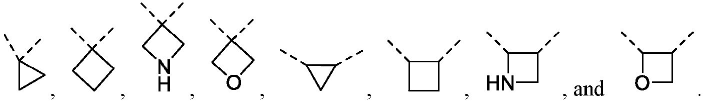

In some embodiments of the present disclosure, L is selected from the group consisting of -CH₂-, -CH(CH₃)-, and

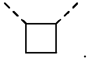

In some embodiments of the present disclosure, L is selected from -CH₂-.

In some embodiments of the present disclosure, when L is selected from the group consisting of -C(R³R⁴)- and - C(R³R⁴)-C(R³R⁴)-, each carbon atom may be a chiral carbon atom present in a form of a single (R) or (S) enantiomer or in a form enriched with one enantiomer.

In some embodiments of the present disclosure, R² is selected from the group consisting of C₃₋₈ cycloalkyl, phenyl, naphthyl, 5- to 10-membered heteroaryl, 3- to 8-membered heterocyclyl, and C₅₋₆ cycloalkenyl, wherein the C₃₋₈ cycloalkyl, phenyl, naphthyl, 5- to 10-membered heteroaryl, 3- to 8-membered heterocyclyl, and C₅₋₆ cycloalkenyl are optionally independently substituted with one or more R^{2a}.

In some embodiments of the present disclosure, R² is selected from the group consisting of C₃₋₆ cycloalkyl, phenyl, naphthyl, 5- to 9-membered heteroaryl, 3- to 6-membered heterocyclyl, and C₅₋₆ cycloalkenyl, wherein the C₃₋₆ cycloalkyl, phenyl, naphthyl, 5- to 9-membered heteroaryl, 3- to 6-membered heterocyclyl, and C₅₋₆ cycloalkenyl are optionally independently substituted with one or more R^{2a}.

In some embodiments of the present disclosure, R² is selected from the group consisting of C₅₋₆ cycloalkyl, phenyl, 5- to 6-membered heteroaryl, 5- to 6-membered heterocyclyl, and C₅₋₆ cycloalkenyl, wherein the C₅₋₆ cycloalkyl, phenyl, 5- to 6-membered heteroaryl, 5- to 6-membered heterocyclyl, and C₅₋₆ cycloalkenyl are optionally independently substituted with one or more R^{2a}.

In some embodiments of the present disclosure, R² is selected from the group consisting of C₅₋₆ cycloalkyl, phenyl, 5- to 6-membered heteroaryl containing 1-3 N atoms, 5- to 6-membered heterocyclyl containing 1-3 N atoms, and C₅₋₆ cycloalkenyl, wherein the C₅₋₆ cycloalkyl, phenyl, 5- to 6-membered heteroaryl containing 1-3 N atoms, 5- to 6-membered heterocyclyl containing 1-3 N atoms, and C₅₋₆ cycloalkenyl are optionally independently substituted with one or more R^{2a}.

In some embodiments of the present disclosure, R² is selected from the group consisting of cyclopentyl, cyclohexyl, phenyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, isoxazolidinyl, piperidinyl, piperazinyl, morpholinyl, pyrrolyl, pyrazolyl, imidazolyl, oxazolyl, isoxazolyl, thiazole ring, isothiazolyl, triazolyl, furanyl, thienyl, pyridinyl, pyrazinyl, pyrimidinyl, pyridazinyl, cyclopentenyl, and cyclohexenyl, wherein the cyclopentyl, cyclohexyl, phenyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, isoxazolidinyl, piperidinyl, piperazinyl, morpholinyl, pyrrolyl, pyrazolyl, imidazolyl, oxazolyl, isoxazolyl, thiazole ring, isothiazolyl, triazolyl, furanyl, thienyl, pyridinyl, pyrazinyl, pyrimidinyl, pyridazinyl, cyclopentenyl, and cyclohexenyl are optionally independently substituted with one or more R^{2a}.

In some embodiments of the present disclosure, R² is selected from the group consisting of phenyl, pyrrolyl, pyrazolyl, oxazolyl, isoxazolyl, thiazole ring, isothiazolyl, pyridinyl, pyrazinyl, pyrimidinyl, and pyridazinyl, wherein the phenyl, pyrrolyl, pyrazolyl, oxazolyl, isoxazolyl, thiazole ring, isothiazolyl, pyridinyl, pyrazinyl, pyrimidinyl, and pyridazinyl are optionally independently substituted with one or more R^{2a}.

In some embodiments of the present disclosure, R² is selected from the group consisting of phenyl, pyrazolyl, pyridinyl, and pyrimidinyl, wherein the phenyl, pyrazolyl, pyridinyl, and pyrimidinyl are optionally independently substituted with one or more R^{2a}.

In some embodiments of the present disclosure, R² is selected from pyrimidinyl, wherein the pyrimidinyl is optionally independently substituted with one or more R^{2a}.

In some embodiments of the present disclosure, R² is selected from the group consisting of 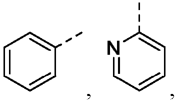 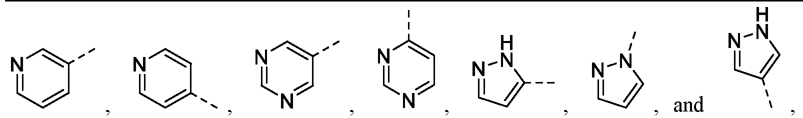 and R² is optionally independently substituted with one or more R^{2a}.

In some embodiments of the present disclosure, R² is selected from the group consisting of 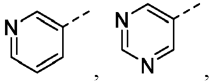 and 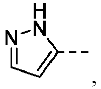 and R² is optionally independently substituted with one or more R^{2a}.

In some embodiments of the present disclosure, R² is selected from 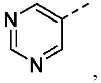 and R² is optionally independently substituted with one or more R^{2a}.

In some embodiments of the present disclosure, each R^{2a} and R^{6a} is independently selected from the group consisting of hydrogen, halogen, -CN, -OH, -SH, -COOH, -NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl, wherein the -OH, -SH, -NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, - NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, - CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more substituents.

In some embodiments of the present disclosure, each R^{2a} and R^{6a} is independently selected from the group consisting of halogen, -CN, -OH, -SH, -COOH, -NH₂, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ alkylthio, -COC₁₋₆ alkyl, -OC(O)C₁₋₆ alkyl, -C(O)OC₁₋₆ alkyl, -OC(O)OC₁₋₆ alkyl, -SO₂C₁₋₆ alkyl, - NHSO₂C₁₋₆ alkyl, -N(C₁₋₆ alkyl)SO₂C₁₋₆ alkyl, -SO₂NH₂, -SO₂NHC₁₋₆ alkyl, -SO₂N(C₁₋₆ alkyl)₂, -CONH₂, - CONHC₁₋₆ alkyl, -CON(C₁₋₆ alkyl)₂, -NHCOC₁₋₆ alkyl, -N(C₁₋₆ alkyl)COC₁₋₆ alkyl, C₃₋₈ cycloalkyl, C₃₋₈ cycloalkenyl, phenyl, 5- to 10-membered heteroaryl, and 3- to 8-membered heterocyclyl, wherein the -OH, -SH, - NH₂, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ alkylthio, -COC₁₋₆ alkyl, -OC(O)C₁₋₆ alkyl, -C(O)OC₁₋₆ alkyl, -OC(O)OC₁₋₆ alkyl, -SO₂C₁₋₆ alkyl, -NHSO₂C₁₋₆ alkyl, -N(C₁₋₆ alkyl)SO₂C₁₋₆ alkyl, -SO₂NH₂, -SO₂NHC₁₋₆ alkyl, -SO₂N(C₁₋₆ alkyl)₂, -CONH₂, -CONHC₁₋₆ alkyl, -CON(C₁₋₆ alkyl)₂, -NHCOC₁₋₆ alkyl, -N(C₁₋₆ alkyl)COC₁₋₆ alkyl, C₃₋₈ cycloalkyl, C₃₋₈ cycloalkenyl, phenyl, 5- to 10-membered heteroaryl, and 3- to 8-membered heterocyclyl are optionally independently substituted with one or more substituents.

In some embodiments of the present disclosure, each R^{2a} and R^{6a} is independently selected from the group consisting of halogen, -CN, -OH, -SH, -COOH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -COC₁₋₃ alkyl, -OC(O)C₁₋₃ alkyl, -C(O)OC₁₋₃ alkyl, -OC(O)OC₁₋₃ alkyl, -SO₂C₁₋₃ alkyl, - NHSO₂C₁₋₃ alkyl, -N(C₁₋₃ alkyl)SO₂C₁₋₃ alkyl, -SO₂NH₂, -SO₂NHC₁₋₃ alkyl, -SO₂N(C₁₋₃ alkyl)₂, -CONH₂, - CONHC₁₋₃ alkyl, -CON(C₁₋₃ alkyl)₂, -NHCOC₁₋₃ alkyl, -N(C₁₋₃ alkyl)COC₁₋₃ alkyl, C₃₋₆ cycloalkyl, C₃₋₆ cycloalkenyl, phenyl, 5- to 6-membered heteroaryl, and 3- to 6-membered heterocyclyl, wherein the -OH, -SH, - NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -COC₁₋₃ alkyl, -OC(O)C₁₋₃ alkyl, -C(O)OC₁₋₃ alkyl, -OC(O)OC₁₋₃ alkyl, -SO₂C₁₋₃ alkyl, -NHSO₂C₁₋₃ alkyl, -N(C₁₋₃ alkyl)SO₂C₁₋₃ alkyl, -SO₂NH₂, -SO₂NHC₁₋₃ alkyl, -SO₂N(C₁₋₃ alkyl)₂, -CONH₂, -CONHC₁₋₃ alkyl, -CON(C₁₋₃ alkyl)₂, -NHCOC₁₋₃ alkyl, -N(C₁₋₃ alkyl)COC₁₋₃ alkyl, C₃₋₆ cycloalkyl, C₃₋₆ cycloalkenyl, phenyl, 5- to 6-membered heteroaryl, and 3-to 6-membered heterocyclyl are optionally independently substituted with one or more substituents.

In some embodiments of the present disclosure, each R^{2a} and R^{6a} is independently selected from the group consisting of halogen, -CN, -OH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl, wherein the -OH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl are optionally independently substituted with one or more substituents.

In some other embodiments of the present disclosure, each R^{2a} and R^{6a} is independently selected from the group consisting of halogen, -CN, -OH, -NH₂, C₁₋₃ alkyl, C₁₋₃ deuterated alkyl, C₁₋₃ alkoxy, C₁₋₃ deuterated alkoxy, C₃₋₆ cycloalkyl, and 3- to 6-membered heterocyclyl, wherein the C₁₋₃ alkyl, C₁₋₃ deuterated alkyl, C₁₋₃ alkoxy, C₁₋₃ deuterated alkoxy, C₃₋₆ cycloalkyl, or 3- to 6-membered heterocyclyl is optionally independently substituted with one or more substituents.

In some embodiments of the present disclosure, each R^{2a} and R^{6a} is independently selected from the group consisting of halogen, -CN, -OH, -NH₂, C₁₋₃ alkyl, C₁₋₃ alkoxy, C₃₋₆ cycloalkyl, and 3- to 6-membered heterocyclyl, wherein the C₁₋₃ alkyl, C₁₋₃ alkoxy, C₃₋₆ cycloalkyl, and 3- to 6-membered heterocyclyl are optionally independently substituted with one or more substituents.

In some embodiments of the present disclosure, each R^{2a} and R^{6a} is independently selected from the group consisting of halogen, -CN, -OH, -NH₂, methyl, ethyl, isopropyl, methoxy, ethoxy, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, and isoxazolidinyl, wherein the methyl, ethyl, isopropyl, methoxy, ethoxy, isopropoxy, cyclopropyl, cyclobutyl, cyclopentyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, and isoxazolidinyl are optionally independently substituted with one or more substituents.

In some embodiments of the present disclosure, each R^{2a} and R^{6a} is independently selected from the group consisting of halogen, -CN, -OH, -NH₂, methyl, ethyl, isopropyl, methoxy, ethoxy, isopropoxy, cyclopropyl, cyclobutyl, oxetanyl, and azetidinyl, wherein the methyl, ethyl, isopropyl, methoxy, ethoxy, isopropoxy, cyclopropyl, cyclobutyl, oxetanyl, and azetidinyl are optionally independently substituted with one or more substituents.

In some other embodiments of the present disclosure, each R^{2a} and R^{6a} is optionally independently substituted with one or more groups selected from the group consisting of halogen, deuterium, OH, CN, and NH₂.

In some embodiments of the present disclosure, each R^{2a} and R^{6a} is optionally independently substituted with 1, 2, or 3 groups selected from the group consisting of halogen, OH, CN, and NH₂.

In some embodiments of the present disclosure, R²" is selected from the group consisting of halogen, -CN, -OH,-NH₂, methyl, ethyl, isopropyl, methoxy, ethoxy, isopropoxy, cyclopropyl, cyclobutyl, oxetanyl, and azetidinyl, wherein the methyl, ethyl, isopropyl, methoxy, ethoxy, isopropoxy, cyclopropyl, cyclobutyl, oxetanyl, and azetidinyl are optionally independently substituted with 1, 2, or 3 groups selected from the group consisting of halogen, OH, CN, and NH₂.

In some embodiments of the present disclosure, R^{2a} is selected from the group consisting of halogen, methyl, ethyl, isopropyl, methoxy, ethoxy, isopropoxy, and cyclopropyl, wherein the methyl, ethyl, isopropyl, methoxy, ethoxy, isopropoxy, and cyclopropyl are optionally independently substituted with 1, 2, or 3 groups selected from the group consisting of halogen, OH, CN, and NH₂.

In some embodiments of the present disclosure, R^{2a} is selected from the group consisting of -F, -Cl, -Br, -I, methyl, isopropyl, methoxy, monofluoromethyl, difluoromethyl, trifluoromethyl, monofluoromethoxy, difluoromethoxy, trifluoromethoxy,

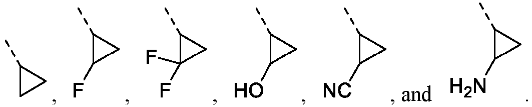

In some embodiments of the present disclosure, R^{2a} is selected from the group consisting of methyl, isopropyl, methoxy, and

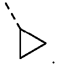

In some embodiments of the present disclosure, R^{2a} is selected from the group consisting of isopropyl, methoxy, and

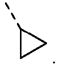

In some embodiments of the present disclosure, R² is selected from the group consisting of

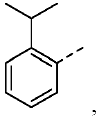

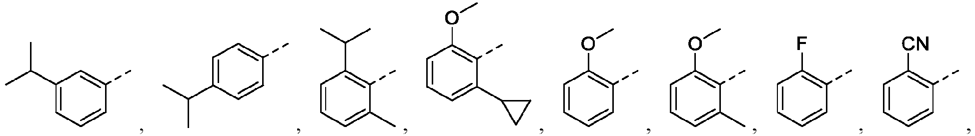

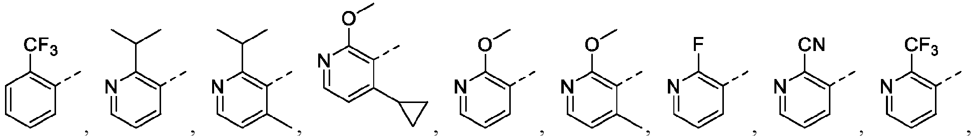

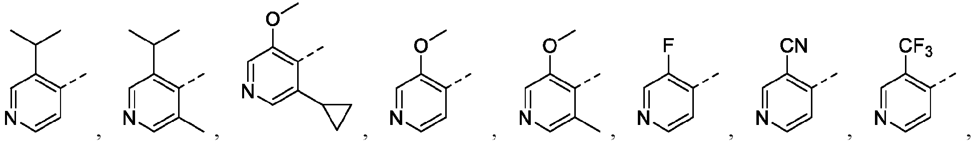

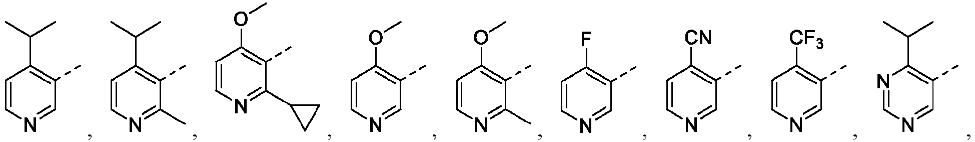

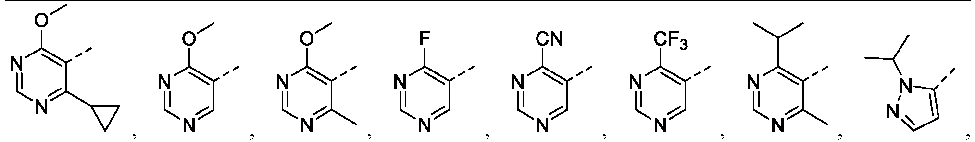

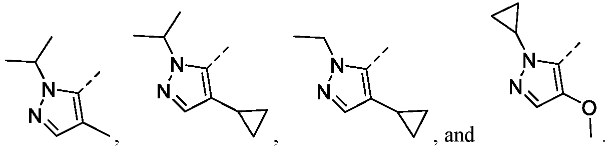

In some embodiments of the present disclosure, R² is selected from the group consisting of

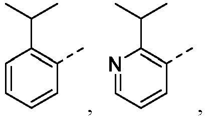

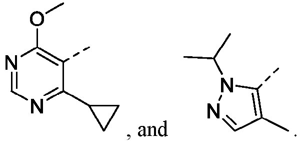

In some embodiments of the present disclosure, R² is selected from

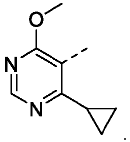

In some embodiments of the present disclosure, R⁶ is selected from the group consisting of hydrogen, halogen, - CN, -OH, -SH, -COOH, -NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, - NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, - CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl, wherein the -OH, - SH, -NH₂, -NHC₁₋₁₂ alkyl, -N(C₁₋₁₂ alkyl)₂, C₁₋₁₂ alkyl, C₂₋₁₂ alkenyl, C₂₋₁₂ alkynyl, C₁₋₁₂ alkoxy, C₁₋₁₂ alkylthio, - COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, - N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, - CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more R^{6a}.

In some embodiments of the present disclosure, R⁶ is selected from the group consisting of hydrogen, halogen, - CN, -OH, -SH, -COOH, -NH₂, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ alkylthio, -COC₁₋₆ alkyl, -OC(O)C₁₋₆ alkyl, -C(O)OC₁₋₆ alkyl, -OC(O)OC₁₋₆ alkyl, -SO₂C₁₋₆ alkyl, -NHSO₂C₁₋₆ alkyl, -N(C₁₋₆ alkyl)SO₂C₁₋₆ alkyl, -SO₂NH₂, -SO₂NHC₁₋₆ alkyl, -SO₂N(C₁₋₆ alkyl)₂, -CONH₂, -CONHC₁₋₆ alkyl, - CON(C₁₋₆ alkyl)₂, -NHCOC₁₋₆ alkyl, -N(C₁₋₆ alkyl)COC₁₋₆ alkyl, C₃₋₈ cycloalkyl, C₃₋₈ cycloalkenyl, phenyl, 5- to 10-membered heteroaryl, and 3- to 8-membered heterocyclyl, wherein the -OH, -SH, -NH₂, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ alkylthio, -COC₁₋₆ alkyl, -OC(O)C₁₋₆ alkyl, -C(O)OC₁₋₆ alkyl, -OC(O)OC₁₋₆ alkyl, -SO₂C₁₋₆ alkyl, -NHSO₂C₁₋₆ alkyl, -N(C₁₋₆ alkyl)SO₂C₁₋₆ alkyl, -SO₂NH₂, -SO₂NHC₁₋₆ alkyl, -SO₂N(C₁₋₆ alkyl)₂, -CONH₂, -CONHC₁₋₆ alkyl, -CON(C₁₋₆ alkyl)₂, -NHCOC₁₋₆ alkyl, -N(C₁₋₆ alkyl)COC₁₋₆ alkyl, C₃₋₈ cycloalkyl, C₃₋₈ cycloalkenyl, phenyl, 5- to 10-membered heteroaryl, and 3- to 8-membered heterocyclyl are optionally independently substituted with one or more R^{6a}.

In some embodiments of the present disclosure, R⁶ is selected from the group consisting of hydrogen, halogen, - CN, -OH, -SH, -COOH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -COC₁₋₃ alkyl, -OC(O)C₁₋₃ alkyl, -C(O)OC₁₋₃ alkyl, -OC(O)OC₁₋₃ alkyl, -SO₂C₁₋₃ alkyl, -NHSO₂C₁₋₃ alkyl, -N(C₁₋₃ alkyl)SO₂C₁₋₃ alkyl, -SO₂NH₂, -SO₂NHC₁₋₃ alkyl, -SO₂N(C₁₋₃ alkyl)₂, -CONH₂, -CONHC₁₋₃ alkyl, - CON(C₁₋₃ alkyl)₂, -NHCOC₁₋₃ alkyl, -N(C₁₋₃ alkyl)COC₁₋₃ alkyl, C₃₋₆ cycloalkyl, C₃₋₆ cycloalkenyl, phenyl, 5- to 6-membered heteroaryl, and 3- to 6-membered heterocyclyl, wherein the -OH, -SH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -COC₁₋₃ alkyl, -OC(O)C₁₋₃ alkyl, -C(O)OC₁₋₃ alkyl, -OC(O)OC₁₋₃ alkyl, -SO₂C₁₋₃ alkyl, -NHSO₂C₁₋₃ alkyl, -N(C₁₋₃ alkyl)SO₂C₁₋₃ alkyl, -SO₂NH₂, -SO₂NHC₁₋₃ alkyl, -SO₂N(C₁₋₃ alkyl)₂, -CONH₂, -CONHC₁₋₃ alkyl, -CON(C₁₋₃ alkyl)₂, -NHCOC₁₋₃ alkyl, -N(C₁₋₃ alkyl)COC₁₋₃ alkyl, C₃₋₆ cycloalkyl, C₃₋₆ cycloalkenyl, phenyl, 5- to 6-membered heteroaryl, and 3- to 6-membered heterocyclyl are optionally independently substituted with one or more R^{6a}.

In some embodiments of the present disclosure, R⁶ is selected from the group consisting of hydrogen, halogen, - CN, -OH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, - SO₂NH₂, -CONH₂, C₃₋₆ cycloalkyl, C₃₋₆ cycloalkenyl, phenyl, 5- to 6-membered heteroaryl, and 3- to 6-membered heterocyclyl, wherein the -OH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl are optionally independently substituted with one or more R^{6a}.

In some other embodiments of the present disclosure, R⁶ is selected from the group consisting of hydrogen, halogen, -CN, -OH, -NH₂, C₁₋₃ alkyl, C₁₋₃ deuterated alkyl, C₁₋₃ alkoxy, C₁₋₃ deuterated alkoxy, C₃₋₆ cycloalkyl, phenyl, 5- to 6-membered heteroaryl, and 3- to 6-membered heterocyclyl, wherein the C₁₋₃ alkyl, C₁₋₃ deuterated alkyl, C₁₋₃ alkoxy, C₁₋₃ deuterated alkoxy, C₃₋₆ cycloalkyl, phenyl, 5- to 6-membered heteroaryl, or 3- to 6-membered heterocyclyl is optionally independently substituted with one or more R^{6a}.

In some embodiments of the present disclosure, R⁶ is selected from the group consisting of hydrogen, halogen, - CN, -OH, -NH₂, C₁₋₃ alkyl, C₁₋₃ alkoxy, C₃₋₆ cycloalkyl, phenyl, 5- to 6-membered heteroaryl, and 3- to 6-membered heterocyclyl, wherein the C₁₋₃ alkyl, C₁₋₃ alkoxy, C₃₋₆ cycloalkyl, phenyl, 5- to 6-membered heteroaryl, and 3- to 6-membered heterocyclyl are optionally independently substituted with one or more R^{6a}.

In some embodiments of the present disclosure, R⁶ is selected from the group consisting of C₃₋₆ cycloalkyl, phenyl, 5- to 6-membered heteroaryl, and 3- to 6-membered heterocyclyl, wherein the C₃₋₆ cycloalkyl, phenyl, 5- to 6-membered heteroaryl, and 3- to 6-membered heterocyclyl are optionally independently substituted with one or more R^{6a}.

In some embodiments of the present disclosure, R⁶ is selected from the group consisting of C₃₋₆ cycloalkyl, phenyl, 5- to 6-membered heteroaryl containing 1-3 N atoms, and 5- to 6-membered heterocyclyl containing 1-3 N atoms, wherein the C₃₋₆ cycloalkyl, phenyl, 5- to 6-membered heteroaryl containing 1-3 N atoms, and 5- to 6-membered heterocyclyl containing 1-3 N atoms are optionally independently substituted with one or more R^{6a}.

In some embodiments of the present disclosure, R⁶ is selected from the group consisting of cyclopropyl, cyclobutyl, cyclopentyl, cyclohexyl, phenyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, isoxazolidinyl, piperidinyl, piperazinyl, morpholinyl, pyrrolyl, pyrazolyl, imidazolyl, oxazolyl, isoxazolyl, thiazole ring, isothiazolyl, triazolyl, furanyl, thienyl, pyridinyl, pyrazinyl, pyrimidinyl, and pyridazinyl, wherein the cyclopropyl, cyclobutyl, cyclopentyl, cyclohexyl, phenyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, isoxazolidinyl, piperidinyl, piperazinyl, morpholinyl, pyrrolyl, pyrazolyl, imidazolyl, oxazolyl, isoxazolyl, thiazole ring, isothiazolyl, triazolyl, furanyl, thienyl, pyridinyl, pyrazinyl, pyrimidinyl, and pyridazinyl are optionally independently substituted with one or more R^{6a}.

In some embodiments of the present disclosure, R⁶ is selected from the group consisting of phenyl, pyrrolyl, pyrazolyl, imidazolyl, oxazolyl, isoxazolyl, thiazole ring, isothiazolyl, triazolyl, pyridinyl, pyrazinyl, pyrimidinyl, and pyridazinyl, wherein the phenyl, pyrrolyl, pyrazolyl, imidazolyl, oxazolyl, isoxazolyl, thiazole ring, isothiazolyl, triazolyl, pyridinyl, pyrazinyl, pyrimidinyl, and pyridazinyl are optionally independently substituted with one or more R^{6a}.

In some embodiments of the present disclosure, R⁶ is selected from the group consisting of phenyl, pyrrolyl, pyrazolyl, imidazolyl, triazolyl, pyridinyl, and pyrimidinyl, wherein the phenyl, pyrrolyl, pyrazolyl, imidazolyl, triazolyl, pyridinyl, and pyrimidinyl are optionally independently substituted with one or more R^{6a}.

In some embodiments of the present disclosure, R⁶ is selected from the group consisting of pyrazolyl and imidazolyl, wherein the pyrazolyl and imidazolyl are optionally independently substituted with one or more R^{6a}.

In some embodiments of the present disclosure, R⁶ is selected from the group consisting of 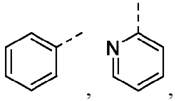 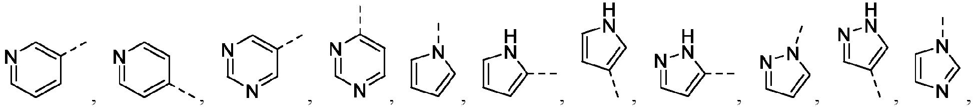 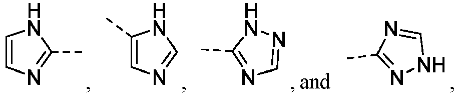 and R⁶ is optionally independently substituted with one or more R^{6a}.

In some embodiments of the present disclosure, R⁶ is selected from the group consisting of 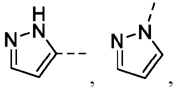 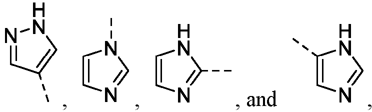 and R⁶ is optionally independently substituted with one or more R^{6a}.

In some embodiments of the present disclosure, R⁶ is selected from the group consisting of 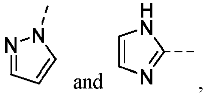 and R⁶ is optionally independently substituted with one or more R^{6a}.

In some embodiments of the present disclosure, R^{6a} is selected from the group consisting of halogen, -CN, -OH, - NH₂, methyl, ethyl, isopropyl, methoxy, ethoxy, isopropoxy, cyclopropyl, cyclobutyl, oxetanyl, and azetidinyl, wherein the methyl, ethyl, isopropyl, methoxy, ethoxy, isopropoxy, cyclopropyl, cyclobutyl, oxetanyl, and azetidinyl are optionally independently substituted with 1, 2, or 3 groups selected from the group consisting of halogen, OH, CN, and NH₂.

In some embodiments of the present disclosure, R^{6a} is selected from the group consisting of halogen, methyl, ethyl, isopropyl, methoxy, ethoxy, isopropoxy, cyclopropyl, and oxetanyl, wherein the methyl, ethyl, isopropyl, methoxy, ethoxy, isopropoxy, cyclopropyl, and oxetanyl are optionally independently substituted with 1, 2, or 3 groups selected from the group consisting of halogen, OH, CN, and NH₂.

In some other embodiments of the present disclosure, R^{6a} is selected from the group consisting of halogen, methyl, -CD₃, ethyl, -CD₂CD₃, isopropyl, -CH(CD₃)₂, methoxy, -OCD₃, ethoxy, -OCD₂CD₃, isopropoxy, cyclopropyl, and oxetanyl, wherein the methyl, -CD₃, ethyl, -CD₂CD₃, isopropyl, -CH(CD₃)₂, methoxy, -OCD₃, ethoxy, -OCD₂CD₃, isopropoxy, cyclopropyl, or oxetanyl is optionally independently substituted with 1, 2, or 3 groups selected from the group consisting of F, Cl, Br, I, OH, CN, and NH₂.

In some embodiments of the present disclosure, R^{6a} is selected from the group consisting of -F, -Cl, -Br, -I, methyl, isopropyl, methoxy, monofluoromethyl, difluoromethyl, trifluoromethyl, monofluoromethoxy, difluoromethoxy, trifluoromethoxy,

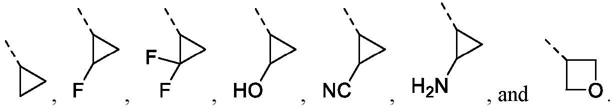

In some other embodiments of the present disclosure, R^{6a} is selected from the group consisting of -F, -Cl, -Br, -I, methyl, -CD₃, isopropyl, methoxy, -OCD₃, monofluoromethyl, difluoromethyl, trifluoromethyl, monofluoromethoxy, difluoromethoxy, trifluoromethoxy, -CH₂CF₃, -CF₂CF₃,

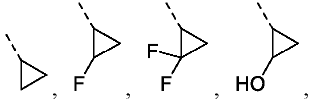

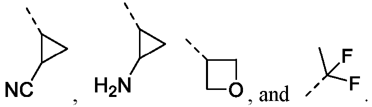

In some embodiments of the present disclosure, R^{6a} is selected from the group consisting of methyl, isopropyl, difluoromethyl, trifluoromethyl,

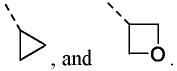

In some other embodiments of the present disclosure, R^{6a} is selected from the group consisting of methyl, -CD₃, isopropyl, difluoromethyl, trifluoromethyl,

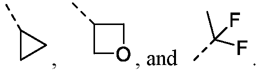

In some other embodiments of the present disclosure, R^{6a} is selected from the group consisting of methyl, isopropyl, difluoromethyl, trifluoromethyl,

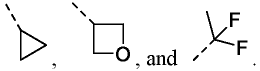

In some other embodiments of the present disclosure, R^{6a} is selected from the group consisting of methyl, isopropyl, and trifluoromethyl.

In some other embodiments of the present disclosure, R^{6a} is selected from the group consisting of methyl, isopropyl, trifluoromethyl, and

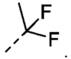

In some embodiments of the present disclosure, R⁶ is selected from the group consisting of 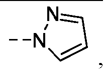 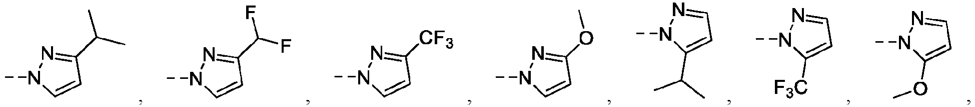 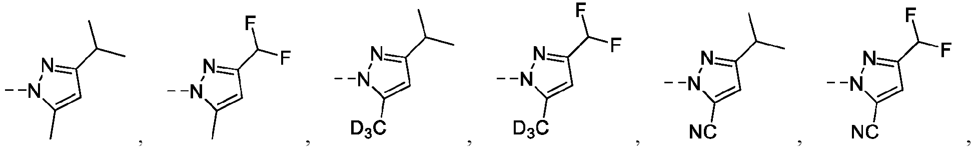 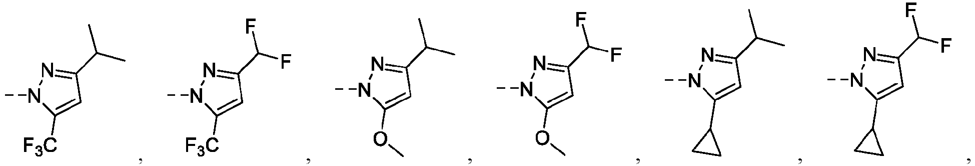 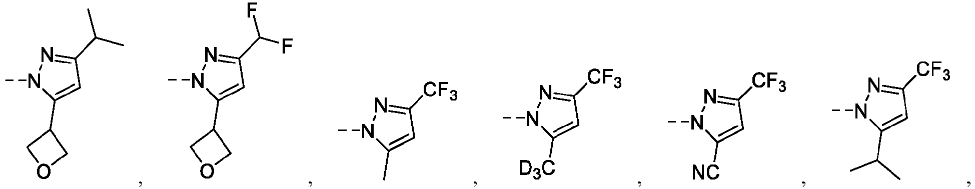 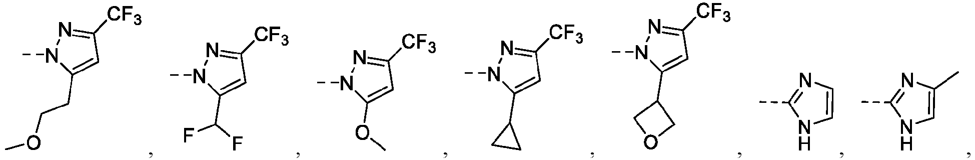 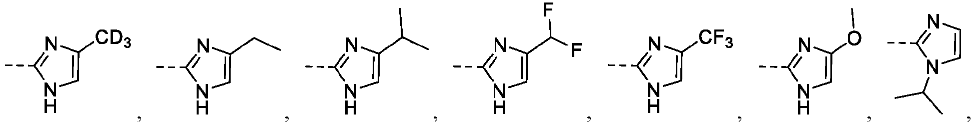 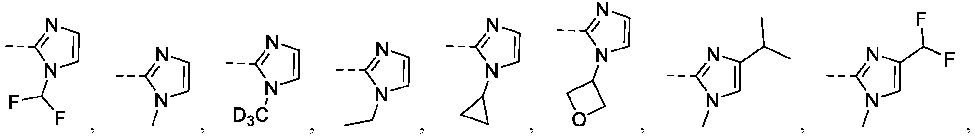 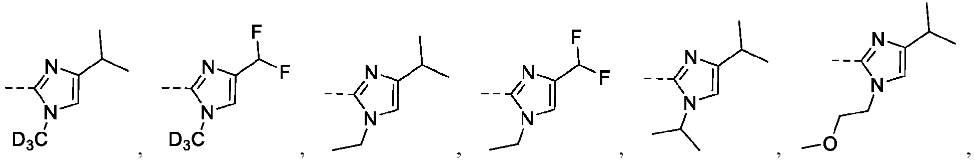 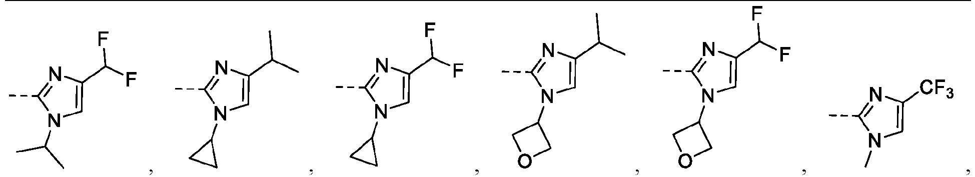 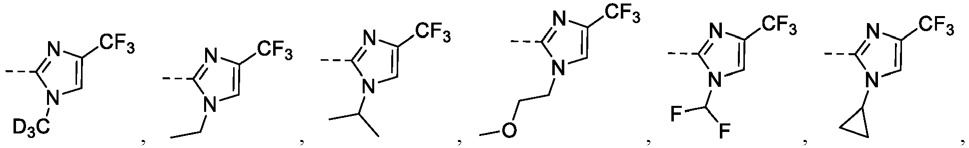 and

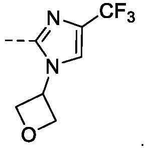

In some other embodiments of the present disclosure, R⁶ is selected from the group consisting of

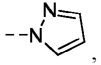

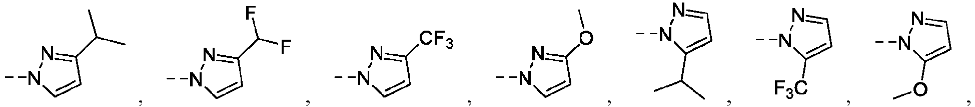

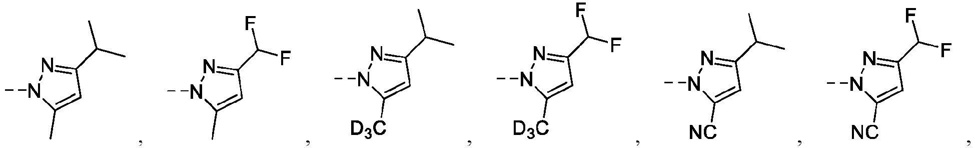

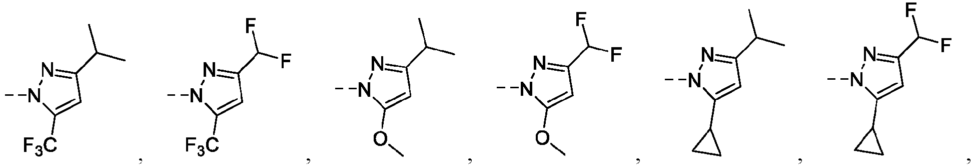

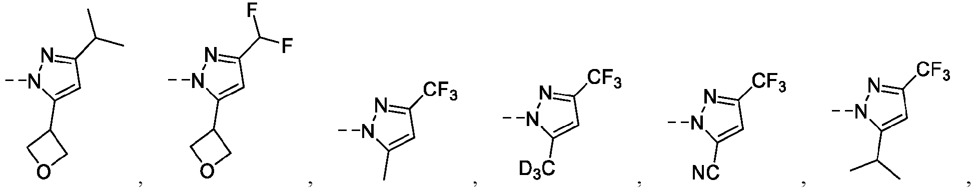

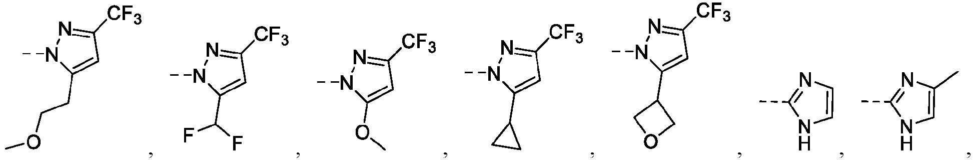

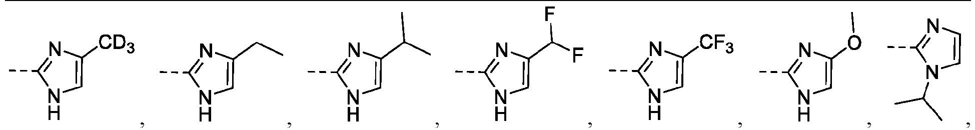

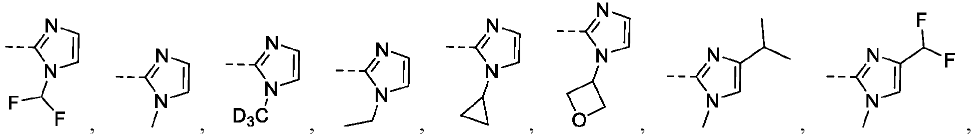

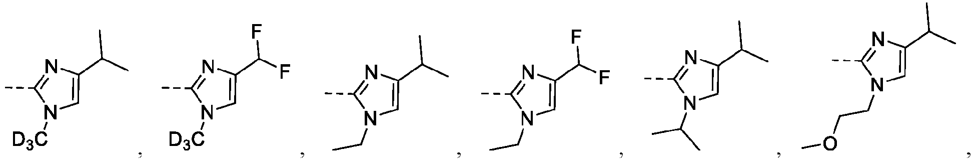

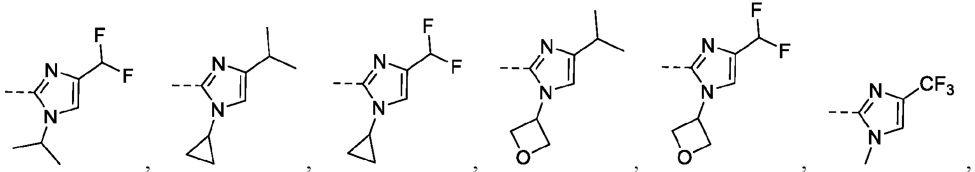

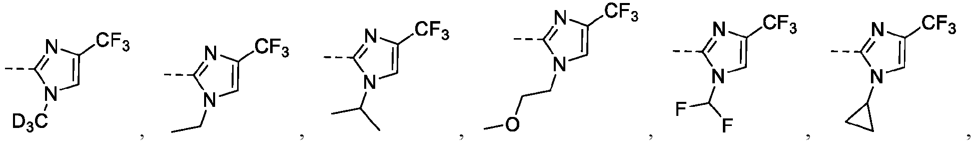

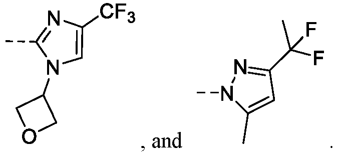

In some embodiments of the present disclosure, R⁶ is selected from the group consisting of

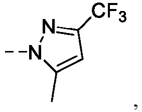

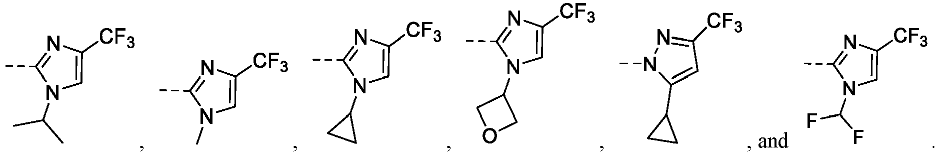

In some other embodiments of the present disclosure, R⁶ is selected from the group consisting of 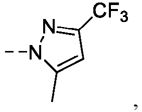 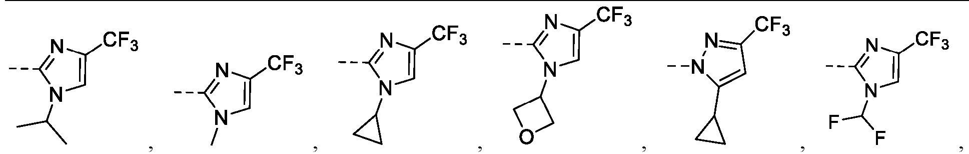 and

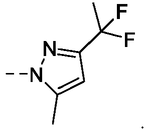

In some embodiments of the present disclosure, R⁶ is selected from the group consisting of

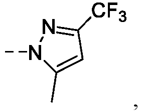

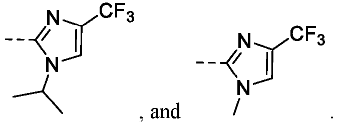

In some other embodiments of the present disclosure, R⁶ is selected from the group consisting of

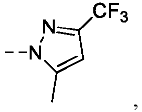

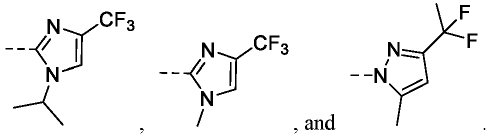

In some embodiments of the present disclosure, R⁶ is selected from

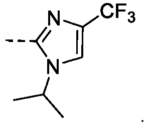

**In** some embodiments of the present disclosure, the "hetero" is each independently selected from the group consisting of heteroatoms of oxygen, sulfur, and nitrogen, wherein the nitrogen atom is optionally quaternized or oxidized to N(O), and the sulfur atom is optionally oxidized to S(O) or S(O)₂.

**In** some embodiments of the present disclosure, the "hetero" is each independently selected from the group consisting of heteroatoms of oxygen, sulfur, and nitrogen, wherein the sulfur heteroatom is optionally oxidized to S(O) or S(O)₂.

In some embodiments of the present disclosure, m is selected from 1.

In some embodiments of the present disclosure, provided is the compound of formula (I), the isomer thereof, or the pharmaceutically acceptable salt thereof, wherein the compound of formula (I) is selected from a compound of formula (I-1), 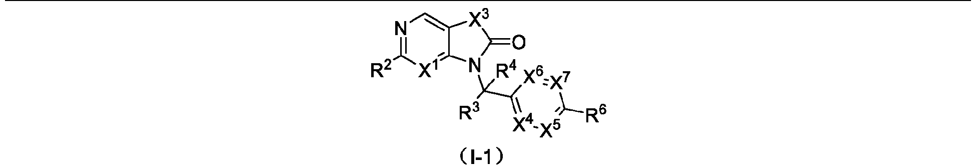
wherein X¹, X³, X⁴, X⁵, X⁶, X⁷, R², R³, R⁴, and R⁶ are as defined in the compound of formula (I);
the carbon atom to which R³ and R⁴ are linked may be a chiral carbon atom present in a form of a single (R) or (S) enantiomer or in a form enriched with one enantiomer.

In some embodiments of the present disclosure, provided is the compound of formula (I), the isomer thereof, or the pharmaceutically acceptable salt thereof, wherein the compound of formula (I) is selected from a compound of formula (I-2), 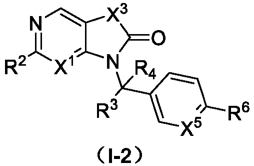 wherein,
X⁵ is selected from the group consisting of CH and N;
X¹, X³, R², R³, R⁴, and R⁶ are as defined in the compound of formula (I);
the carbon atom to which R³ and R⁴ are linked may be a chiral carbon atom present in a form of a single (R) or (S) enantiomer or in a form enriched with one enantiomer.

In some embodiments of the present disclosure, provided is the compound of formula (I), the isomer thereof, or the pharmaceutically acceptable salt thereof, wherein the compound of formula (I) is selected from a compound of formula (I-3), 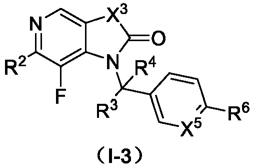 wherein,
X⁵ is selected from the group consisting of CH and N;
X³, R², R³, R⁴, and R⁶ are as defined in the compound of formula (I);
the carbon atom to which R³ and R⁴ are linked may be a chiral carbon atom present in a form of a single (R) or (S) enantiomer or in a form enriched with one enantiomer.

In some embodiments of the present disclosure, provided is the compound of formula (I), the isomer thereof, or the pharmaceutically acceptable salt thereof, wherein the compound of formula (I) is selected from a compound of formula (I-4), 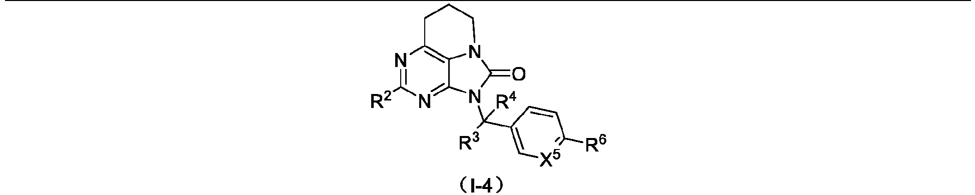 wherein,
X⁵ is selected from the group consisting of CH and N;
R², R³, R⁴, and R⁶ are as defined in the compound of formula (I);
the carbon atom to which R³ and R⁴ are linked may be a chiral carbon atom present in a form of a single (R) or (S) enantiomer or in a form enriched with one enantiomer.

In some embodiments of the present disclosure, provided is the compound of formula (I), the isomer thereof, or the pharmaceutically acceptable salt thereof, wherein the compound of formula (I) is selected from a compound of formula (I-5), 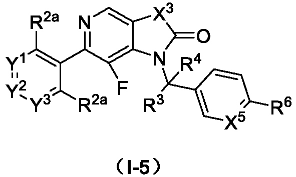 wherein,
X⁵ is selected from the group consisting of CH and N;
Y¹, Y², and Y³ are each independently selected from the group consisting of CH and N;
X³, R³, R⁴, R⁶, and R^{2a} are as defined in the compound of formula (I);
the carbon atom to which R³ and R⁴ are linked may be a chiral carbon atom present in a form of a single (R) or (S) enantiomer or in a form enriched with one enantiomer.

In some embodiments of the present disclosure, Y¹ is selected from N.

In some embodiments of the present disclosure, Y¹ and Y³ are both selected from N.

In some embodiments of the present disclosure, Y¹ is selected from N, and Y² and Y³ are both selected from CH. In some embodiments of the present disclosure, Y¹ and Y³ are both selected from N, and Y² is selected from CH. In some embodiments of the present disclosure, provided is the compound of formula (I), the isomer thereof, or the pharmaceutically acceptable salt thereof, wherein the compound of formula (I) is selected from a compound of formula (I-6), 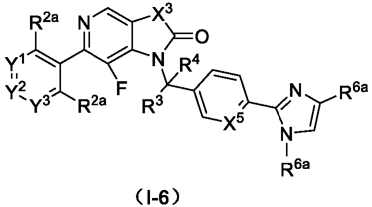 wherein,
X⁵ is selected from the group consisting of CH and N;
Y¹, Y², and Y³ are as defined in the compound of formula (I-5);
X³, R^{2a}, R³, R⁴, and R^{6a} are as defined in the compound of formula (I);
the carbon atom to which R³ and R⁴ are linked may be a chiral carbon atom present in a form of a single (R) or (S) enantiomer or in a form enriched with one enantiomer.

In some embodiments of the present disclosure, provided is the compound of formula (I), the isomer thereof, or the pharmaceutically acceptable salt thereof, wherein the compound of formula (I) is selected from a compound of formula (I-7), 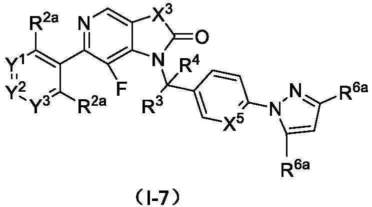 wherein,
X⁵ is selected from the group consisting of CH and N;
Y¹, Y², and Y³ are as defined in the compound of formula (I-5);
X³, R^{2a}, R³, R⁴, and R^{6a} are as defined in the compound of formula (I);
the carbon atom to which R³ and R⁴ are linked may be a chiral carbon atom present in a form of a single (R) or (S) enantiomer or in a form enriched with one enantiomer.

Some other embodiments of the present disclosure are derived from any combination of the variables.

In another aspect, the present disclosure further provides compounds of the formulas below, isomers thereof, or pharmaceutically acceptable salts thereof,

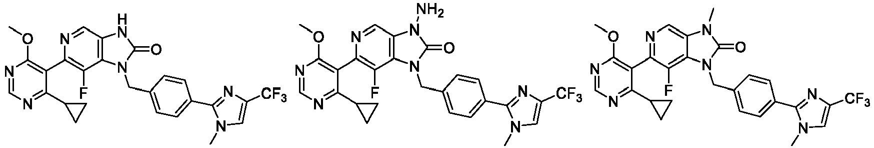

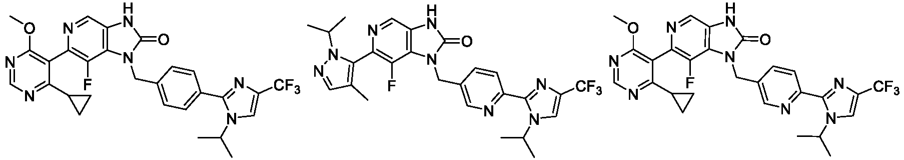

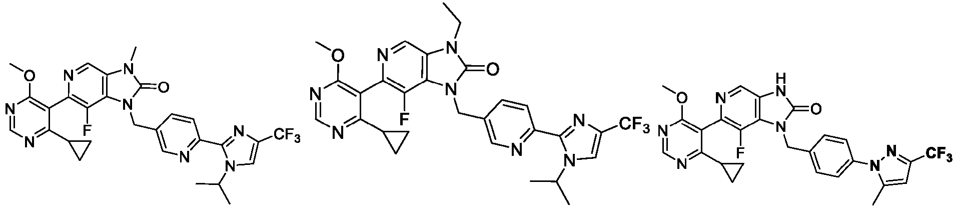

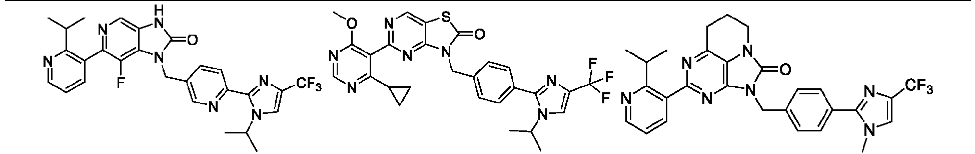

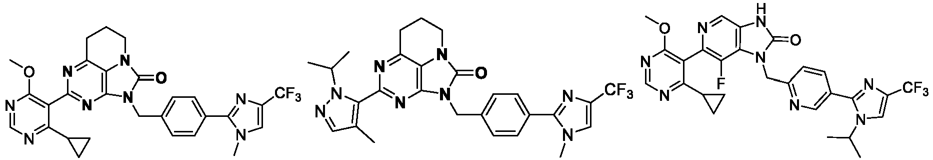

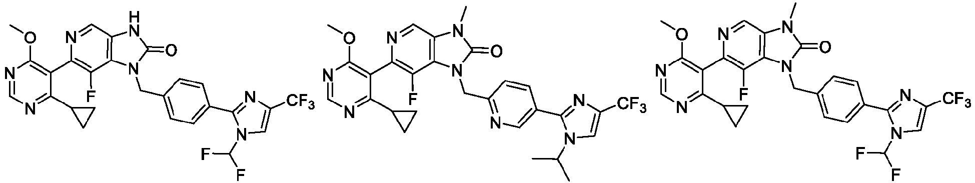

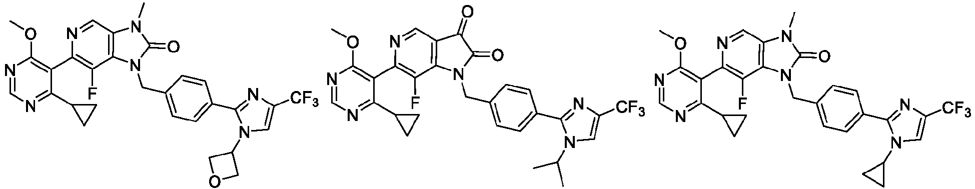

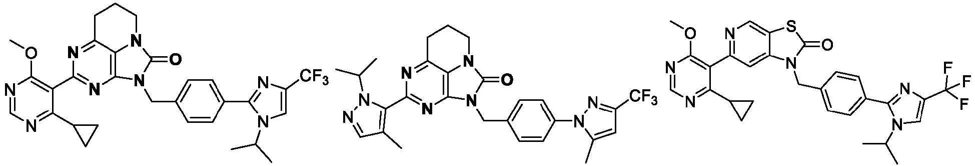

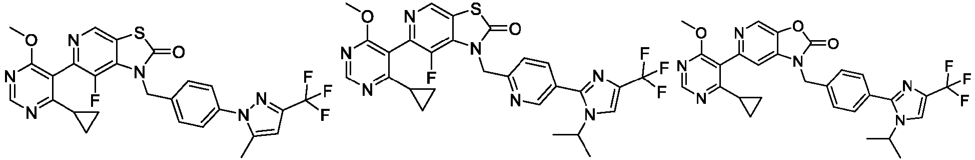

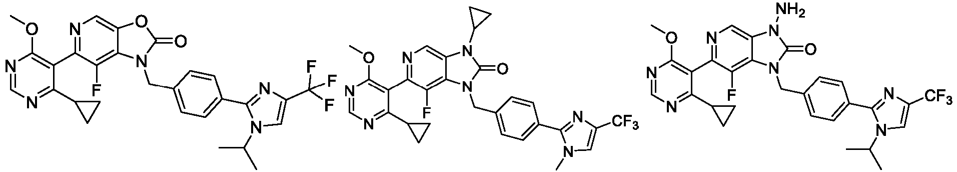

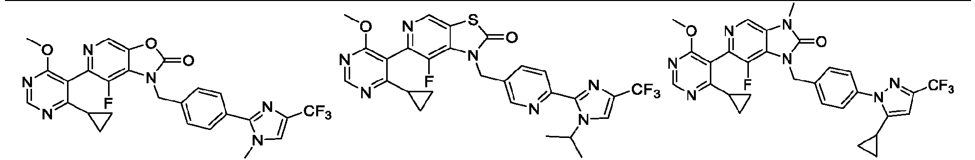

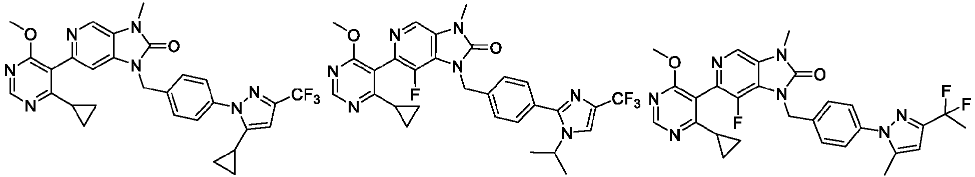

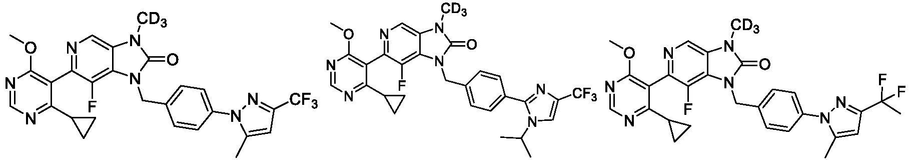

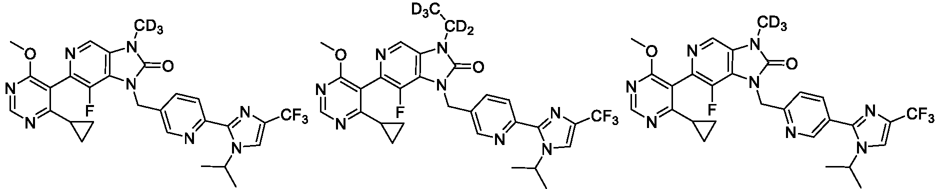

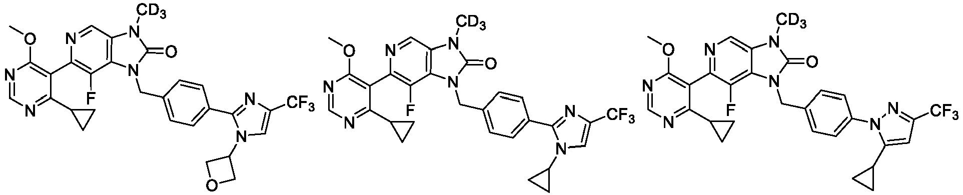

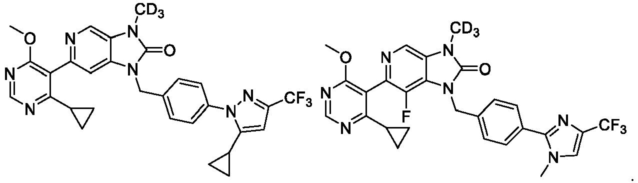

In another aspect, the present disclosure further provides a pharmaceutical composition comprising a therapeutically or prophylactically effective amount of the compound, the isomer thereof, or the pharmaceutically acceptable salt thereof described herein. In some embodiments, the pharmaceutical composition of the present disclosure further comprises a pharmaceutically acceptable excipient.

In another aspect, the present disclosure further provides use of the compound, the isomer thereof, or the pharmaceutically acceptable salt thereof, or the pharmaceutical composition thereof described herein for preparing a medicament for treating or preventing a disease associated with inhibition of ubiquitin specific peptidase 1 (USP1). In another aspect, the present disclosure further provides a method for treating or preventing a disease associated with inhibition of ubiquitin specific peptidase 1 (USP1), which comprises administering to a mammal (preferably a human) in need of the treatment or the prevention a therapeutically or prophylactically effective amount of the compound, the isomer thereof, or the pharmaceutically acceptable salt thereof, or the pharmaceutical composition thereof described herein.

In another aspect, the present disclosure further provides use of the compound, the isomer thereof, or the pharmaceutically acceptable salt thereof, or the pharmaceutical composition thereof described herein for treating or preventing a disease associated with inhibition of ubiquitin specific peptidase 1 (USP1).

In another aspect, the present disclosure further provides the compound, the isomer thereof, or the pharmaceutically acceptable salt thereof, or the pharmaceutical composition thereof described herein for use in treating or preventing a disease associated with inhibition of ubiquitin specific peptidase 1 (USP1).

In another aspect, in some embodiments of the present disclosure, the disease associated with the inhibition of ubiquitin specific peptidase 1 (USP1) is selected from the group consisting of a tumor and cancer (e.g., breast cancer).

### Technical effects

The compound in the present disclosure is used as an inhibitor of ubiquitin specific peptidase 1 (USP1) with a brand-new structure, and has a good inhibitory effect on signaling of ubiquitin specific peptidase 1 (USP1). The compound in the present disclosure has good *in-vivo* and *in-vitro* inhibitory effects, including but not limited to, good *in-vitro* inhibitory effects on the enzyme activity and cell activity, good liver microsome stability, good pharmacokinetic properties, a good *in-vivo* drug effect, and the like, and can be developed into a novel USP1 inhibitor drug.

### Definitions

Unless otherwise stated, the following terms and phrases used herein are intended to have the following meanings. A particular term or phrase, unless otherwise specifically defined, should not be considered indefinite or unclear, but construed according to its common meaning. When referring to a trade name, it is intended to refer to its corresponding commercial product or its active ingredient.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound of the present disclosure, which is prepared from the compound having particular substituents discovered by the present disclosure and a relatively nontoxic acid or base. When the compound of the present disclosure contains a relatively acidic functional group, a base addition salt can be obtained by contacting such a compound with a sufficient amount of a base in a pure solution or a suitable inert solvent. When the compound of the present disclosure contains a relatively basic functional group, an acid addition salt can be obtained by contacting such a compound with a sufficient amount of an acid in a pure solution or a suitable inert solvent. Certain specific compounds of the present disclosure contain both basic and acidic functional groups that allow the compounds to be converted into either base or acid addition salts.

The pharmaceutically acceptable salt of the present disclosure can be synthesized from a parent compound containing an acid radical or a basic group by conventional chemical methods. In general, such salts are prepared by subjecting the compounds in a free acid or base form to a reaction with a stoichiometric amount of appropriate base or acid in water or an organic solvent or a mixture thereof.

The compounds of the present disclosure may exist in the form of a specific geometric isomer or stereoisomer. All such compounds are contemplated herein, including *cis* and *trans* isomers, (-)- and (+)-enantiomers, (*R*)- and (*S*)-enantiomers, diastereoisomers, (*D*)-isomers, (*L*)-isomers, and racemic mixtures and other mixtures thereof, such as an enantiomer or diastereomer enriched mixture, all of which are encompassed within the scope of the present disclosure. The substituents such as alkyl may have an additional asymmetric carbon atom. All these isomers and mixtures thereof are encompassed within the scope of the present disclosure.

Unless otherwise specified, the absolute configuration of a stereogenic center is represented by a wedged solid bond ( 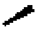 ) and a wedged dashed bond ( 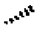 ), and the relative configuration of a stereogenic center is represented by a straight solid bond ( 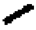 ) and a straight dashed bond ( 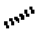 ).

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of a proton tautomer is an imidazole moiety where a proton can transfer between two ring nitrogen atoms. A valence tautomer includes the interconversion via recombination of some bonding electrons.

The compound of the present disclosure may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen can be substituted with deuterium to form a deuterated drug, and the bond formed by deuterium and carbon is firmer than that formed by common hydrogen and carbon. Compared with an un-deuterated drug, the deuterated drug has the advantages of reduced toxic side effects, increased stability, enhanced efficacy, prolonged biological half-life, and the like. All isotopic variations of the compound of the present disclosure, whether radioactive or not, are encompassed within the scope of the present disclosure.

The term "optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted with substituents, wherein the substituents may include deuterium and hydrogen variants, as long as the valence of the specific atom is normal and the substituted compound is stable. When the substituent is oxygen (i.e., =O), it means that two hydrogen atoms are substituted. Substitution with oxygen does not occur on aromatic groups. The term "optionally substituted" means that an atom can be or cannot be substituted with a substituent. Unless otherwise specified, the type and number of the substituent may be arbitrary as long as being chemically achievable.

The "substituent" described herein includes all substituents mentioned in the text, for example, including those defined by the related groups, such as the terms "C₁₋₁₂ alkyl", "C₁₋₁₂ alkoxy", "C₁₋₁₂ alkylthio", "C₂₋₁₂ alkenyl", "C₂₋₁₂ alkynyl", "halogen", "C₃₋₁₂ cycloalkyl", "C₃₋₁₂ cycloalkenyl", "3- to 6-membered heterocycloalkyl", "C₆₋₁₀ aryl", "5- to 10-membered heteroaryl", "3- to 12-membered heterocyclyl", and the like, and the corresponding non-limiting or exemplary groups mentioned below, wherein some non-limiting examples of the "substituent" include a deuterium atom, hydroxy, sulfydryl, halogen, amino, nitro, nitroso, cyano, azido, a sulfoxide group, a sulfone group, a sulfonamide group, carboxyl, a carboxaldehyde group, an imine group, alkyl, halogenated alkyl, cycloalkyl, halogenated cycloalkyl, alkenyl, halogenated alkenyl, cycloalkenyl, halogenated cycloalkenyl, alkynyl, halogenated alkynyl, cycloalkynyl, halogenated cycloalkynyl, heteroalkyl, halogenated heteroalkyl, alkoxy, alkylthio, aryl, aryloxy, arylthio, arylalkyl, arylalkoxy, arylalkylthio, heteroaryl, heteroaryloxy, heteroarylthio, heteroarylalkyl, heteroarylalkoxy, heteroarylalkylthio, heterocyclyl, heterocyclyloxy, heterocyclylthio, heterocyclylalkylene, heterocyclylalkoxy, heterocyclylthio, acyl, acyloxy, a carbamate group, an amide group, ureido, an epoxy group, an ester group, oxo, thio, and the like, wherein the groups are optionally substituted with one or more substituents selected from the group consisting of the following substituents: a deuterium atom, oxo, hydroxy, amino, nitro, halogen, cyano, alkyl, alkenyl, alkynyl, alkoxy, halogenated alkoxy, alkylamino, dialkylamino, halogenated alkylamino, halogenated dialkylamino, carboxyl, -C(O)O-alkyl, -OC(O)-alkyl, -C(O)NH₂, -C(O)NH-alkyl, - C(O)N(alkyl)₂, -NHC(O)-alkyl, -C(O)-alkyl, -S(O)-alkyl, -S(O)₂-alkyl, -S(O)₂NH₂, -S(O)₂NH-alkyl, - S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

In some embodiments herein, the substituent is selected from the group consisting of a deuterium atom, hydroxy, sulfydryl, halogen, amino, nitro, nitroso, cyano, azido, a sulfoxide group, a sulfone group, a sulfonamide group, carboxyl, an aldehyde group, an imine group, C₁₋₁₂ alkyl, halogenated C₁₋₁₂ alkyl, 3- to 12-membered cycloalkyl, halogenated 3- to 12-membered cycloalkyl, C₂₋₁₂ alkenyl, halogenated C₂₋₁₂ alkenyl, 3- to 12-membered cycloalkenyl, halogenated 3- to 12-membered cycloalkenyl, C₂₋₁₂ alkynyl, halogenated C₂₋₁₂ alkynyl, 8- to 12-membered cycloalkynyl, halogenated 8- to 12-membered cycloalkynyl, C₁₋₁₂ heteroalkyl, halogenated C₁₋₁₂ heteroalkyl, C₁₋₁₂ alkoxy, C₁₋₁₂ alkylthio, 6- to 10-membered aryl, 6- to 10-membered aryloxy, 6- to 10-membered arylthio, 6- to 10-membered aryl C₁₋₁₂ alkylene, 6- to 10-membered aryl C₁₋₁₂ alkoxy, 6- to 10-membered aryl C₁₋₁₂ alkylthio, 5- to 10-membered heteroaryl, 5- to 10-membered heteroaryloxy, 5- to 10-membered heteroarylthio, 5-to 10-membered heteroarylalkylene, 5- to 10-membered heteroarylalkoxy, 5- to 10-membered heteroarylalkylthio, 3- to 12-membered heterocyclyl, 3- to 12-membered heterocyclyloxy, 3- to 12-membered heterocyclylthio, 3- to 12-membered heterocyclyl C₁₋₁₂ alkylene, 3-to 12-membered heterocyclyl C₁₋₁₂ alkoxy, 3- to 12-membered heterocyclyl C₁₋₁₂ alkylthio, C₁₋₁₂ acyl, C₁₋₁₂ acyloxy, a carbamate group, C₁₋₁₂ amido, ureido, an epoxy group, a C₂₋₁₂ ester group, oxo, thio, and the like, wherein the substituents are optionally substituted with one or more substituents selected from the group consisting of the following substituents: a deuterium atom, oxo, hydroxy, amino, nitro, halogen, cyano, C₁₋₁₂ alkyl, C₂₋₁₂ alkenyl, C₂₋₁₂ alkynyl, C₁₋₁₂ alkoxy, halogenated C₁₋₁₂ alkoxy, C₁₋₁₂ alkylamino, di-C₁₋₁₂ alkylamino, halogenated C₁₋₁₂ alkylamino, halogenated di-C₁₋₁₂ alkylamino, carboxyl, -C(O)O-C₁₋₁₂ alkyl, -OC(O)-C₁₋₁₂ alkyl, -C(O)NH₂, -C(O)NH-C₁₋₁₂ alkyl, -C(O)N(C₁₋₁₂ alkyl)₂, -NHC(O)-C₁₋₁₂ alkyl, -C(O)-C₁₋₁₂ alkyl, -S(O)-C₁₋₁₂ alkyl, -S(O)₂-C₁₋₁₂ alkyl, -S(O)₂NH₂, -S(O)₂NH-C₁₋₁₂ alkyl, -S(O)₂N(C₁₋₁₂ alkyl)₂, 3- to 12-membered cycloalkyl, 3- to 12-membered cycloalkyl C₁₋₁₂ alkylene, 3- to 12-membered cycloalkyloxy, 3- to 12-membered heterocyclyl, 3- to 12-membered heterocyclyl C₁₋₁₂ alkylene, 3- to 12-membered heterocyclyloxy, 3- to 12-membered heterocycloalkyl, 3- to 12-membered heterocycloalkyl C₁₋₁₂ alkylene, 3- to 12-membered heterocycloalkyloxy, 5- to 10-membered heteroaryl, 5- to 10-membered heteroaryl C1-12 alkylene, 5- to 10-membered heteroaryloxy, 6- to 10-membered aryl, 6- to 10-membered aryl C₁₋₁₂ alkylene, and 6- to 10-membered aryloxy.

When any variable (e.g., R) occurs once or more times in the constitution or structure of a compound, the definition of the variable in each case is independent. Thus, for example, if a group is substituted with 0-2 R, the group can be optionally substituted with up to two R, and the definition of R in each case is independent. Furthermore, a combination of the substituent and/or the variant thereof is permissible only if the combination can result in a stable compound.

When one of the variables is selected from a single bond, it means that the two groups linked by the single bond are linked directly. For example, in A-L-Z, when L represents a single bond, it means that the structure is actually A-Z. When the direction for connection of the listed connecting group is not specified, the direction for connection is arbitrary. For example, when the connecting group L contained in 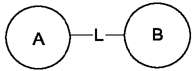 is -M-W-, -M-W- can either connect ring A and ring B in a direction same as left-to-right reading order to form 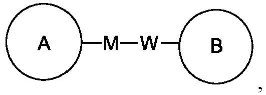 or connect ring A and ring B in a direction opposite to the left-to-right reading order to form 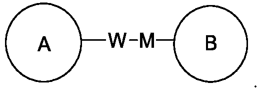 A combination of the connecting group, the substituent, and/or the variant thereof is permissible only if the combination can result in a stable compound.

Unless otherwise specified, when a group has one or more connectable sites, any one or more of the sites of the group may be connected to other groups by chemical bonds. If there is no designated connecting mode for chemical bonds and H atoms are present at a connectable site, when the connectable site is connected to chemical bonds, the number of the H atoms at the connectable site is correspondingly reduced based on the number of the connected chemical bonds, so that the resulting groups have corresponding valences. The chemical bond that connects the site and another group may be represented by a straight solid bond ( 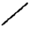 ), a straight dashed bond ( 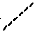 ), or a wavy line 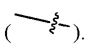 For example, the straight solid bond in -OCH₃ refers to being connected to another group via the oxygen atom in the group; the straight dashed bond in 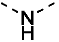 refers to being connected to another group via two ends of the nitrogen atom in the group; the wavy line in 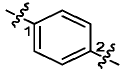 refers to being connected to another group via the carbon atoms at positions 1 and 2 in the phenyl group; 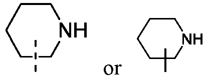 means that any connectable site on the piperidinyl can be connected to another group via 1 chemical bond in at least 4 connecting modes: 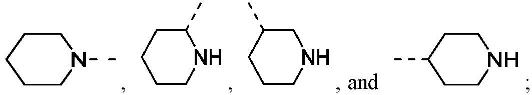 even if -N- is connected with an H atom, 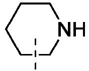 includes the connection mode of 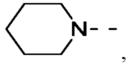 but when 1 chemical bond is connected to a site, the number of H at that site is correspondingly reduced by 1 and a monovalent piperidinyl is thus formed.

Unless otherwise specified, the term "C₁₋₁₂ alkyl", by itself or as part of another substituent, refers to a linear or branched saturated hydrocarbon group consisting of 1 to 12 carbon atoms. The C₁₋₁₂ alkyl may be C₁₋₆ alkyl or C₁₋₃ alkyl. Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆, and C₅ alkyl, etc., and may be monovalent (e.g., methyl), divalent (e.g., methylene) or polyvalent (e.g., methenyl). Examples of C₁₋₆ alkyl include, but are not limited to, methyl (Me), ethyl (Et), propyl (including n-propyl and isopropyl), butyl (including n-butyl, isobutyl, s-butyl, and t-butyl), pentyl (including n-pentyl, isopentyl, and neopentyl), hexyl, and the like. Unless otherwise specified, the term "C₁₋₁₂ deuterated alkyl" refers to the "C₁₋₁₂ alkyl" described above in which any number of H atoms or H atoms at any position are substituted with deuterium atoms. The C₁₋₁₂ deuterated alkyl may be C₁₋₆ deuterated alkyl or C₁₋₃ deuterated alkyl. Examples of deuterated alkyl include, but are not limited to, - CH₂D, -CHD₂, -CD₃, 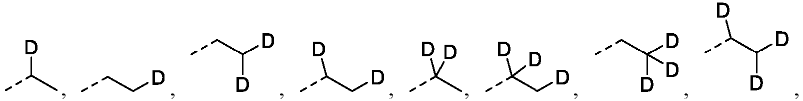 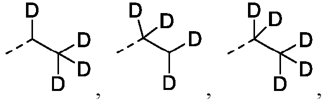 and the like.

Unless otherwise specified, the term "C₁₋₁₂ alkylene", by itself or as part of another substituent, refers to a linear or branched divalent hydrocarbon group consisting of 1 to 12 carbon atoms, including those having 1 to 12, 1 to 8, 1 to 6, 1 to 4, 1 to 3, or 1 to 2 carbon atoms. For example, the term "C₁₋₆ alkylene" refers to alkylene containing 1 to 6 carbon atoms. Non-limiting examples of alkylene include, but are not limited to, methylene (-CH₂-), ethylene (-CH₂CH₂-), propylene (-CH₂CH₂CH₂- or -CH₂CH(CH₃)-), butylene (-CH₂CH₂CH₂CH₂-, -CH₂CH(CH₃)CH₂-, or - CH₂CH₂CH(CH₃)-), and the like. The alkylene is optionally substituted with one or more substituents selected from the group consisting of the following substituents: oxo, hydroxy, amino, nitro, halogen, cyano, alkenyl, alkynyl, alkoxy, halogenated alkoxy, alkylamino, dialkylamino, halogenated alkylamino, halogenated dialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

Unless otherwise specified, the term "C₁₋₁₂ alkoxy" refers to those alkyl groups that each contains 1 to 12 carbon atoms and are linked to the rest part of the molecule via an oxygen atom. The C₁₋₁₂ alkoxy may be C₁₋₆ alkoxy or C₁₋₃ alkoxy. Unless otherwise specified, the term "C₁₋₆ alkoxy" refers to those alkyl groups that each contains 1 to 6 carbon atoms and are linked to the rest part of the molecule via an oxygen atom. The C₁₋₆ alkoxy includes C₁₋₅, C₁₋₄, C₁₋₃, C₁₋₂, C₂₋₆, C₂₋₄, C₆, C₅ alkoxy, and the like. Examples of C₁₋₆ alkoxy include, but are not limited to, methoxy, ethoxy, propoxy (including n-propoxy and isopropoxy), butoxy (including *n*-butoxy, isobutoxy, *sec-*butoxy, *tert*-butoxy, and the like), pentyloxy (including *n*-pentyloxy, isopentyloxy, and neopentyloxy), hexyloxy, and the like.

Unless otherwise specified, the term "C₁₋₁₂ alkylthio" refers to those alkyl groups that each contains 1 to 12 carbon atoms and are linked to the rest part of the molecule via a sulfur atom. The C₁₋₁₂ alkylthio may be C₁₋₆ alkylthio or C₁₋₃ alkylthio. Unless otherwise specified, the term "C₁₋₆ alkylthio" refers to those alkyl groups that each contains 1 to 6 carbon atoms and are linked to the rest part of the molecule via a sulfur atom. The C₁₋₆ alkylthio includes C₁₋₅, C₁₋₄, C₁₋₃, C₁₋₂, C₂₋₆, C₂₋₄, C₆, C₅ alkylthio, and the like. Examples of C₁₋₆ alkylthio include, but are not limited to, methylthio, ethylthio, propylthio (including *n*-propylthio and isopropylthio), butylthio (including *n*-butylthio, isobutylthio, *sec*-butylthio, and *tert*-butylthio), pentylthio (including *n*-pentylthio, isopentylthio, and neopentylthio), hexylthio, and the like.

Unless otherwise specified, "C₂₋₁₂ alkenyl" is used to denote a linear or branched hydrocarbon group containing 2 to 12 carbon atoms and at least one carbon-carbon double bond, which may be located anywhere in the group. The C₂₋₁₂ alkenyl may be C₂₋₆ alkenyl, C₂₋₃ alkenyl, or C₂₋₃ alkenyl. The C₂₋₄ alkenyl includes C₂₋₃, C₄, C₃, and C₂ alkenyl, and the like; the C₂₋₄ alkenyl may be monovalent, divalent, or polyvalent. Examples of C₂₋₄ alkenyl include, but are not limited to, ethenyl, propenyl, butenyl, butadienyl, and the like. Unless otherwise specified, "C₂₋₃ alkenyl" is used to denote a linear or branched hydrocarbon group containing 2 to 3 carbon atoms and at least one carbon-carbon double bond, which may be located anywhere in the group. The C₂₋₃ alkenyl includes C₃ and C₂ alkenyl; the C₂₋₃ alkenyl may be monovalent, divalent, or polyvalent. Examples of C₂₋₃ alkenyl include, but are not limited to, ethenyl, propenyl, and the like.

Unless otherwise specified, "C₂₋₁₂ alkynyl" is used to denote a linear or branched hydrocarbon group containing 2 to 12 carbon atoms and at least one carbon-carbon triple bond, which may be located anywhere in the group. The C₂₋₁₂ alkynyl includes C₂₋₆, C₂₋₃, C₄, C₃, and C₂ alkynyl, and the like. It may be monovalent, divalent, or polyvalent. Examples of C₂₋₃ alkynyl include, but are not limited to, ethynyl, propynyl, and the like.

Unless otherwise specified, the term "halogen", by itself or as part of another substituent, refers to a fluorine, chlorine, bromine, or iodine atom.

Unless otherwise specified, Cₙ₋ₙ₊ₘ or Cₙ-Cₙ₊ₘ includes any one of the specific cases of n to n+m carbon atoms. For example, C₁₋₁₂ includes C₁, C₂, C₃, C₄, C₅, C₆, C₇, C₈, C₉, C₁₀, C₁₁, and C₁₂. Also, any range within n to n+m may be included. For example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, and the like. Similarly, n-to n+m-membered represents that the number of atoms on the ring is n to n+m. For example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring. n- to n+m-membered also represents any range within n to n+m. For example, 3- to 12-membered ring includes 3- to 6-membered ring, 3- to 9-membered ring, 5- to 6-membered ring, 5- to 7-membered ring, 6- to 7-membered ring, 6-to 8-membered ring, 6- to 10-membered ring, and the like.

Unless otherwise specified, the term "ring" refers to substituted or unsubstituted cycloalkyl, heterocyclyl, heterocycloalkyl, cycloalkenyl, heterocycloalkenyl, cycloalkynyl, heterocycloalkynyl, aryl, or heteroaryl. The so-called ring includes monocyclic, bicyclic, spiro, fused, or bridged rings. The number of atoms on a ring is generally defined as the member number of the ring. For example, "5- to 7-membered ring" means that 5 to 7 atoms are arranged in a circle. Unless otherwise specified, the ring optionally contains 1-3 heteroatoms. Thus, "5- to 7-membered ring" includes, for example, phenyl, pyridinyl, and piperidinyl; the term "ring" further includes a ring system containing at least one ring, in which each "ring" independently meets the definition described above. Unless otherwise specified, "C₃₋₁₂ cycloalkyl" refers to a saturated cyclic hydrocarbon group consisting of 3 to 12 carbon atoms, including monocyclic and bicyclic ring systems. The C₃₋₁₂ cycloalkyl includes C₃₋₈ cycloalkyl, C₃₋₆ cycloalkyl, C₅₋₆ cycloalkyl, and the like, and may be monovalent, divalent, or polyvalent. Examples of C₃₋₁₂ cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like.

Unless otherwise specified, the term "C₃₋₁₂ cycloalkenyl", by itself or in combination with other terms, refers to a partially unsaturated cyclic hydrocarbon group containing 3 to 12 ring atoms and at least one carbon-carbon double bond, which may be monovalent, divalent, or polyvalent. The C₃₋₁₂ cycloalkenyl includes C₃₋₆ cycloalkenyl, C₅₋₆ cycloalkenyl, and the like. Examples of C₃₋₆ cycloalkenyl include, but are not limited to, 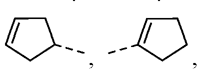 or

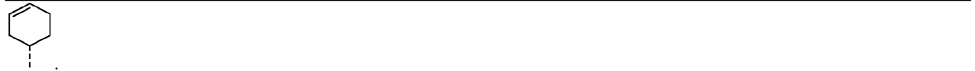

Unless otherwise specified, the term "5- to 7-membered heterocycloalkenyl", by itself or in combination with other terms, refers to a partially unsaturated cyclic group containing 5 to 7 ring atoms and at least one carbon-carbon double bond, of which 1, 2, 3, or 4 ring atoms are heteroatoms independently selected from the group consisting of O, S, N, and Se, with the remaining being carbon atoms. The nitrogen atom is optionally quaternized, and the nitrogen and sulfur heteroatoms can be optionally oxidized (i.e., NO and S(O)ₚ, wherein p is 1 or 2). It includes monocyclic and bicyclic systems, wherein the bicyclic system includes spiro, fused, and bridged rings, and any ring of this system is nonaromatic. It may be monovalent, divalent, or polyvalent. Furthermore, with respect to the "5-to 7-membered heterocycloalkenyl", a heteroatom may occupy the position where the heterocycloalkenyl is linked to the rest of the molecule. The 5- to 7-membered heterocycloalkenyl includes 5- to 6-membered heterocycloalkenyl. Examples of 5- to 6-membered heterocycloalkenyl include, but are not limited to,

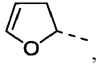

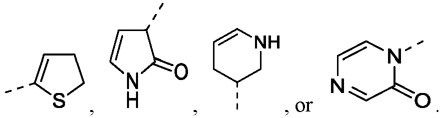

Unless otherwise specified, the term "3- to 6-membered heterocycloalkyl", by itself or in combination with other terms, refers to a saturated cyclic group consisting of 3 to 6 ring atoms, of which 1, 2, 3, or 4 ring atoms are heteroatoms independently selected from the group consisting of O, S, and N, with the remaining being carbon atoms. The nitrogen atom is optionally quaternized, and the nitrogen and sulfur heteroatoms can be optionally oxidized (i.e., NO and S(O)ₚ, wherein p is 1 or 2). It is a monocyclic ring system. Furthermore, with respect to the "3- to 6-membered heterocycloalkyl", a heteroatom may occupy the position where the heterocycloalkyl is linked to the rest part of the molecule. The 3- to 6-membered heterocycloalkyl includes 3-membered heterocycloalkyl, 4-membered heterocycloalkyl, 5-membered heterocycloalkyl, 6-membered heterocycloalkyl, and the like. Examples of 3- to 6-membered heterocycloalkyl include, but are not limited to, oxiranyl, thiiranyl, aziridinyl, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, and the like), tetrahydrothien-1,1-dioxide-3-yl, tetrahydrothien-1,1-dioxide-2-yl, tetrahydrofuranyl (including tetrahydrofuran-2-yl, and the like), tetrahydropyranyl, piperidinyl (including 1-piperidinyl, 2-piperidinyl, 3-piperidinyl, and the like), piperazinyl (including 1-piperazinyl, 2-piperazinyl, and the like), morpholinyl (including 3-morpholinyl, 4-morpholinyl, and the like), dioxanyl, dithianyl, isoxazolidinyl, isothiazolidinyl, 1,2-oxazinyl, 1,2-thiazinyl, hexahydropyridazinyl, hyperpiperazinyl, hyperpiperidinyl, or the like. Unless otherwise specified, the term "C₆₋₁₀ aryl" refers to a cyclic hydrocarbon group consisting of 6 to 10 carbon atoms and having a conjugated π-electron system, which may be a monocyclic, fused bicyclic, or fused tricyclic ring system, wherein each ring is aromatic. It may be monovalent, divalent, or polyvalent. The C₆₋₁₀ aryl includes C₉, C₁₀, and C₆ aryl. Examples of C₆₋₁₀ aryl include, but are not limited to, phenyl and naphthyl (including 1-naphthyl, 2-naphthyl, and the like).

Unless otherwise specified, the term "5- to 10-membered heteroaryl" refers to a cyclic group consisting of 5 to 10 ring atoms and having a conjugated π-electron system, of which 1, 2, 3, or 4 ring atoms are heteroatoms independently selected from the group consisting of O, S, N, and Se, with the remaining being carbon atoms. It may be a monocyclic, fused bicyclic, or fused tricyclic system, wherein each ring is aromatic. The nitrogen atom is optionally quaternized, and the nitrogen and sulfur heteroatoms can be optionally oxidized (i.e., NO and S(O)ₚ, wherein p is 1 or 2). It may be monovalent, divalent, or polyvalent. The 5- to 10-membered heteroaryl can be linked to the rest of the molecule via a heteroatom or a carbon atom. The 5- to 10-membered heteroaryl includes 5- to 8-membered, 5- to 7-membered, 5- to 6-membered, 5-membered, and 6-membered heteroaryl, and the like. Examples of the 5- to 10-membered heteroaryl include, but are not limited to, pyrrolyl (including *N*-pyrrolyl, 2-pyrrolyl, 3-pyrrolyl, and the like), pyrazolyl (including 2-pyrazolyl, 3-pyrazolyl, and the like), imidazolyl (including *N-*imidazolyl, 2-imidazolyl, 4-imidazolyl, 5-imidazolyl, and the like), oxazolyl (including 2-oxazolyl, 4-oxazolyl, 5-oxazolyl, and the like), triazolyl (including 1*H*-1,2,3-triazolyl, 2*H*-1,2,3-triazolyl, 1*H*-1,2,4-triazolyl, 4*H*-1,2,4-triazolyl, and the like), tetrazolyl, isoxazolyl (including 3-isoxazolyl, 4-isoxazolyl, 5-isoxazolyl, and the like), thiazolyl (including 2-thiazolyl, 4-thiazolyl, 5-thiazolyl, and the like), furanyl (including 2-furanyl, 3-furanyl, and the like), thienyl (including 2-thienyl, 3-thienyl, and the like), pyridinyl (including 2-pyridinyl, 3-pyridinyl, 4-pyridinyl, and the like), pyrazinyl, pyrimidinyl (including 2-pyrimidinyl, 4-pyrimidinyl, and the like), benzothiazolyl (including 5-benzothiazolyl and the like), purinyl, benzimidazolyl (including 2-benzimidazolyl and the like), benzoxazolyl, indolyl (including 5-indolyl and the like), isoquinolinyl (including 1-isoquinolinyl, 5-isoquinolinyl, and the like), quinoxalinyl (including 2-quinoxalinyl, 5-quinoxalinyl, and the like), or quinolyl (including 3-quinolyl, 6-quinolyl, and the like).

The term "3- to 12-membered heterocyclyl" refers to a fully saturated or partially unsaturated (but not fully unsaturated heteroaromatic group) nonaromatic ring that may exist in the form of a monocyclic, bridged cyclic, or spirocyclic structure. Unless otherwise specified, the heterocyclyl is usually a 3- to 12-membered ring containing 1 to 3 heteroatoms (preferably 1 or 2 heteroatoms) independently selected from the group consisting of sulfur, oxygen, and/or nitrogen. Non-limiting examples of heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4*H-*pyranyl, morpholinyl, sulfomorpholinyl, tetrahydrothienyl, and the like. The heterocyclyl is optionally substituted with one or more of the following groups: oxo, hydroxy, amino, nitro, halogen, cyano, alkyl, alkenyl, alkynyl, alkoxy, halogenated alkoxy, alkylamino, dialkylamino, halogenated alkylamino, halogenated dialkylamino, carboxyl, -C(O)O-alkyl, -OC(O)-alkyl, -C(O)NH₂, -C(O)NH-alkyl, -C(O)N(alkyl)₂, -NHC(O)-alkyl, -C(O)-alkyl, - S(O)-alkyl, -S(O)₂-alkyl, -S(O)₂NH₂, -S(O)₂NH-alkyl, -S(O)₂N(alkyl)₂, cycloalkyl, cycloalkyloxy, heterocyclyl, heterocyclyloxy, heterocycloalkyl, heterocycloalkyloxy, heteroaryl, heteroaryloxy, aryl, or aryloxy. The 3- to 12-membered heterocycloalkyl includes 3- to 8-membered, 3- to 6-membered, 4- to 5-membered, 4- to 6-membered, 5- to 6-membered, 4-membered, 5-membered, and 6-membered heterocycloalkyl, and the like. Examples of "3- to 12-membered heterocycloalkyl" and "3- to 6-membered heterocycloalkyl" include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, and the like), tetrahydrofuranyl (including tetrahydrofuran-2-yl and the like), tetrahydropyranyl, piperidinyl (including 1-piperidinyl, 2-piperidinyl, 3-piperidinyl, and the like), piperazinyl (including 1-piperazinyl, 2-piperazinyl, and the like), morpholinyl (including 3-morpholinyl, 4-morpholinyl, and the like), dioxanyl, dithianyl, isoxazolidinyl, isothiazolidinyl, 1,2-oxazinyl, 1,2-thiazinyl, hexahydropyridazinyl, hyperpiperazinyl, hyperpiperidinyl, dioxepanyl, or the like.

The term "treat", "treating", or "treatment" refers to administering the compound or formulation described herein to ameliorate or eliminate a disease or one or more symptoms related to the disease, and includes:
(i) inhibiting a disease or disease state, i.e., arresting its development; and
(ii) alleviating a disease or disease state, i.e., causing its regression.

The term "prevent", "preventing", or "prevention" refers to administering the compound or formulation described herein to prevent a disease or one or more symptoms related to the disease, and includes preventing the occurrence of the disease or disease state in a mammal, particularly when such a mammal is predisposed to the disease state but has not yet been diagnosed with it.

The term "therapeutically or prophylactically effective amount" refers to an amount of the compound of the present disclosure for (i) treating a specific disease, condition, or disorder, or (ii) alleviating, ameliorating, or eliminating one or more symptoms of a specific disease, condition, or disorder, or (iii) preventing or delaying the specific disease, condition, or disorder described herein. The amount of the compound of the present disclosure composing the "therapeutically or prophylactically effective amount" varies depending on the compound, the disease state and its severity, the mode of administration, and the age of the mammal to be treated, but may be determined routinely by those skilled in the art in accordance with their knowledge and the present disclosure.

Therapeutic or prophylactic dose of the compound of the present disclosure may be determined by, for example, the specific use of a treatment or prevention, the mode of administration of the compound, the health and condition of a patient, and the judgment of a prescribing physician. The proportion or concentration of the compound of the present disclosure in a pharmaceutical composition may not be constant and depends on a variety of factors including doses, chemical properties (e.g., hydrophobicity), and routes of administration. For example, the compound of the present disclosure may be provided for parenteral administration by a physiological buffered aqueous solution containing about 0.1-10% w/v of the compound. Certain typical doses range from about 1 µg/kg body weight/day to about 1 g/kg body weight/day. In certain embodiments, the dose ranges from about 0.01 mg/kg body weight/day to about 100 mg/kg body weight/day. The dose is likely to depend on such variables as the type and degree of progression of the disease or disorder, the general health condition of a particular patient, the relative biological potency of the compound selected, the excipient formulation, and its route of administration. Effective doses can be extrapolated from dose-response curves derived from *in vitro* or animal model test systems.

The word "comprise" and variations thereof such as "comprises" or "comprising" will be understood in an open, non-exclusive sense, i.e., "including but not limited to".

"Pharmaceutical composition" refers to a composition comprising one or more of the compound, the isomer thereof, or the pharmaceutically acceptable salt thereof described herein, as well as other components such as a physiologically/pharmaceutically acceptable carrier and excipient. The pharmaceutical composition is intended to promote the administration to organisms, facilitate the absorption of the active ingredients and thus exert biological activity.

The pharmaceutical composition of the present disclosure may be prepared by combining the compound of the present disclosure with a suitable pharmaceutically acceptable excipient.

The pharmaceutical composition of the present disclosure can be manufactured using methods well known in the art, such as conventional mixing, dissolving, granulating, dragee-making, levigating, emulsifying, and lyophilizing. The compounds of the present disclosure can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present disclosure.

The chemical reactions of the embodiments of the present disclosure are conducted in a proper solvent that must be suitable for the chemical changes in the present disclosure and the reagents and materials required. In order to obtain the compounds of the present disclosure, it is sometimes necessary for those skilled in the art to modify or select a synthesis procedure or a reaction process based on the existing embodiments.

The starting materials or intermediates used in the embodiments of the present disclosure may be obtained commercially or prepared by methods known in the art.

An important consideration in synthetic route planning in the art is the selection of suitable protecting groups for reactive functional groups (e.g., amino in the present disclosure). For example, reference may be made to Greene's Protective Groups in Organic Synthesis (4th Ed.) Hoboken, New Jersey: John Wiley & Sons, Inc.

In some embodiments, some of the compounds of the present disclosure can be prepared by those skilled in the art of organic synthesis by reference to the following routes: 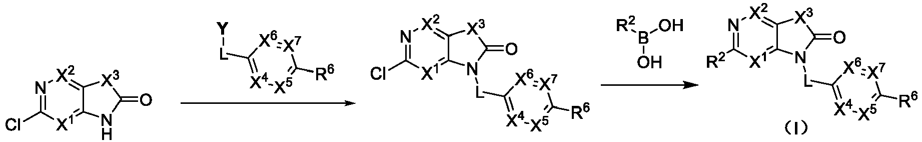 wherein,
X¹, X², X³, X⁴, X⁵, X⁶, X⁷, L, R², and R⁶ are as described in the compound of formula (I) above;
Y is selected from the group consisting of halogen, -OH, methylsulfonyloxy, phenylsulfonyloxy, and *p-*toluenesulfonyloxy.

In some embodiments of the present application, Y is selected from the group consisting of -Cl, -Br, -I, -OH, methylsulfonyloxy, and*p*-toluenesulfonyloxy.

In some embodiments of the present application, Y is selected from -Cl.

In some embodiments, some of the compounds of the present disclosure can be prepared by those skilled in the art of organic synthesis by reference to the following routes: 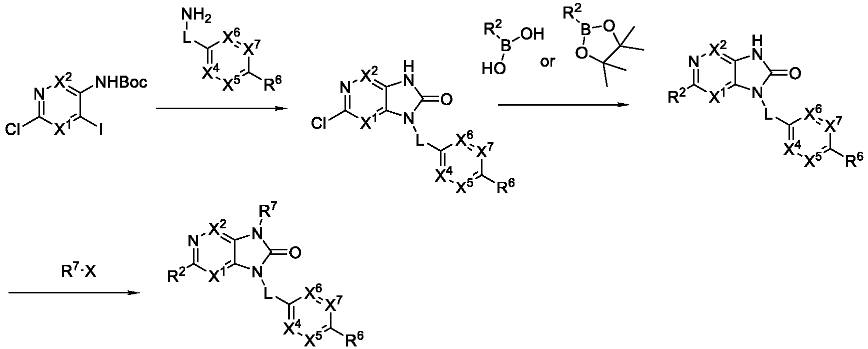 wherein,
X¹, X², X⁴, X⁵, X⁶, X⁷, L, R², R⁶, and R⁷ are as described in the compound of formula (I) above, and X is selected from halogen.

In some embodiments of the present application, X is selected from the group consisting of -Cl, -Br, and -I.

In some embodiments of the present application, X is selected from -I.

For clarity, the present disclosure is further described with the following examples, which are, however, not intended to limit the scope of the present disclosure. All the reagents used in the present disclosure are commercially available and can be used without further purification.

### DETAILED DESCRIPTION

The present disclosure is described in detail below by way of examples. However, this is by no means disadvantageously limiting the scope of the present disclosure. Although the present disclosure has been described in detail herein and specific embodiments have also been disclosed, it will be apparent to those skilled in the art that various changes and modifications can be made to the specific embodiments without departing from the spirit and scope of the present disclosure.

### Example 1

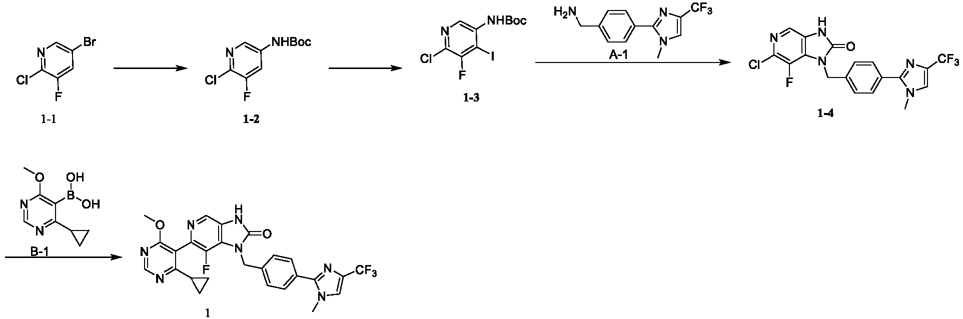

### Step 1: Preparation of intermediate 1-2

**1-1** (1 g), *tert-butyl* carbamate (0.612 g), 4,5-bis-diphenylphosphino-9,9-dimethylxanthene (0.192 g), tris(dibenzylideneacetone)dipalladium(0) (0.218 g), cesium carbonate (3.10 g), and 1,4-dioxane (20 mL) were added in sequence to a 100 mL single-necked flask. The mixture was reacted at 85 °C for 6 h under nitrogen atmosphere. The reaction solution was concentrated by evaporation under reduced pressure to remove the solvent, and the resulting crude product was separated and purified by silica gel column chromatography to give intermediate **1-2** (0.8 g).

¹H NMR (500 MHz, DMSO-*d*₆) *δ* 10.00 (s, 1H), 8.30 (d, *J =* 2.3 Hz, 1H), 7.99 (dd, *J =* 10.9, 2.2 Hz, 1H), 1.49 (s, 9H).

MS (ESI, [M+H]⁺) m/z: 247.20.

### Step 2: Preparation of intermediate 1-3

A solution of *n*-butyllithium in tetrahydrofuran (2.5 M, 3.99 mL) was slowly added dropwise to a solution of intermediate **1-2** (0.8 g) and *N*,*N*,*N*',*N*'-tetramethylethylenediamine (1.13 g) in tetrahydrofuran (10 mL) at -78 °C. The mixture was stirred at -20 °C for 1.5 h. The mixture was cooled to -78 °C, and a solution of iodine (2.47 g) in tetrahydrofuran (5 mL) was slowly added to the system. The resulting mixture was warmed to room temperature and reacted for 3 h. A saturated ammonium chloride solution (20 mL) was added to the reaction solution to quench the reaction, and the mixture was extracted with ethyl acetate. The organic phase was washed with saturated brine, dried over anhydrous sodium sulfate, filtered, and concentrated, and the resulting crude product was separated and purified by silica gel column chromatography to give intermediate 1-3 (0.7 g).

¹H NMR (500 MHz, DMSO-*d*₆) *δ* 9.08 (s, 1H), 8.16 (s, 1H), 1.47 (s, 9H).

MS (ESI, [M+H]⁺) m/z: 373.03.

### Step 3: Preparation of intermediate 1-4

Intermediate **1-3** (0.70 g), intermediate **A-1** (0.528 g), tris(dibenzylideneacetone)dipalladium(0) (0.172 g), 4,5-bis-diphenylphosphino-9,9-dimethylxanthene (0.13 g), cesium carbonate (1.84 g), and 1,4-dioxane (15 mL) were added in sequence to a 50 mL single-necked flask. The mixture was reacted at 100 °C for 12 h under nitrogen atmosphere. The reaction solution was concentrated by evaporation under reduced pressure to remove the solvent, and the resulting crude product was separated and purified by silica gel column chromatography to give intermediate **1-4** (0.4 g).

¹H NMR (500 MHz, DMSO-*d*₆) *δ* 11.84 (s, 1H), 7.96 (s, 1H), 7.92 (d, *J =* 1.3 Hz, 1H), 7.75-7.70 (m, 2H), 7.41 (d, *J =* 8.0 Hz, 2H), 5.17 (s, 2H), 3.77 (s, 3H).

MS (ESI, [M+H]⁺) m/z: 426.24.

### Step 4: Preparation of compound 1

Intermediate **1-4** (0.4 g), intermediate **B-1** (0.365 g), potassium phosphate (0.399 g), dicyclohexyl(2',4',6'-triisopropyl-[1,1'-biphenyl]-2-yl)phosphine (0.179 g), tris(dibenzylideneacetone)dipalladium(0) (0.172 g), 1,4-dioxane (20 mL), and water (1 mL) were added in sequence to a 35 mL microwave tube. The mixture was microwaved to 130 °C and reacted for 2 h under nitrogen atmosphere. The reaction solution was concentrated by evaporation under reduced pressure to remove the solvent, and the resulting crude product was separated and purified by silica gel column chromatography to give compound 1 (0.33 g).

¹H NMR (500 MHz, DMSO-*d*₆) *δ* 11.77 (s, 1H), 8.66 (s, 1H), 8.26 (d, *J =* 1.3 Hz, 1H), 7.92 (d, *J =* 1.4 Hz, 1H), 7.73-7.69 (m, 2H), 7.42 (d, *J =* 8.1 Hz, 2H), 5.18 (s, 2H), 3.80 (s, 3H), 3.76 (s, 3H), 1.71- 1.65 (m, 1H), 1.09-1.04 (m, 1H), 0.97-0.93 (m, 1H), 0.92-0.87 (m, 1H), 0.82-0.77 (m, 1H).

HRMS (ESI) *m*/*z* [M + H] ⁺: 540.1774.

### Example 2

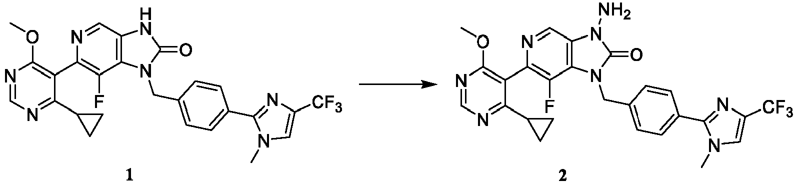

### Preparation of compound 2:

A solution of potassium hydroxide (0.042 g, 0.741 mmol) in water (1 mL) was added dropwise to a solution of compound 1 (0.1 g), tetrabutylammonium bromide (0.006 g), and 2,4-dinitrophenylhydroxylamine (0.148 g) in dichloromethane (5 mL). The mixture was reacted at 40 °C for 2 h. The reaction solution was concentrated by evaporation under reduced pressure to remove the solvent, and the resulting crude product was separated and purified by silica gel column chromatography to give compound 2 (0.03 g).

¹H NMR (500 MHz, DMSO-*d*₆) *δ* 8.66 (s, 1H), 8.35 (d, *J =* 1.1 Hz, 1H), 7.92 (d, *J =* 1.3 Hz, 1H), 7.73-7.69 (m, 2H), 7.44-7.40 (m, 2H), 5.66 (s, 2H), 5.23 (s, 2H), 3.80 (s, 3H), 3.75 (s, 3H), 1.69-1.64 (m, 1H), 1.10-1.05 (m, 1H), 0.99-0.94 (m, 1H), 0.92-0.87 (m, 1H), 0.84-0.78 (m, 1H).

HRMS (ESI) *m*/*z* [M + H]⁺: 555.1886.

### Example 3

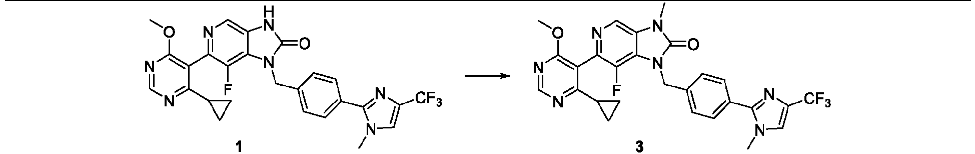

### Preparation of compound 3:

60% sodium hydride (0.006 g) was added to a mixed solution of compound **1** (0.060 g) in *N*,*N*-dimethylformamide (3 mL) at 0 °C. The mixture was reacted at 0 °C for 30 min. Iodomethane (0.024 g) was added to the reaction solution, and the mixture was warmed to room temperature and reacted for 40 min. The reaction solution was poured into water (50 mL), and the resulting solution was extracted with ethyl acetate (100 mL). The organic phase was washed with water and saturated brine, dried over anhydrous sodium sulfate, filtered, and concentrated, and the resulting crude product was separated and purified by silica gel column chromatography to give compound 3 (0.048 g).

¹H NMR (500 MHz, DMSO-*d*₆) *δ* 8.66 (s, 1H), 8.47 (s, 1H), 7.92 (s, 1H), 7.70 (d, *J =* 7.9 Hz, 2H), 7.42 (d, *J =* 7.9 Hz, 2H), 5.22 (s, 2H), 3.80 (s, 3H), 3.75 (s, 3H), 3.50 (s, 3H), 1.71-1.61 (m, 1H), 1.35-1.21 (m, 2H), 1.05-0.98 (m, 2H).

HRMS (ESI) *m*/*z* [M + H]⁺: 554.1935.

### Example 4

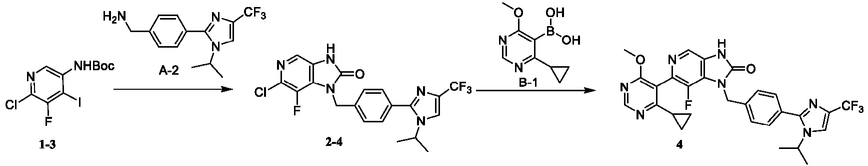

### Step 1: Preparation of intermediate 2-4

Intermediate **1-3** (0.3 g), intermediate **A-2** (0.251 g), 4,5-bis-diphenylphosphino-9,9-dimethylxanthene (0.093 g), tris(dibenzylideneacetone)dipalladium(0) (0.074 g), cesium carbonate (0.787 g), and 1,4-dioxane (15 mL) were added in sequence to a 50 mL single-necked flask. The mixture was reacted at 100 °C for 12 h under nitrogen atmosphere. The reaction solution was concentrated by evaporation under reduced pressure to remove the solvent, and the resulting crude product was separated and purified by silica gel column chromatography to give intermediate **2-4** (0.125 g).

¹H NMR (500 MHz, DMSO-*d*₆) *δ* 11.85 (s, 1H), 8.17 (s, 1H), 7.96 (s, 1H),7.57 (d, *J =* 8.5 Hz, 2H), 7.43 (d, *J =* 8.5 Hz, 2H), 5.18 (s, 2H), 4.50-4.42 (m, 1H), 1.39 (d, *J =* 6.5 Hz, 6H).

MS (ESI, [M+H]⁺) m/z: 454.21.

### Step 2: Preparation of compound 4

Intermediate **2-4** (0.10 g, 0.220 mmol), intermediate **B-1** (0.085 g), dicyclohexyl(2',4',6'-triisopropyl-[1,1'-biphenyl]-2-yl)phosphine (0.042 g), potassium phosphate (0.140 g), tris(dibenzylideneacetone)dipalladium(0) (0.040 g), 1,4-dioxane (5 mL), and water (0.25 mL) were added in sequence to a 25 mL single-necked flask. The mixture was reacted at 100 °C for 1 h under nitrogen atmosphere. The reaction solution was concentrated by evaporation under reduced pressure to remove the solvent, and the resulting crude product was separated and purified by silica gel column chromatography to give compound **4** (0.068 g).

¹H NMR (500 MHz, DMSO-*d*₆) *δ* 11.77 (s, 1H). 8.66 (s, 1H), 8.27 (s, 1H), 8.16 (s, 1H), 7.56 (d, *J =* 8.5 Hz, 2H), 7.43 (d, *J =* 8.5 Hz, 2H), 5.19 (s, 2H), 4.47-4.41 (m, 1H), 3.80 (s, 3H), 1.71- 1.65 (m, 1H), 1.38 (d, *J* = 6.5 Hz, 6H), 1.11-1.05 (m, 1H), 0.97-0.93 (m, 1H), 0.91-0.85 (m, 1H), 0.83-0.76 (m, 1H).

HRMS (ESI) *m*/*z* [M + H]⁺: 568.2097.

### Example 5

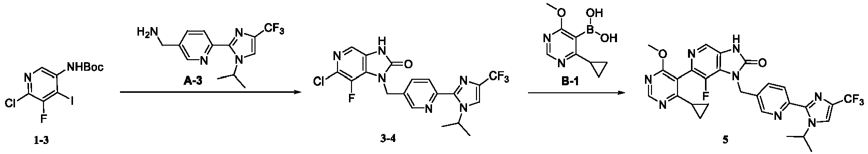

### Step 1: Preparation of intermediate 3-4

Intermediate **1-3** (0.40 g), intermediate **A-3** (0.34 g), tris(dibenzylideneacetone)dipalladium(0) (0.10 g), 4,5-bis-diphenylphosphino-9,9-dimethylxanthene (0.074 g), cesium carbonate (1.05 g), and 1,4-dioxane (15 mL) were added in sequence to a 50 mL single-necked flask. The mixture was reacted at 100 °C for 12 h under nitrogen atmosphere. The reaction solution was concentrated by evaporation under reduced pressure to remove the solvent, and the resulting crude product was separated and purified by silica gel column chromatography to give intermediate **3-4** (0.21 g).

¹H NMR (500 MHz, DMSO-d₆) *δ* 11.85 (s, 1H), 8.63 (d, *J =* 2.2 Hz, 1H), 8.23 (d, *J =* 1.3 Hz, 1H), 8.07-8.02 (m, 1H), 7.95 (s, 1H), 7.85 (dd, *J =* 8.3, 2.3 Hz, 1H), 5.76-5.68 (m, 1H), 5.20 (s, 2H), 1.44 (d, *J =* 6.7 Hz, 6H).

MS (ESI, [M+H]⁺) m/z: 455.22.

### Step 2: Preparation of compound 6

Intermediate **3-4** (0.2 g), intermediate **B-1** (0.171 g), potassium phosphate (0.187 g), dicyclohexyl(2',4',6'-triisopropyl-[1,1'-biphenyl]-2-yl)phosphine (0.084 g), tris(dibenzylideneacetone)dipalladium(0) (0.081 g), 1,4-dioxane (20 mL), and water (1 mL) were added in sequence to a 35 mL microwave tube. The mixture was microwaved to 130 °C and reacted for 2 h under nitrogen atmosphere. The reaction solution was concentrated by evaporation under reduced pressure to remove the solvent, and the resulting crude product was separated and purified by silica gel column chromatography to give compound 6 (0.15 g).

¹H NMR (500 MHz, DMSO-d₆) *δ* 11.79 (s, 1H), 8.66 (s, 1H), 8.63 (d, *J =* 2.2 Hz, 1H), 8.26 (d, *J =* 1.3 Hz, 1H), 8.23 (d, *J =* 1.3 Hz, 1H), 8.04 (d, *J =* 8.2 Hz, 1H), 7.85 (dd, *J =* 8.3, 2.3 Hz, 1H), 5.74-5.67 (m, 1H), 5.22 (s, 2H), 3.79 (s, 3H), 1.71-1.65 (m, 1H), 1.43 (d, *J =* 6.7 Hz, 6H), 1.09-1.05 (m, 1H), 0.97-0.93 (m, 1H), 0.89-0.85 (m, 1H), 0.81-0.77 (m, 1H).

HRMS (ESI) *m*/*z* [M + H]⁺: 569.2043.

### Example 6

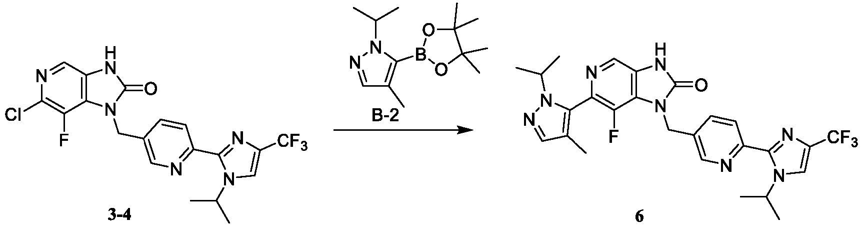

### Preparation of compound 6:

Intermediate **3-4** (0.050 mmol), intermediate **B-2** (0.055 g), dicyclohexyl(2',4',6'-triisopropyl-[1,1'-biphenyl]-2-yl)phosphine (0.021 g), potassium phosphate (0.070 g), tris(dibenzylideneacetone)dipalladium(0) (0.020 g), 1,4-dioxane (2.5 mL), and water (0.125 mL) were added in sequence to a 25 mL single-necked flask. The mixture was reacted at 100 °C for 1 h under nitrogen atmosphere. The reaction solution was concentrated by evaporation under reduced pressure to remove the solvent, and the resulting crude product was separated and purified by silica gel column chromatography to give compound 6 (0.035 g).

¹H NMR (500 MHz, DMSO-*d*₆) *δ* 11.84 (s, 1H), 8.66-8.62 (m, 1H), 8.32-8.28 (m, 1H), 8.24-8.21 (m, 1H), 8.02 (d, *J* = 5 Hz, 1H), 7.87-7.82 (m, 1H), 7.36(s, 1H), 5.73-5.65 (m, 1H), 5.23 (s, 2H), 4.38-4.29 (m, 1H), 1.81 (s, 3H), 1.42 (d, *J* = 5 Hz, 6H), 1.25 (d, *J =* 5 Hz, 6H).

HRMS (ESI) *m*/*z* [M + H] ⁺: 543.2245.

### Example 7

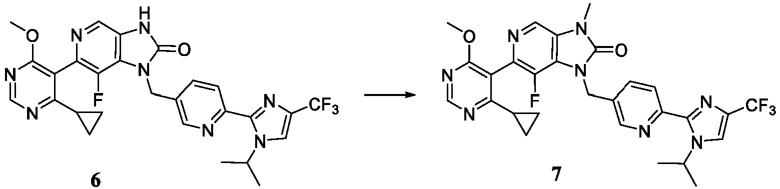

### Preparation of compound 7:

60% sodium hydride (0.005 g) was added to a solution of compound 6 (0.050 g) in *N*,*N*-dimethylformamide (1 mL) at 0 °C. The mixture was reacted at 0 °C for 30 min. Iodomethane (0.019 g) was added to the reaction solution, and the mixture was warmed to room temperature and reacted for 1 h. The reaction solution was poured into water (10 mL), and the resulting solution was extracted with ethyl acetate. The organic phase was washed with saturated brine, dried, and concentrated, and the resulting crude product was separated and purified by silica gel column chromatography to give compound **7** (0.035 g).

¹H NMR (500 MHz, DMSO-d₆) *δ* 8.67 (s, 1H), 8.63 (d, *J =* 2.2 Hz, 1H), 8.47 (d, *J =* 1.2 Hz, 1H), 8.24-8.22 (m, 1H), 8.04 (d, *J =* 8.3 Hz, 1H), 7.86 (dd, *J =* 8.3, 2.3 Hz, 1H), 5.73-5.67 (m, 1H), 5.27 (s, 2H), 3.79 (s, 3H), 3.50 (s, 3H), 1.69- 1.64 (m, 1H), 1.43 (d, *J =* 6.7 Hz, 6H), 1.10-1.05 (m, 1H), 0.98-0.93 (m, 1H), 0.90-0.85 (m, 1H), 0.81-0.76 (m, 1H).

HRMS (ESI) *m*/*z* [M + H] ⁺: 583.2206.

### Example 8

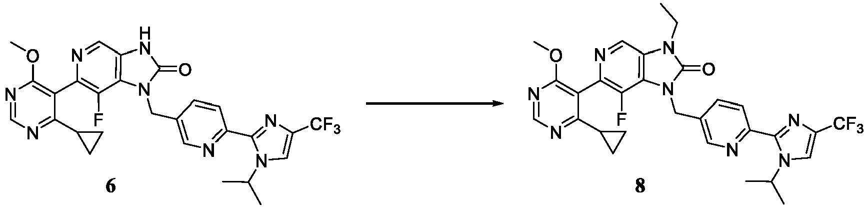

### Preparation of compound 8:

60% sodium hydride (0.007 g) was added to a solution of compound 6 (0.080 g) in *N*,*N*-dimethylformamide (2 mL) at 0 °C. The mixture was reacted at 0 °C for 30 min. Iodoethane (0.033 g) was added to the reaction solution, and the mixture was warmed to room temperature and reacted for 2 h. The reaction solution was poured into water (10 mL), and the resulting solution was extracted with ethyl acetate. The organic phase was washed with saturated brine, dried, and concentrated under reduced pressure, and the resulting crude product was separated and purified by silica gel column chromatography to give compound 7 (0.04 g).

¹H NMR (500 MHz, DMSO-d₆) *δ* 8.67 (s, 1H), 8.63 (d, *J =* 2.1 Hz, 1H), 8.54 (d, *J =* 1.2 Hz, 1H), 8.23 (d, *J =* 1.3 Hz, 1H), 8.04 (d, *J =* 8.3 Hz, 1H), 7.85 (dd, *J =* 8.3, 2.3 Hz, 1H), 5.73-5.67 (m, 1H), 5.26 (s, 2H), 4.03 (q, *J =* 7.1 Hz, 2H), 3.79 (s, 3H), 1.71-1.65 (m, 1H), 1.43 (d, *J =* 6.7 Hz, 6H), 1.34 (t, *J =* 7.2 Hz, 3H), 1.10-1.05 (m, 1H), 0.97-0.93 (m, 1H), 0.91-0.87 (m, 1H), 0.81-0.77 (m, 1H).

HRMS (ESI) *m*/*z* [M + H] ⁺: 597.2357.

### Example 9

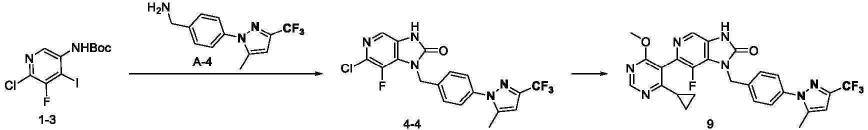

### Step 1: Preparation of intermediate 4-4

Intermediate **1-3** (0.3 g), intermediate **A-4** (0.226 g), cesium carbonate (0.787 g), tris(dibenzylideneacetone)dipalladium(0) (0.074 g), 4,5-bis-diphenylphosphino-9,9-dimethylxanthene (0.056 g), and 1,4-dioxane (15 mL) were added in sequence to a 50 mL single-necked flask. The mixture was reacted at 100 °C for 12 h under nitrogen atmosphere. The reaction solution was concentrated by evaporation under reduced pressure to remove the solvent, and the resulting crude product was separated and purified by silica gel column chromatography to give intermediate **4-4** (0.086 g).

¹H NMR (500 MHz, DMSO-*d*₆) *δ* 11.82 (s, 1H), 7.96 (s, 1H), 7.60-7.54 (m, 2H), 7.47 (d, *J =* 8.2 Hz, 2H), 6.75 (s, 1H), 5.19 (s, 2H), 2.33 (s, 3H).

MS (ESI, [M+H]⁺) m/z: 426.18.

### Step 2: Preparation of compound 9

Intermediate **4-4** (0.050 g), intermediate **B-1** (0.091 g), tris(dibenzylideneacetone)dipalladium(0) (0.054 g), potassium phosphate (0.075 g), 2-dicyclohexylphosphine-2',4',6'-triisopropylbiphenyl (0.050 g), 1,4-dioxane (3 mL), and water (0.15 mL) were added in sequence to an 8 mL microwave tube. The mixture was microwaved to 130 °C and reacted for 2 h under nitrogen atmosphere. The reaction solution was concentrated by evaporation under reduced pressure to remove the solvent, and the resulting crude product was separated and purified by silica gel column chromatography to give compound 9 (0.040 g).

¹H NMR (500 MHz, DMSO-*d*₆) *δ* 11.78 (s, 1H), 8.66 (s, 1H), 8.26 (d, *J =* 1.3 Hz, 1H), 7.60-7.54 (m, 2H), 7.46 (d, *J* = 8.3 Hz, 2H), 6.75 (s, 1H), 5.20 (s, 2H), 3.80 (s, 3H), 2.32 (s, 3H), 1.72-1.62 (m, 1H), 0.99-0.81 (m, 4H). HRMS (ESI) *m*/*z* [M + H] ⁺: 540.1767.

### Example 10

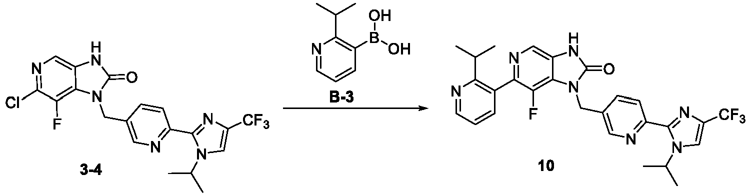

### Preparation of compound 10:

Intermediate **3-4** (0.050 g), intermediate **B-3** (0.036 g), 2-dicyclohexylphosphine-2',4',6'-triisopropylbiphenyl (0.021 g), potassium phosphate (0.070 g), tris(dibenzylideneacetone)dipalladium(0) (0.020 g), 1,4-dioxane (3 mL), and water (0.15 mL) were added in sequence to an 8 mL microwave tube. The mixture was microwaved to 130 °C and reacted for 2 h under nitrogen atmosphere. The reaction solution was concentrated by evaporation under reduced pressure to remove the solvent, and the resulting crude product was separated and purified by silica gel column chromatography to give compound **10** (0.011 g).

¹H NMR (500 MHz, DMSO-*d*₆) *δ* 11.77 (s, 1H), 8.66 - 8.56 (m, 2H), 8.29-8.19 (m, 2H), 8.03 (d, *J =* 8.2 Hz, 1H), 7.89-7.79 (m, 1H), 7.68-7.58 (m, 1H), 7.34-7.24 (m, 1H), 5.75-5.650 (m, 1H), 5.21 (s, 2H), 2.89-2.79 (m, 1H), 1.43 (d, *J =* 6.7 Hz, 6H), 1.03 (d, *J =* 6.6 Hz, 6H).

HRMS (ESI) *m*/*z* [M + H] ⁺: 540.2148.

### Example 11

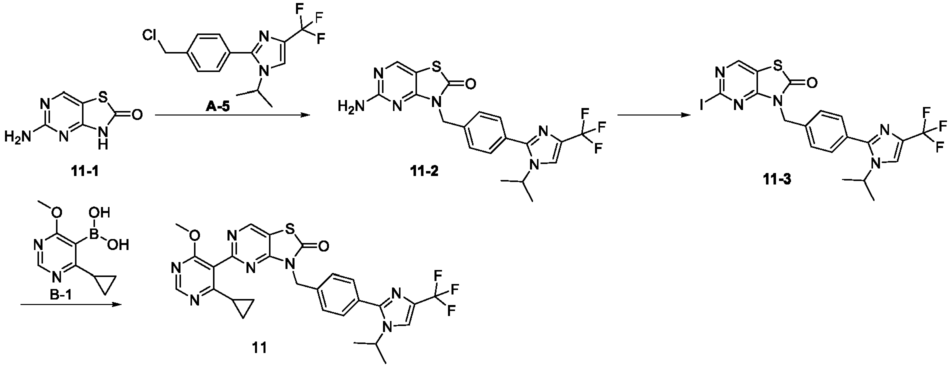

### Step 1: Preparation of intermediate 11-2

Intermediate **11-1** (0.40 g), *N,N*-dimethylformamide (8 mL), intermediate **A-5** (0.72 g), and potassium carbonate (0.82 g) were added in sequence to a 50 mL single-necked flask. The mixture was heated to 40 °C and reacted for 3 h. The reaction solution was poured into water (100 mL), and the resulting solution was extracted with ethyl acetate. The organic phases were combined, washed with saturated brine, dried over anhydrous sodium sulfate, filtered under vacuum, and concentrated by evaporation under reduced pressure to remove the solvent. The residue was separated and purified by silica gel column chromatography to give intermediate **11-2** (0.65 g).

¹H NMR (500 MHz, DMSO-*d*₆) δ 8.37 (s, 1H), 8.17 (s, 1H), 7.55 (d, *J =* 8.2 Hz, 2H), 7.45 (d, *J =* 8.2 Hz, 2H), 6.88 (s, 2H), 5.12 (s, 2H), 4.46 (hept, *J =* 6.6 Hz, 1H), 1.39 (d, *J =* 6.6 Hz, 6H).

MS (ESI, [M+H]⁺) m/z: 435.17.

### Step 2: Preparation of intermediate 11-3

Intermediate **11-2** (0.20 g), *N,N*-dimethylformamide (12 mL), iodine (1.23 g), copper(I) iodide (8.77 mg), and isoamyl nitrite (0.27 g) were added in sequence to a 30 mL microwave tube. The mixture was microwaved to 120 °C and reacted for 1 h. The reaction solution was poured into a saturated sodium sulfite solution (100 mL), and the resulting solution was extracted with ethyl acetate. The organic phases were combined, washed with saturated brine, dried over anhydrous sodium sulfate, filtered under vacuum, and concentrated by evaporation under reduced pressure to remove the solvent. The residue was separated and purified by silica gel column chromatography to give intermediate **11-3** (0.2 g).

¹H NMR (500 MHz, DMSO-*d*₆) δ 8.71 (s, 1H), 8.17 (s, 1H), 7.56 (d, *J* = 8.2 Hz, 2H), 7.47 (d, *J* = 8.2 Hz, 2H), 5.17 (s, 2H), 4.46 (dt, *J* = 13.2, 6.6 Hz, 1H), 1.40 (d, *J =* 6.6 Hz, 6H).

MS (ESI, [M+H]⁺) m/z:546.03.

### Step 3: Preparation of compound 11

Intermediate **11-3** (0.15 g), intermediate **B-1** (0.11 g), 1,4-dioxane (2 mL), water (0.2 mL), 2-dicyclohexylphosphine-2',4',6'-triisopropylbiphenyl (0.026 g), potassium phosphate (0.18 g), and tris(dibenzylideneacetone)dipalladium(0) (0.025 g) were added in sequence to a 10 mL single-necked flask. The mixture was heated to 100 °C and reacted for 3 h under nitrogen atmosphere. The reaction solution was cooled to room temperature and poured into water (100 mL), and the resulting solution was extracted with ethyl acetate. The organic phases were combined, washed with saturated brine, dried over anhydrous sodium sulfate, filtered under vacuum, and concentrated by evaporation under reduced pressure to remove the solvent. The residue was separated and purified by silica gel column chromatography to give compound **11** (65 mg).

¹H NMR (500 MHz, DMSO-*d*₆) δ 9.11 (s, 1H), 8.69 (s, 1H), 8.17 (s, 1H), 7.52 (dd, *J =* 23.5, 8.2 Hz, 4H), 5.24 (s, 2H), 4.43 (dt, *J =* 13.3, 6.6 Hz, 1H), 3.86 (s, 3H), 1.81 - 1.73 (m, 1H), 1.39 (d, *J* = 6.7 Hz, 6H), 1.06 - 1.02 (m, 2H), 0.86 - 0.79 (m, 2H).

HRMS (ESI) *m*/*z* [M + H] ⁺: 568.1749.

### Example 12

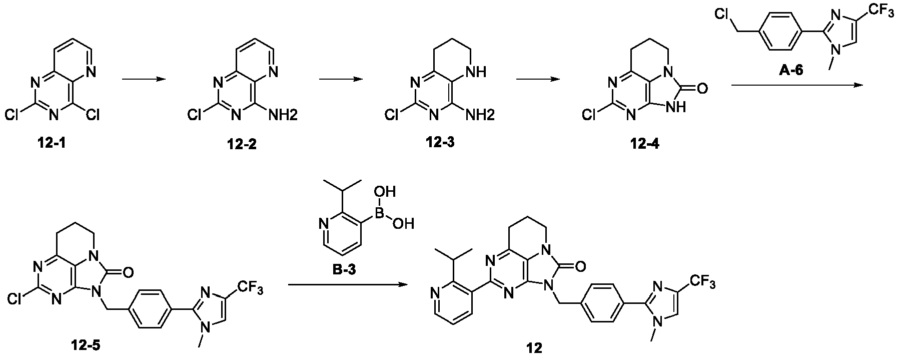

### Step 1: Preparation of intermediate 12-2

Intermediate **12-1** (1.0 g), tetrahydrofuran (20 mL), and ammonium hydroxide (1 mL) were added in sequence to a 100 mL single-necked flask under an ice bath. The mixture was naturally warmed to room temperature and reacted for 2 h. The reaction solution was concentrated, water (50 mL) was added, and then the mixture was vigorously stirred for 10 min and filtered. The filter cake was washed with water (10 mL) and dried to give intermediate **12-2** (1.5 g).

¹H NMR (500 MHz, DMSO-*d*₆) δ 8.81 (dd, *J =* 4.2, 1.6 Hz, 1H), 8.50 (d, *J =* 18.9 Hz, 2H), 8.05 (dd, *J =* 8.4, 1.5 Hz, 1H), 7.85 (dd, *J* = 8.5, 4.2 Hz, 1H).

MS (ESI, [M+H]⁺) m/z: 181.06.

### Step 2: Preparation of intermediate 12-3

Intermediate **12-2** (1.0 g), ethanol (10 mL), and trifluoroacetic acid (0.43 mL) were added in sequence to a 100 mL single-necked flask under an ice bath, and platinum oxide (0.38 g) was added under nitrogen atmosphere. The mixture was purged with hydrogen and hydrogenated overnight. The reaction solution was filtered under vacuum through diatomite pad, the filtrate was concentrated, and the resulting crude product was separated and purified by silica gel column chromatography to give intermediate **12-3** (0.51 g).

¹H NMR (500 MHz, DMSO-*d*₆) δ 7.00 (s, 1H), 3.26 - 3.16 (m, 2H), 2.60 (t, *J =* 6.4 Hz, 2H), 1.87 - 1.77 (m, 2H). MS (ESI, [M+H]⁺) m/z: 185.11.

### Step 3: Preparation of intermediate 12-4

Intermediate **12-3** (100 mg), tetrahydrofuran (5 mL), and triphosgene (96 mg) were added in sequence to a 10 mL reaction flask under an ice bath. The mixture was naturally warmed to room temperature and stirred for 1 h. Imidazole (737 mg) was added, and the mixture was stirred at room temperature for 5 min, heated to 70 °C, and reacted for 1.5 h. Water (50 mL) and ethyl acetate (50 mL) were added to the reaction solution, and liquid separation was performed. The organic phase was concentrated, and the resulting crude product was separated and purified by silica gel column chromatography to give intermediate **12-4** (85 mg).

¹H NMR (500 MHz, DMSO-*d*₆) δ 7.65 (s, 1H), 3.73 (t, *J =* 5.7 Hz, 2H), 2.79 (t, *J =* 6.1 Hz, 2H), 2.16 - 2.07 (m, 2H).

MS (ESI, [M+H]⁺) m/z: 211.11.

### Step 4: Preparation of intermediate 12-5

A mixture of intermediate **12-4** (50 mg, 0.237 mmol), intermediate **A-6** (78 mg), potassium carbonate (98 mg, 0.712 mmol), and *N,N*-dimethylformamide (2 mL) was stirred at room temperature for 4 h. The reaction solution was poured into a saturated sodium chloride solution (20 mL), and the resulting solution was extracted with ethyl acetate (20 mL × 2). The organic phase was concentrated, and the resulting crude product was separated and purified by silica gel column chromatography to give intermediate **12-5** (67 mg).

¹H NMR (500 MHz, DMSO-*d*₆) δ 7.95 - 7.91 (m, 1H), 7.74 - 7.69 (m, 2H), 7.49 - 7.45 (m, 2H), 5.08 (s, 2H), 3.84 - 3.80 (m, 2H), 3.76 (s, 3H), 2.84 (t, *J* = 6.0 Hz, 2H), 2.21 - 2.11 (m, 2H).

MS (ESI, [M+H]⁺) m/z: 449.26.

### Step 5: Preparation of compound 12

Intermediate **12-5** (50.0 mg), intermediate **B-3** (92 mg), tris(dibenzylideneacetone)dipalladium(0) (51.0 mg), potassium phosphate (70.9 mg), 2-dicyclohexylphosphine-2',4',6'-triisopropylbiphenyl (47.8 mg), 1,4-dioxane (2 mL), and water (0.04 mL) were added in sequence to a 10 mL microwave tube. The mixture was microwaved to 130 °C and reacted for 2 h under nitrogen atmosphere. The reaction solution was concentrated, and the resulting crude product was separated and purified by silica gel column chromatography to give compound 12 (25 mg).

¹H NMR (500 MHz, DMSO-*d*₆) δ 8.63 - 8.53 (m, 1H), 7.91 (d, *J =* 9.6 Hz, 2H), 7.70 (d, *J =* 8.3 Hz, 2H), 7.51 (d, *J =* 8.2 Hz, 2H), 7.34 - 7.24 (m, 1H), 5.12 (s, 2H), 3.88 (t, *J =* 5.6 Hz, 2H), 3.75 (s, 3H), 3.62 - 3.52 (m, 1H), 2.92 (t, *J =* 6.0 Hz, 2H), 2.28 - 2.18 (m, 2H), 1.16 (d, *J* = 6.7 Hz, 6H).

HRMS (ESI) *m*/*z* [M + H] ⁺: 534.2234.

### Example 13

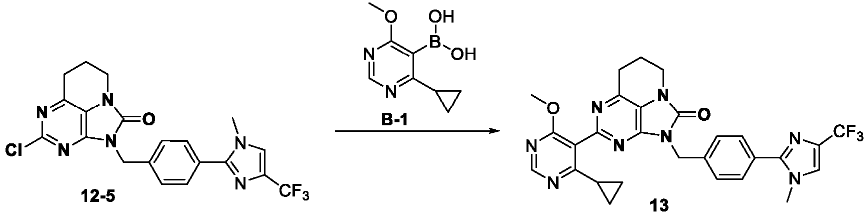

Intermediate **12-5** (50.0 mg), intermediate **B-1** (43.2 mg), tris(dibenzylideneacetone)dipalladium(0) (51.0 mg, 0.056 mmol), potassium phosphate (70.9 mg), 2-dicyclohexylphosphine-2',4',6'-triisopropylbiphenyl (47.8 mg), 1,4-dioxane (3 mL), and water (0.15 mL) were added in sequence to a 10 mL microwave tube. The mixture was microwaved to 130 °C and reacted for 2 h under nitrogen atmosphere. The reaction solution was concentrated, and the resulting crude product was separated and purified by silica gel column chromatography to give compound **13** (60 mg).

¹H NMR (500 MHz, DMSO-*d*₆) δ 8.64 (s, 1H), 7.92 (s, 1H), 7.70 - 7.64 (m, 2H), 7.52 - 7.46 (m, 2H), 5.11 (s, 2H), 3.89 - 3.86 (m, 2H), 3.83 (s, 3H), 3.75 (s, 3H), 2.91 (t, *J =* 6.0 Hz, 2H), 2.27 - 2.17 (m, 2H), 1.7 - 1.6 (m, 1H), 1.07 - 0.97 (m, 2H), 0.84 (s, 2H).

HRMS (ESI) *m*/*z* [M + H] ⁺: 563.2130.

### Example 14

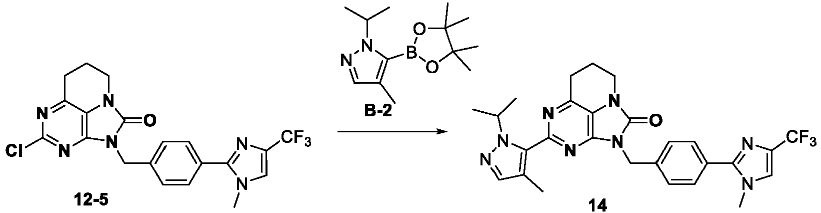

Intermediate **12-5** (50.0 mg), intermediate **B-2** (55.7 mg), tris(dibenzylideneacetone)dipalladium(0) (51.0 mg), potassium phosphate (70.9 mg), 2-dicyclohexylphosphine-2',4',6'-triisopropylbiphenyl (47.8 mg), 1,4-dioxane (3 mL), and water (0.15 mL) were added in sequence to a 10 mL microwave tube. The mixture was microwaved to 130 °C and reacted for 2 h under nitrogen atmosphere. The reaction solution was concentrated, and the resulting crude product was separated and purified by silica gel column chromatography to give compound 14 (34 mg).

¹H NMR (500 MHz, DMSO-*d*₆) δ 7.92 (d, *J* = 1.3 Hz, 1H), 7.76 - 7.65 (m, 2H), 7.52 (d, *J* = 8.2 Hz, 2H), 7.33 (s, 1H), 5.26 - 5.16 (m, 1H), 5.12 (s, 2H), 3.88 (t, *J* = 5.6 Hz, 2H), 3.75 (s, 3H), 2.90 (t, *J* = 6.0 Hz, 2H), 2.27 - 2.17 (m, 2H), 2.12 (s, 3H), 1.33 (d, *J =* 6.6 Hz, 6H).

HRMS (ESI) *m*/*z* [M + H] ⁺: 537.2339.

### Example 15

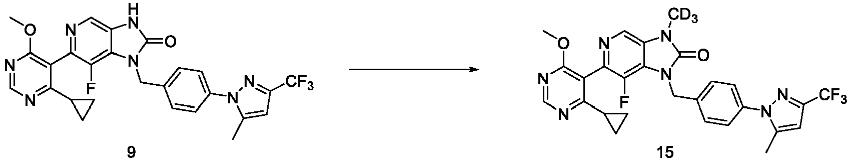

Sodium hydride (11.12 mg) was added to a solution of compound **9** (100 mg) in *N,N*-dimethylformamide (1 mL) at 0 °C. After the addition, the mixture was maintained at 0 °C and reacted for 30 min. Deuterated iodomethane (28.2 mg) was added to the reaction solution, and the mixture was reacted at 0 °C. After the reaction was completed, the reaction solution was poured into water (10 mL), and the resulting solution was extracted with ethyl acetate (10 mL). The organic phase was washed with saturated brine, dried, and concentrated, and the resulting crude product was separated and purified by silica gel column chromatography (dichloromethane:methanol = 98:2) to give compound **15** (77 mg).

HRMS (ESI) *m*/*z* [M + H] ⁺: 557.1737.

¹H NMR (500 MHz, DMSO-*d*₆) δ 8.66 (s, 1H), 8.47 (s, 1H), 7.56 (d, *J* = 8.5 Hz, 2H), 7.47 (d, *J =* 8.4 Hz, 2H), 6.75 (s, 1H), 5.24 (s, 2H), 3.80 (s, 3H), 2.31 (s, 3H), 1.69 - 1.62 (m, 1H), 1.11-1.04 (m, 1H), 0.99-0.93 (m, 1H), 0.93-0.86 (m, 1H), 0.84-0.77 (m, 1H).

### Example 16

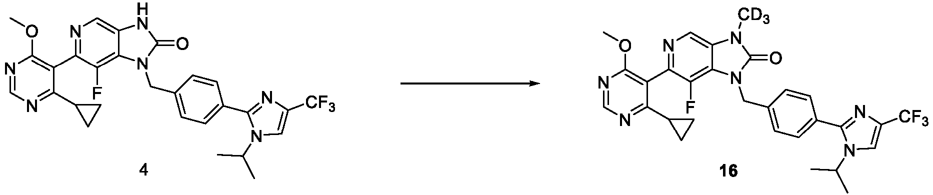

Sodium hydride (8.46 mg, 60% dispersed in mineral oil) was added to a solution of compound **4** (80 mg) in *N,N-*dimethylformamide (1 mL) at 0 °C. After the addition, the mixture was maintained at 0 °C and reacted for 30 min. Deuterated iodomethane (21.45 mg) was added to the reaction solution, and the mixture was warmed to room temperature and reacted. After the reaction was completed, the reaction solution was poured into water (10 mL), and the resulting solution was extracted with ethyl acetate (10 mL). The organic phase was washed with saturated brine, dried, and concentrated, and the resulting crude product was separated and purified by silica gel column chromatography (dichloromethane:methanol = 98:2) to give compound 16 (45 mg).

HRMS (ESI) *m*/*z* [M + H] ⁺: 585.2446.

¹H NMR (500 MHz, DMSO-*d*₆) δ 8.67 (s, 1H), 8.47 (s, 1H), 8.17 (s, 1H), 7.56 (d, *J* = 8.5 Hz, 2H), 7.44 (d, *J* = 8.5 Hz, 2H), 5.23 (s, 2H), 4.46-4.38 (m, 1H), 3.80 (s, 3H), 1.71-1.62 (m, 1H), 1.39 (d, *J* = 6.5 Hz, 6H), 1.11-1.05 (m, 1H), 0.98-0.94 (m, 1H), 0.92-0.86 (m, 1H), 0.83-0.76 (m, 1H).

### Example 17

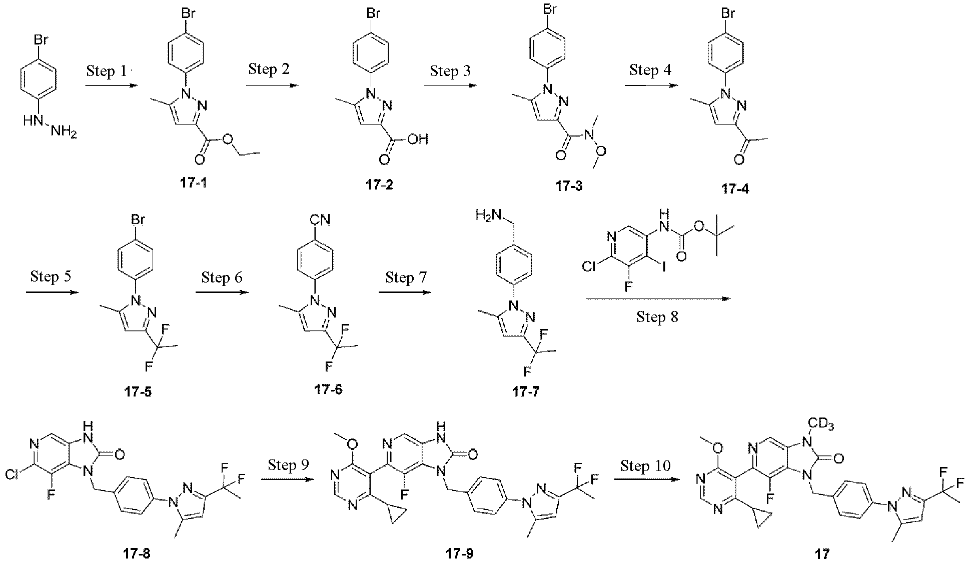

### Step 1: Synthesis of intermediate 17-1

(4-Bromophenyl)hydrazine (24.07 g) and hexafluoroisopropanol (300 mL) were mixed, and ethyl 2,4-dioxovalerate (18.5 g) was added under an ice bath. The mixture was warmed to room temperature and stirred for 12 h. The reaction solution was directly concentrated, and the resulting crude product was separated and purified by silica gel column chromatography (petroleum ether:ethyl acetate = 90:10) to give intermediate **17-1** (27.8 g).

MS (ESI): *m*/*z* = 309.08 [M+H]⁺.

¹H NMR (500 MHz, DMSO-*d*₆) δ 7.79-7.74 (m, 2H), 7.59-7.53 (m, 2H), 6.77 (s, 1H), 4.29 (q, *J =* 7.1 Hz, 2H), 2.34 (s, 3H), 1.29 (t, *J =* 7.1 Hz, 3H).

### Step 2: Synthesis of intermediate 17-2

Intermediate **17-1** (10 g), methanol (40 mL), water (20 mL), and lithium hydroxide hydrate (6.79 g) were mixed, and the mixture was stirred at room temperature for 12 h. The reaction solution was concentrated to remove methanol, and the residue was diluted with ethyl acetate (50 mL). The pH was adjusted to 6 with a saturated aqueous citric acid solution, and the organic phase was directly concentrated to give intermediate **17-2** (8.9 g).

MS (ESI): m/z = 281.18[M+H]⁺

¹H NMR (500 MHz, DMSO-*d*₆) *δ* 12.81 (br, 1H), 7.76 (d, *J =* 8.8 Hz, 2H), 7.55 (d, *J =* 8.8 Hz, 2H), 6.72 (s, 1H), 2.34 (s, 3H).

### Step 3: Synthesis of intermediate 17-3

Intermediate **17-2** (6 g) and dichloromethane (60 mL) were mixed, the reaction solution was cooled to 0 °C, and *N,O*-dimethylhydroxylamine hydrochloride (2.29 g), 4-methylmorpholine (2.38 g), and 3-(((ethylimino)methylene)amino)-*N,N*-dimethylpropan-1-amine hydrochloride (4.50 g) were added thereto. The mixture was warmed to room temperature and stirred for 3 h. The reaction solution was adjusted to pH 5-6 with 1 M diluted hydrochloric acid, and the resulting solution was extracted with dichloromethane (100 mL). The organic phase was washed with water and saturated brine, dried over anhydrous sodium sulfate, filtered, and concentrated, and the resulting crude product was separated and purified by silica gel column chromatography to give intermediate **17-3** (6.4 g).

MS (ESI): m/z= 324.05 [M+H]⁺.

¹H NMR (500 MHz, DMSO-*d*₆) δ 7.75 (d, *J =* 8.7 Hz, 2H), 7.56 (d, *J =* 8.7 Hz, 2H), 6.66 (s, 1H), 3.73 (s, 3H), 3.34 (s, 3H), 2.36 (s, 3H).

### Step 4: Synthesis of intermediate 17-4

Methyl magnesium bromide (3 M, 7.77 mL) was slowly added dropwise to a solution of intermediate **17-3** (6.3 g, 19.43 mmol) in tetrahydrofuran (50 mL) at 0 °C under nitrogen atmosphere. After the addition, the mixture was warmed to room temperature and stirred for 12 h. The reaction solution was poured into a saturated aqueous ammonium chloride solution (50 mL), and the resulting solution was extracted with ethyl acetate (100 mL). The organic phase was washed with water (100 mL) and saturated brine (100 mL), dried over anhydrous sodium sulfate, filtered, and concentrated to give intermediate **17-4** (5.4 g).

MS (ESI): m/z= 279.08 [M+H]⁺.

¹H NMR (500 MHz, DMSO-*d*₆) δ 7.77 (d, *J =* 8.8 Hz, 2H), 7.58 (d, *J =* 8.7 Hz, 2H), 6.72 (s, 1H), 2.49 (s, 3H), 2.35 (s, 3H).

### Step 5: Synthesis of intermediate 17-5

Intermediate **17-4** (1.6 g) and 1,2-dichloroethane (40 mL) were mixed, the mixture was cooled to 0 °C, and *N,N-*diethyl-1,1,1-trifluoro-14-sulfonamide (7.39 g) and triethylamine trihydrofluoride (4.62 g) were added dropwise thereto. After the addition, the mixture was heated to 70 °C and stirred for 12 h. The reaction solution was poured into water (20 mL) and adjusted to pH 7-8 with saturated sodium bicarbonate, and the resulting solution was extracted with ethyl acetate (100 mL). The organic phase was washed with water and saturated brine, dried over anhydrous sodium sulfate, filtered, and concentrated, and the resulting crude product was separated and purified by silica gel column chromatography to give intermediate **17-5** (0.72 g).

MS (ESI): m/z= 301.04 [M+H]⁺.

¹H NMR (500 MHz, DMSO-*d*₆) *δ* 7.76- 7.71 (m, 2H), 7.55-7.51 (m, 2H), 6.53 (s, 1H), 2.34 (s, 3H), 1.99 (t, *J* = 18.8 Hz, 3H).

### Step 6: Synthesis of intermediate 17-6

Intermediate **17-5** (1.5 g), zinc powder (39.1 mg), 1,1'-bis(diphenylphosphino)ferrocene (276 mg), tris(dibenzylideneacetone)palladium (228 mg), zinc cyanide (360 mg), and *N,N*-dimethylacetamide (10 mL) were mixed. The mixture was reacted at 150 °C for 12 h under nitrogen atmosphere. The reaction solution was poured into water (50 mL), and the resulting solution was extracted with ethyl acetate (100 mL). The organic phase was washed with water and saturated brine, dried over anhydrous sodium sulfate, filtered, and concentrated, and the resulting crude product was separated and purified by silica gel column chromatography to give intermediate **17-6** (800 mg).

MS (ESI): m/z = 248.22 [M+H]⁺.

### Step 7: Synthesis of intermediate 17-7

Intermediate **17-6** (770 mg) and tetrahydrofuran (5 mL) were mixed, and lithium aluminum hydride (2.5 M, 2.49 mL) was added under an ice bath. After the addition, the mixture was warmed to room temperature and stirred for 1 h. The reaction solution was poured into water (50 mL), and the resulting solution was extracted with ethyl acetate (100 mL). The organic phase was washed with water and saturated brine, dried over anhydrous sodium sulfate, filtered, and concentrated, and the resulting crude product was separated and purified by silica gel column chromatography to give intermediate **17-7** (720 mg).

MS (ESI): m/z = 252.25 [M+H]⁺.

### Step 8: Synthesis of intermediate 17-8

Intermediate **17-7** (300 mg), tris(dibenzylideneacetone)dipalladium(0) (109 mg), cesium carbonate (1167 mg), 4,5-bis-diphenylphosphino-9,9-dimethylxanthene (138 mg), *tert*-butyl (6-chloro-5-fluoro-4-iodopyridin-3-yl)carbamate (445 mg), and 1,4-dioxane (5 mL) were mixed. The mixture was reacted at 100 °C for 12 h under nitrogen atmosphere. The reaction solution was directly concentrated, and the resulting crude product was separated and purified by silica gel column chromatography to give intermediate **17-8** (300 mg).

MS (ESI): m/z = 422.22 [M+H]⁺.

¹H NMR (500 MHz, DMSO-*d*₆) *δ* 7.96 (s, 1H), 7.56-7.52 (m, 2H), 7.44 (d, *J* = 8.5 Hz, 2H), 6.50 (s, 1H), 5.17 (s, 2H), 2.31 (s, 3H), 1.97 (t, *J* = 18.8 Hz, 3H).

### Step 9: Synthesis of intermediate 17-9

Intermediate **17-8** (300 mg), potassium phosphate (453 mg), (4-cyclopropyl-6-methoxypyrimidin-5-yl)boronic acid (276 mg), tris(dibenzylideneacetone)dipalladium(0) (65.1 mg), dicyclohexyl(2',4',6'-triisopropyl-[1,1'-biphenyl]-2-yl)phosphine (67.8 mg), 1,4-dioxane (5 mL), and water (0.25 mL) were mixed. The mixture was reacted at 100 °C for 1 h under nitrogen atmosphere. The reaction solution was directly concentrated, and the resulting crude product was separated and purified by silica gel column chromatography to give intermediate **17-9** (70 mg).

MS (ESI): m/z = 536.22 [M+H]⁺.

¹H NMR (500 MHz, DMSO-*d*₆) *δ* 11.77 (s, 1H), 8.65 (s, 1H), 8.26 (s, 1H), 7.52 (d, *J* = 6.4 Hz, 2H), 7.43 (d, *J* = 8.5 Hz, 2H), 6.50 (s, 1H), 5.18 (s, 2H), 3.80 (s, 3H), 2.30 (s, 3H), 1.97 (t, *J =* 18.8 Hz, 3H), 1.66 (td, *J* = 8.1, 4.0 Hz, 1H), 1.06 (ddd, *J* = 7.5, 4.7, 2.5 Hz, 1H), 0.99-0.92 (m, 1H), 0.92-0.84 (m, 1H), 0.84-0.76 (m, 1H).

### Step 10: Synthesis of compound 17

Sodium hydride (11 mg) was slowly added to a stirred solution of intermediate **17-9** (100 mg) in *N,N-*dimethylformamide (3 mL) at 0 °C. After the addition, the mixture was maintained at 0 °C and reacted for 30 min. Deuterated iodomethane (28 mg) was added to the reaction solution, and the mixture was warmed to room temperature and reacted for 1 h. The reaction solution was poured into water (10 mL), and the resulting solution was extracted with ethyl acetate (10 mL). The organic phase was washed with saturated brine, dried, and concentrated, and the resulting crude product was separated and purified by silica gel column chromatography to give compound **17** (42 mg).

HRMS (ESI) *m*/*z* [M + H] ⁺: 553.2368.

¹H NMR (500 MHz, DMSO-*d*₆) *δ* 8.66 (s, 1H), 8.47 (s, 1H), 7.52 (d, *J* = 8.5 Hz, 2H), 7.44 (d, *J* = 8.4 Hz, 2H), 6.50 (s, 1H), 5.27-5.19 (m, 2H), 3.80 (s, 3H), 2.30 (d, *J* = 8.9 Hz, 3H), 1.97 (t, *J =* 18.8 Hz, 3H), 1.65 (td, *J =* 8.0, 4.1 Hz, 1H), 1.11-1.04 (m, 1H), 0.99-0.93 (m, 1H), 0.92-0.86 (m, 1H), 0.83-0.77 (m, 1H).

### Test Example 1: Inhibitory Activity for In Vitro Cell Proliferation

### 1.1 Assay on inhibitory activity for MDA-MB-436 cell proliferation

MDA-MB-436 cells in a good growth state were collected into a centrifuge tube, adjusted to a cell density of 10⁴ cells/mL, and seeded in a 96-well plate (100 µL/well), and the plate was incubated overnight in a cell incubator. Compounds were added using a nanoliter pipettor, such that the final concentrations of the compounds were 1000 nM-0.457 nM. The wells were set in duplicate, and a control well was set. After another 7 days of incubation in the cell incubator, the detection reagent CCK-8 (manufacturer: Dojindo Laboratories; 10 µL/well) was added. After 2 h of incubation in the cell incubator, absorbance values were detected at 450 nm using an Envision microplate reader, the four-parameter analysis was performed, the dose-response curves were fit, and IC₅₀ was calculated. The experimental results are shown in Table 1, where A represents 0 nM < IC₅₀ ≤ 50 nM; B represents 50 nM < IC₅₀ ≤ 100 nM; C represents 100 nM < IC₅₀ ≤ 1000 nM; and D represents 1000 nM < IC₅₀.

**Table 1. Inhibitory activity for MDA-MB-436 cell proliferation**

| **Compound** | IC₅₀ (nM) |
|---|---|
| Example 1 | A |
| Example 2 | B |
| Example 3 | A |
| Example 11 | A |
| Example 12 | C |
| Example 13 | A |
| Example 15 | B |
| Example 16 | A |
| Example 17 | B |

### Test Example 2: In Vitro Enzymatic Inhibitory Activity

### 2.1 Assay on USP1/UAF1 enzymatic inhibitory activity

A USP1/UAF1 protein solution (with a concentration of 10 nM) was added to assay wells at 10 µL/well, and different compounds dissolved in DMSO were separately added to the assay wells using a nanoliter pipettor, such that the final concentrations of the compounds were 1000 nM-0.24 nM. The wells were set in duplicate, and a control well was set. After incubation of the system described above at room temperature for 30 min, Ub-Rhodamine (with a concentration of 300 nM) was added to the assay wells at 10 µL/well. The mixtures were reacted at room temperature for 90 min, and then the plate was read using a PerkinElmer Envision multifunctional microplate reader (Ex. 490 nm/Em. 520 nm). IC₅₀ was calculated by four-parameter fitting.

The experimental results are shown in Table 2, where A represents 0 nM < IC₅₀ ≤ 50 nM; B represents 50 nM < IC₅₀ ≤ 100 nM; C represents 100 nM < IC₅₀ ≤ 1000 nM; and D represents 1000 nM < IC₅₀.

**Table 2. USP1/UAF1 enzymatic inhibitory activity**

| **Compound** | IC₅₀ (nM) |
|---|---|
| Example 3 | A |
| Example 15 | A |
| Example 16 | A |
| Example 17 | B |

The compounds in the present disclosure have good USP1/UAF1 enzymatic inhibitory activity.

### Test Example 3: In Vitro Inhibitory Activity for CYP450 Enzyme

A human liver microsome incubation system was prepared by mixing a PBS buffer (pH 7.4), a liver microsome solution (0.2 mg/mL), a CYP450 specific substrate, a test compound, and an NADPH + MgCl₂ solution, and incubated at 37 °C and 300 rpm for 0.5 h. Additionally, a positive control group and a negative control group were set. For the positive control group, a specific inhibitor was used in place of the test compound in the system described above, and for the negative control group, a solvent was used in place of the test compound in the system described above. An acetonitrile solution containing an internal standard was added to the incubated samples, and supernatants were prepared by protein precipitation, diluted, and then assayed by LC/MS/MS for the metabolites of the specific substrates. The inhibition rate was calculated using the following formula: (1 - (test group/negative control group)) × 100%. The compounds of the present disclosure have low *in vitro* inhibitory effects on the CYP450 enzyme.

### Test Example 4: Mouse Pharmacokinetics

ICR mice weighing 18-22 g were randomized into groups of 9 after 3-5 days of acclimatization and then intragastrically given a test compound solution at a dose of 10 mg/kg.

Plasma samples to be tested were prepared by taking blood from the orbit at time points of 15 min, 0.5 h, 1 h, 2 h, 4 h, 6 h, 8 h, 10 h, and 24 h.

30 µL of each of the plasma samples to be tested and a standard curve sample were taken, and an acetonitrile solution containing an internal standard was added. Supernatants were obtained by protein precipitation, diluted, and then assayed by LC/MS/MS.

Data were fitted using a non-compartmental model. The compounds of the present disclosure have good pharmacokinetic properties.

## Claims

1. A compound of formula (I), an isomer thereof, or a pharmaceutically acceptable salt thereof, 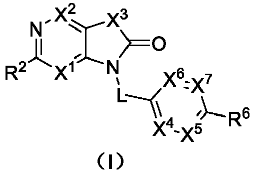 wherein,
X¹ and X² are each independently selected from the group consisting of C(R¹) and N;
X³ is selected from the group consisting of C(R¹)₂, C(=Z), N(R⁷), S, and O;
X⁴, X⁵, X⁶, and X⁷ are each independently selected from the group consisting of C(R⁵) and N;
each R¹ and R⁵ is independently selected from the group consisting of hydrogen, deuterium, halogen, -CN, -OH, -SH, -COOH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3-to 12-membered heterocyclyl, wherein the -OH, -SH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more substituents;
Z is selected from the group consisting of O, S, and N(R⁸);
or X² and X³ are linked to each other to form a 5- to 7-membered ring optionally substituted with one or more substituents;
or R⁵ groups on two adjacent carbon atoms, together with the carbon atoms to which they are linked, form C₅₋₇ cycloalkenyl, 5- to 7-membered heterocycloalkenyl, phenyl, or 5- to 6-membered heteroaryl optionally substituted with one or more substituents;
L is selected from the group consisting of -(C(R³R⁴))ₘ-, -O-, -S-, -N(R⁹)-, -S(O)-, and -S(O)₂-;
R² is selected from the group consisting of C₃₋₁₂ cycloalkyl, C₆₋₁₀ aryl, 5- to 10-membered heteroaryl, 3- to 12-membered heterocyclyl, and C₅₋₇ cycloalkenyl, wherein the C₃₋₁₂ cycloalkyl, C₆₋₁₀ aryl, 5- to 10-membered heteroaryl, 3- to 12-membered heterocyclyl, and C₅₋₇ cycloalkenyl are optionally independently substituted with one or more R^{2a};
each R³ and R⁴ is independently selected from the group consisting of hydrogen, deuterium, halogen, -CN, -OH, -SH, -COOH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3-to 12-membered heterocyclyl, wherein the -OH, -SH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more substituents;
or R³ and R⁴, together with the carbon atom to which they are linked, form C₃₋₆ cycloalkyl or 3- to 6-membered heterocycloalkyl optionally substituted with one or more substituents;
R⁶ is selected from the group consisting of hydrogen, halogen, -CN, -OH, -SH, -COOH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl, wherein the -OH, -SH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more R^{6a};
each R⁷, R⁸, or R⁹ is independently selected from the group consisting of hydrogen, -NH₂, C₁₋₁₂ alkyl, C₁₋₁₂ deuterated alkyl, C₁₋₁₂ alkoxy, C₁₋₁₂ deuterated alkoxy, C₃₋₁₂ cycloalkyl, and 3- to 12-membered heterocyclyl, wherein the -NH₂, C₁₋₁₂ alkyl, C₁₋₁₂ deuterated alkyl, C₁₋₁₂ alkoxy, C₁₋₁₂ deuterated alkoxy, C₃₋₁₂ cycloalkyl, or 3-to 12-membered heterocyclyl is optionally substituted with one or more substituents;
each R^{2a} and R^{6a} is independently selected from the group consisting of hydrogen, deuterium, halogen, -CN, -OH, -SH, -COOH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3-to 12-membered heterocyclyl, wherein the -OH, -SH, -NH₂, -NHC₁₋₁₂ alkyl, -N(C₁₋₁₂ alkyl)₂, C₁₋₁₂ alkyl, C₁₋₁₂ deuterated alkyl, C₂₋₁₂ alkenyl, C₂₋₁₂ alkynyl, C₁₋₁₂ alkoxy, C₁₋₁₂ deuterated alkoxy, C₁₋₁₂ alkylthio, -COC₁₋₁₂ alkyl, -OC(O)C₁₋₁₂ alkyl, -C(O)OC₁₋₁₂ alkyl, -OC(O)OC₁₋₁₂ alkyl, -SO₂C₁₋₁₂ alkyl, -NHSO₂C₁₋₁₂ alkyl, -N(C₁₋₁₂ alkyl)SO₂C₁₋₁₂ alkyl, -SO₂NH₂, -SO₂NHC₁₋₁₂ alkyl, -SO₂N(C₁₋₁₂ alkyl)₂, -CONH₂, -CONHC₁₋₁₂ alkyl, -CON(C₁₋₁₂ alkyl)₂, -NHCOC₁₋₁₂ alkyl, -N(C₁₋₁₂ alkyl)COC₁₋₁₂ alkyl, C₃₋₁₂ cycloalkyl, C₃₋₁₂ cycloalkenyl, C₆₋₁₀ aryl, 5- to 10-membered heteroaryl, and 3- to 12-membered heterocyclyl are optionally independently substituted with one or more substituents;
m is selected from the group consisting of 1, 2, and 3; provided that when X¹ is N, X³ is not NH.

2. The compound of formula (I), the isomer thereof, or the pharmaceutically acceptable salt thereof according to claim 1, wherein at least one of X¹ and X² is selected from C(R¹), and X³ is selected from the group consisting of C=O, N(R⁷), S, and O; preferably, X² is selected from C(R¹), X¹ is selected from N, and X³ is selected from the group consisting of C=O, N(R⁷), S, and O; or X² is selected from the N, X¹ is selected from C(R¹), and X³ is selected from the group consisting of C=O, N(R⁷), S, and O; or X¹ is selected from C(R¹), X² is selected from C(R¹), and X³ is selected from the group consisting of C=O, N(R⁷), S, and O; further preferably, X² is selected from C(R¹), X¹ is selected from N, and X³ is selected from N(R⁷); or X² is selected from N, X¹ is selected from C(R¹), and X³ is selected from N(R⁷); or X¹ is selected from C(R¹), X² is selected from C(R¹), and X³ is selected from N(R⁷).

3. The compound of formula (I), the isomer thereof, or the pharmaceutically acceptable salt thereof according to claim 1, wherein X² and X³ are linked to each other to form a 5- to 7-membered ring optionally substituted with one or more substituents, wherein the 5- to 7-membered ring is selected from the group consisting of 5- to 7-membered heterocycloalkenyl, C₅₋₇ cycloalkenyl, phenyl, and 5- to 6-membered heteroaryl; preferably, X² and X³ are linked to each other to form a 5- to 7-membered ring optionally substituted with one or more substituents, wherein the ring atom at the position indicated by X³ is a N atom; further preferably, X² and X³ are linked to each other to form a 5- to 7-membered ring optionally substituted with one or more substituents, wherein the ring atom at the position indicated by X² is a C atom; most preferably, X² and X³ are linked to each other to form a 5- to 7-membered ring optionally substituted with one or more substituents, wherein the ring atom at the position indicated by X² is a C atom, and the ring atom at the position indicated by X³ is a N atom.

4. The compound of formula (I), the isomer thereof, or the pharmaceutically acceptable salt thereof according to claim 1, wherein at least 2 of X⁴, X⁵, X⁶, and X⁷ are selected from C(R⁵); preferably, X⁴ is selected from C(R⁵), and X⁵ is selected from C(R⁵); or preferably, X⁶ is selected from C(R⁵), and X⁷ is selected from C(R⁵).

5. The compound of formula (I), the isomer thereof, or the pharmaceutically acceptable salt thereof according to any one of claim 2 or 4, wherein each R¹ and R⁵ is independently selected from the group consisting of hydrogen, deuterium, halogen, -CN, -OH, -NH₂, -NHC₁₋₃ alkyl, -N(C₁₋₃ alkyl)₂, C₁₋₃ alkyl, C₁₋₃ deuterated alkyl, C₁₋₃ alkoxy, and C₁₋₃ deuterated alkoxy, wherein the C₁₋₃ alkoxy, C₁₋₃ deuterated alkoxy, C₁₋₃ alkoxy, or C₁₋₃ deuterated alkoxy is optionally independently substituted with one or more substituents; preferably, each R¹ and R⁵ is optionally independently substituted with one or more groups selected from the group consisting of halogen, deuterium, OH, CN, and NH₂; preferably, each R¹ is independently selected from the group consisting of hydrogen, -F, methyl, -CD₃, ethyl, -CD₂CD₃, and CN;
or preferably, each R⁵ is independently selected from the group consisting of hydrogen and -F.

6. The compound of formula (I), the isomer thereof, or the pharmaceutically acceptable salt thereof according to claim 1, wherein L is selected from the group consisting of -(C(R³R⁴))ₘ-, -O-, -S-, and -N(R⁹)-; preferably, L is selected from the group consisting of -(C(R³R⁴))ₘ-, -O-, -S-, and -NH-.

7. The compound of formula (I), the isomer thereof, or the pharmaceutically acceptable salt thereof according to claim 6, wherein each R³ and R⁴ is independently selected from the group consisting of hydrogen, halogen, -CN, -OH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl, wherein the -OH, -NH₂, -NHC₁₋₃ alkyl, -N(C₁₋₃ alkyl)₂, C₁₋₃ alkyl, C₂₋₃ alkenyl, C₂₋₃ alkynyl, C₁₋₃ alkoxy, C₁₋₃ alkylthio, -SO₂NH₂, -CONH₂, C₃₋₆ cycloalkyl, C₃₋₆ cycloalkenyl, phenyl, 5- to 6-membered heteroaryl, and 3- to 6-membered heterocyclyl are optionally independently substituted with one or more substituents; preferably, each R³ and R⁴ is independently selected from the group consisting of hydrogen, halogen, -CN, -OH, -NH₂, C₁₋₃ alkyl, C₁₋₃ deuterated alkyl, C₁₋₃ alkoxy, and C₁₋₃ deuterated alkoxy, wherein the C₁₋₃ alkyl, C₁₋₃ deuterated alkyl, C₁₋₃ alkoxy, or C₁₋₃ deuterated alkoxy is optionally independently substituted with one or more substituents; preferably, each R³ and R⁴ is optionally independently substituted with one or more groups selected from the group consisting of halogen, deuterium, OH, CN, and NH₂;
or R³ and R⁴, together with the carbon atom to which they are linked, form cyclopropyl, cyclobutyl, cyclopentyl, cyclohexyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, isoxazolidinyl, piperidinyl, piperazinyl, or morpholinyl optionally substituted with one or more substituents; preferably, R³ and R⁴, together with the carbon atom to which they are linked, form cyclopropyl, cyclobutyl, oxetanyl, thietanyl, or azetidinyl optionally substituted with one or more substituents.

8. The compound of formula (I), the isomer thereof, or the pharmaceutically acceptable salt thereof according to claim 1, wherein R² is selected from the group consisting of C₃₋₈ cycloalkyl, phenyl, naphthyl, 5- to 10-membered heteroaryl, 3- to 8-membered heterocyclyl, and C₅₋₆ cycloalkenyl, wherein the C₃₋₈ cycloalkyl, phenyl, naphthyl, 5- to 10-membered heteroaryl, 3- to 8-membered heterocyclyl, and C₅₋₆ cycloalkenyl are optionally independently substituted with one or more R^{2a}; preferably, R² is selected from the group consisting of C₃₋₆ cycloalkyl, phenyl, naphthyl, 5- to 9-membered heteroaryl, 3- to 6-membered heterocyclyl, and C₅₋₆ cycloalkenyl, wherein the C₃₋₆ cycloalkyl, phenyl, naphthyl, 5- to 9-membered heteroaryl, 3- to 6-membered heterocyclyl, and C₅₋₆ cycloalkenyl are optionally independently substituted with one or more R^{2a}; further preferably, R² is selected from the group consisting of C₅₋₆ cycloalkyl, phenyl, 5- to 6-membered heteroaryl, 5- to 6-membered heterocyclyl, and C₅₋₆ cycloalkenyl, wherein the C₅₋₆ cycloalkyl, phenyl, 5- to 6-membered heteroaryl, 5- to 6-membered heterocyclyl, and C₅₋₆ cycloalkenyl are optionally independently substituted with one or more R^{2a}.

9. The compound of formula (I), the isomer thereof, or the pharmaceutically acceptable salt thereof according to claim 1, wherein R⁶ is selected from the group consisting of hydrogen, halogen, -CN, -OH, -NH₂, C₁₋₃ alkyl, C₁₋₃ deuterated alkyl, C₁₋₃ alkoxy, C₁₋₃ deuterated alkoxy, C₃₋₆ cycloalkyl, phenyl, 5- to 6-membered heteroaryl, and 3-to 6-membered heterocyclyl, wherein the C₁₋₃ alkyl, C₁₋₃ deuterated alkyl, C₁₋₃ alkoxy, C₁₋₃ deuterated alkoxy, C₃₋₆ cycloalkyl, phenyl, 5- to 6-membered heteroaryl, or 3- to 6-membered heterocyclyl is optionally independently substituted with one or more R^{6a}; preferably, R⁶ is selected from the group consisting of phenyl, pyrrolyl, pyrazolyl, imidazolyl, triazolyl, pyridinyl, and pyrimidinyl, wherein the phenyl, pyrrolyl, pyrazolyl, imidazolyl, triazolyl, pyridinyl, and pyrimidinyl are optionally independently substituted with one or more R^{6a}.

10. The compound of formula (I), the isomer thereof, or the pharmaceutically acceptable salt thereof according to any one of claim 8 or 9, wherein each R^{2a} and R^{6a} is independently selected from the group consisting of halogen, -CN, -OH, -NH₂, C₁₋₃ alkyl, C₁₋₃ deuterated alkyl, C₁₋₃ alkoxy, C₁₋₃ deuterated alkoxy, C₃₋₆ cycloalkyl, and 3- to 6-membered heterocyclyl, wherein the C₁₋₃ alkyl, C₁₋₃ deuterated alkyl, C₁₋₃ alkoxy, C₁₋₃ deuterated alkoxy, C₃₋₆ cycloalkyl, or 3- to 6-membered heterocyclyl is optionally independently substituted with one or more substituents; preferably, each R^{2a} and R^{6a} is independently selected from the group consisting of halogen, -CN, -OH, -NH₂, methyl, ethyl, isopropyl, methoxy, ethoxy, isopropoxy, cyclopropyl, cyclobutyl, oxetanyl, and azetidinyl, wherein the methyl, ethyl, isopropyl, methoxy, ethoxy, isopropoxy, cyclopropyl, cyclobutyl, oxetanyl, and azetidinyl are optionally independently substituted with one or more substituents; preferably, each R^{2a} and R^{6a} is optionally independently substituted with one or more groups selected from the group consisting of halogen, deuterium, OH, CN, and NH₂;
or R^{2a} is selected from the group consisting of -F, -Cl, -Br, -1, methyl, isopropyl, methoxy, monofluoromethyl, difluoromethyl, trifluoromethyl, monofluoromethoxy, difluoromethoxy, trifluoromethoxy, 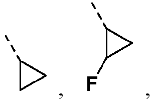 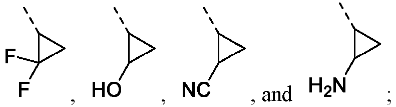
or R^{6a} is selected from the group consisting of -F, -Cl, -Br, -I, methyl, isopropyl, methoxy, monofluoromethyl, difluoromethyl, trifluoromethyl, monofluoromethoxy, difluoromethoxy, trifluoromethoxy,

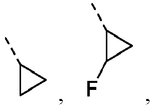

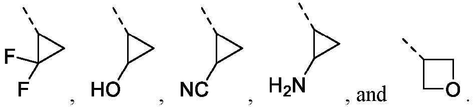

11. The compound of formula (I), the isomer thereof, or the pharmaceutically acceptable salt thereof according to claim 1, wherein each R⁷, R⁸, or R⁹ is independently selected from the group consisting of hydrogen, -NH₂, C₁₋₃ deuterated alkyl, C₁₋₃ alkyl, C₁₋₃ alkoxy, C₁₋₃ deuterated alkoxy, C₃₋₆ cycloalkyl, and 3- to 6-membered heterocyclyl, wherein the -NH₂, C₁₋₃ deuterated alkyl, C₁₋₃ alkyl, C₁₋₃ alkoxy, C₁₋₃ deuterated alkoxy, C₃₋₆ cycloalkyl, or 3- to 6-membered heterocyclyl is optionally substituted with one or more substituents; preferably, each R⁷, R⁸, or R⁹ is optionally independently substituted with one or more groups selected from the group consisting of halogen, deuterium, OH, CN, and NH₂; preferably, each R⁷, R⁸, or R⁹ is independently selected from the group consisting of hydrogen, -NH₂, methyl, ethyl, -CD₂CD₃, cyclopropyl, and -CD₃.

12. The compound of formula (I), the isomer thereof, or the pharmaceutically acceptable salt thereof according to claim 1, selected from a compound of formula (I-1), 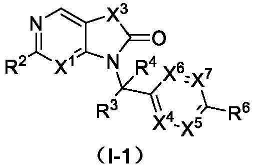
wherein X¹, X³, X⁴, X⁵, X⁶, X⁷, R², R³, R⁴, and R⁶ are as defined in the compound of formula (I);
the carbon atom to which R³ and R⁴ are linked may be a chiral carbon atom present in a form of a single (R) or (S) enantiomer or in a form enriched with one enantiomer;
or the compound of formula (I), the isomer thereof, or the pharmaceutically acceptable salt thereof according to claim 1, selected from a compound of formula (I-2), 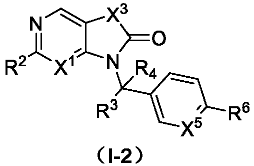 wherein,
X⁵ is selected from the group consisting of CH and N;
X¹, X³, R², R³, R⁴, and R⁶ are as defined in the compound of formula (I);
the carbon atom to which R³ and R⁴ are linked may be a chiral carbon atom present in a form of a single (R) or (S) enantiomer or in a form enriched with one enantiomer;
or the compound of formula (I), the isomer thereof, or the pharmaceutically acceptable salt thereof according to claim 1, selected from a compound of formula (I-3), 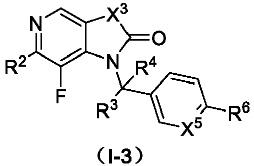 wherein,
X⁵ is selected from the group consisting of CH and N;
X³, R², R³, R⁴, and R⁶ are as defined in the compound of formula (I);
the carbon atom to which R³ and R⁴ are linked may be a chiral carbon atom present in a form of a single (R) or (S) enantiomer or in a form enriched with one enantiomer;
or the compound of formula (I), the isomer thereof, or the pharmaceutically acceptable salt thereof according to claim 1, selected from a compound of formula (1-4), 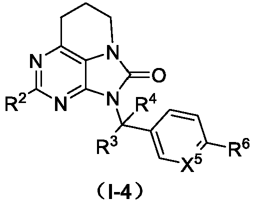 wherein,
X⁵ is selected from the group consisting of CH and N;
R², R³, R⁴, and R⁶ are as defined in the compound of formula (I);
the carbon atom to which R³ and R⁴ are linked may be a chiral carbon atom present in a form of a single (R) or (S) enantiomer or in a form enriched with one enantiomer;
or the compound of formula (I), the isomer thereof, or the pharmaceutically acceptable salt thereof according to claim 1, selected from a compound of formula (I-5), 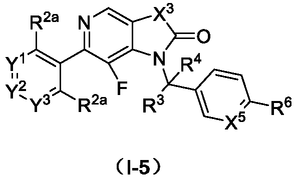 wherein,
X⁵ is selected from the group consisting of CH and N;
Y¹, Y², and Y³ are each independently selected from the group consisting of CH and N;
X³, R³, R⁴, R⁶, and R^{2a} are as defined in the compound of formula (I);
the carbon atom to which R³ and R⁴ are linked may be a chiral carbon atom present in a form of a single (R) or (S) enantiomer or in a form enriched with one enantiomer;
or the compound of formula (I), the isomer thereof, or the pharmaceutically acceptable salt thereof according to claim 1, selected from a compound of formula (I-6), 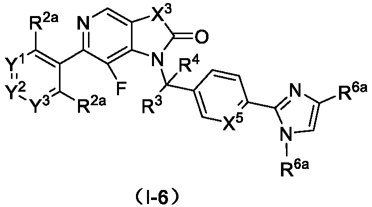 wherein,
X⁵ is selected from the group consisting of CH and N;
Y¹, Y², and Y³ are as defined in the compound of formula (I-5);
X¹, R^{2a}, R³, R⁴, and R^{6a} are as defined in the compound of formula (I);
the carbon atom to which R³ and R⁴ are linked may be a chiral carbon atom present in a form of a single (R) or (S) enantiomer or in a form enriched with one enantiomer;
or the compound of formula (I), the isomer thereof, or the pharmaceutically acceptable salt thereof according to claim 1, selected from a compound of formula (I-7), 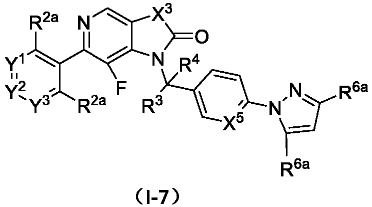 wherein,
X⁵ is selected from the group consisting of CH and N;
Y¹, Y², and Y³ are as defined in the compound of formula (I-5);
X¹, R^{2a}, R³, R⁴, and R^{6a} are as defined in the compound of formula (I);
the carbon atom to which R³ and R⁴ are linked may be a chiral carbon atom present in a form of a single (R) or (S) enantiomer or in a form enriched with one enantiomer.

13. A compound of the formula below, an isomer thereof, or a pharmaceutically acceptable salt thereof,

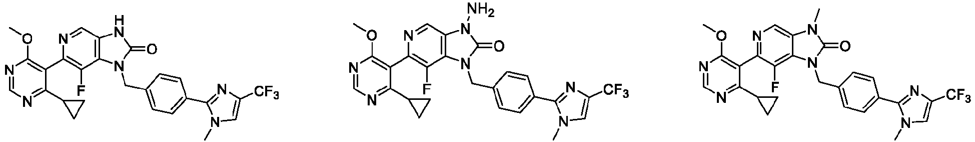

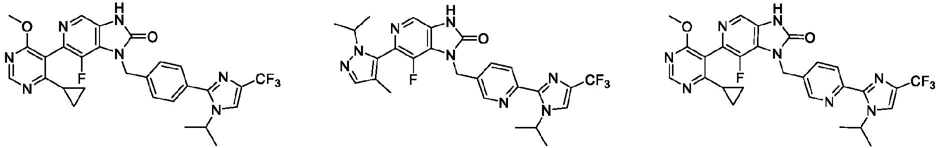

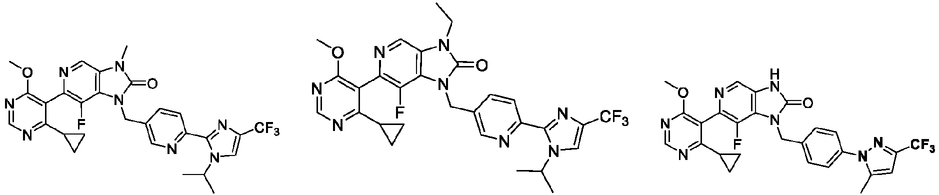

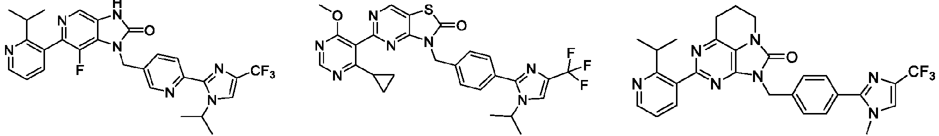

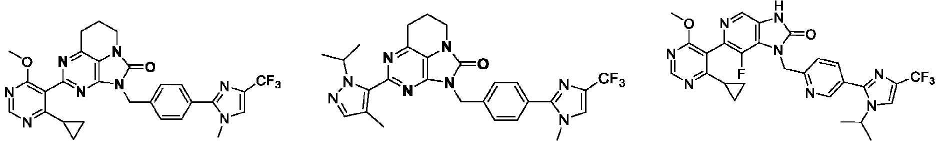

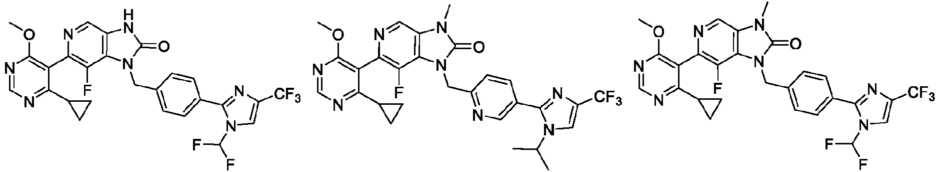

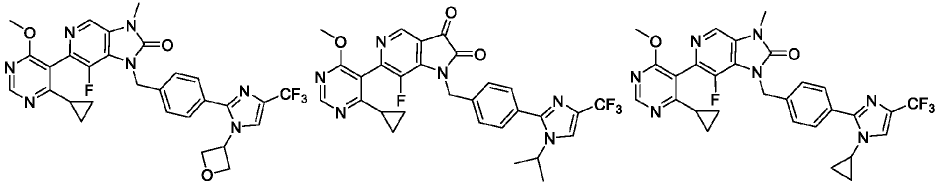

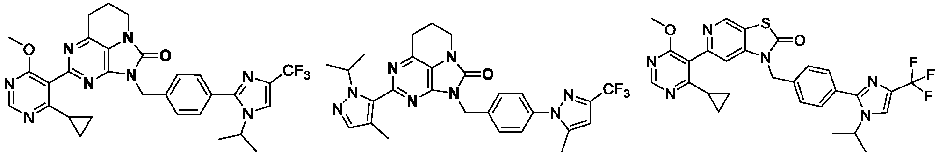

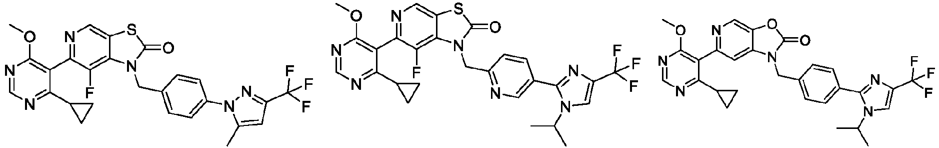

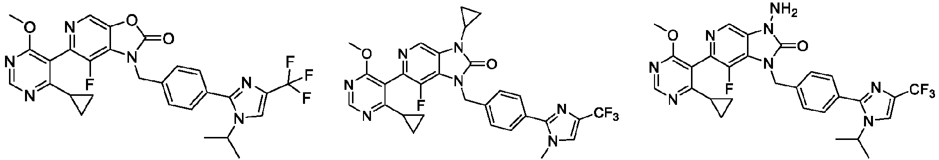

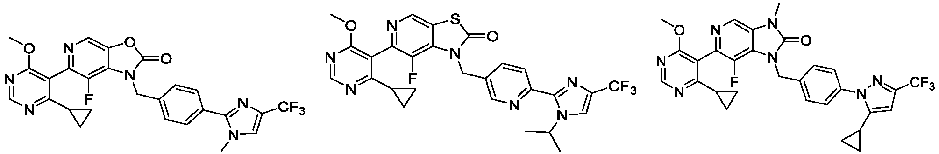

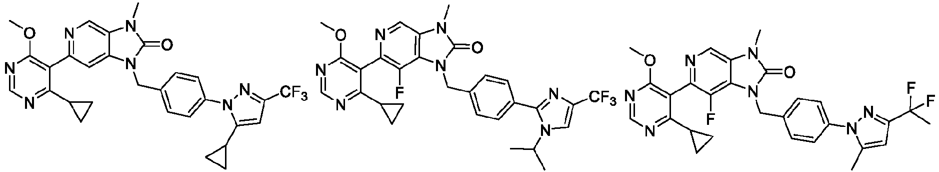

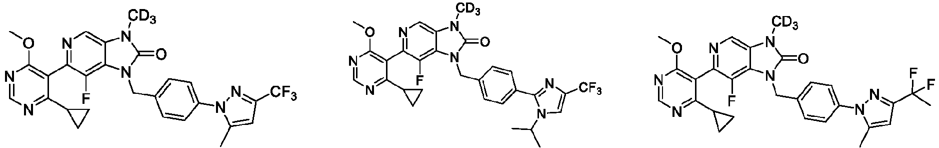

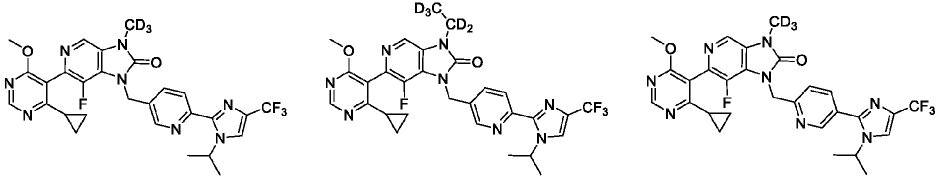

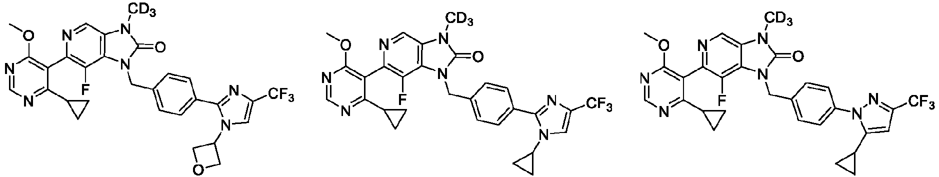

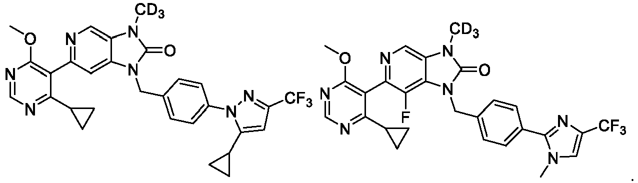

14. A pharmaceutical composition, comprising a therapeutically or prophylactically effective amount of the compound, the isomer thereof, or the pharmaceutically acceptable salt thereof according to any one of claims 1-13.

15. Use of the compound, the isomer thereof, or the pharmaceutically acceptable salt thereof according to any one of claims 1-13 for preparing a medicament for treating or preventing a disease associated with inhibition of ubiquitin specific peptidase 1 (USP1).
